(19) Europäisches Patentamt
European Patent Office
Office européen des brevets

(11) EP 1 004 665 A1

(12) **EUROPEAN PATENT APPLICATION**
published in accordance with Art. 158(3) EPC

(43) Date of publication:
**31.05.2000 Bulletin 2000/22**

(21) Application number: **98937802.1**

(22) Date of filing: **12.08.1998**

(51) Int. Cl.[7]: **C12N 15/12**, C12P 21/08, C12N 5/20, C12N 5/10

(86) International application number:
**PCT/JP98/03602**

(87) International publication number:
**WO 99/09159 (25.02.1999 Gazette 1999/08)**

(84) Designated Contracting States:
**AT BE CH CY DE DK ES FI FR GB GR IE IT LI LU MC NL PT SE**

(30) Priority: **13.08.1997 JP 23339697**
**30.07.1998 JP 23012198**

(71) Applicant: **Japan Tobacco Inc.**
**Tokyo 105-8422 (JP)**

(72) Inventors:
- **NAKAMURA, Yusuke**
**Yokohama-shi Kanagawa 225-0011 (JP)**
- **TOKINO, Takashi**
**Shinagawa-ku Tokyo 140-0000 (JP)**

(74) Representative:
**VOSSIUS & PARTNER**
**Siebertstrasse 4**
**81675 München (DE)**

# (54) SCAVENGER RECEPTOR-LIKE PROTEINS

(57) cDNAs encoding three novel scavenger receptors named CSR1, CSR2 and CSR3 obtained from a cDNA library originating in human fetal brain with the use of a human genomic DNA having the ability (reactivity) of binding to a tumor-inhibitory protein p53 as a probe. Each novel scavenger receptor has a secondary structure of protein characteristic of macrophage scavenger receptors and the expression thereof is induced depending on various intracellular stresses. Moreover, these receptors have inhibitory effects on cell death induced by these stresses.

EP 1 004 665 A1

## Description

### Technical Field

The present invention relates to novel scavenger receptor-like proteins and fragments thereof, DNAs encoding said proteins and fragments thereof, expression vectors containing said DNAs, transformants transformed with said expression vectors, antibodies capable of reacting with said proteins or fragments thereof, and cells producing said antibodies.

### Background Art

Cholesterols are present in every tissue and plasma of the body in the free, long-chain fatty acid, or esterified form. The former two forms play critical roles in cell membrane construction, and the esterified one is a physiologically inactive storage form. Cholesterols in the body originates from dietary cholesterol absorbed through small intestine following food intake, or are biosynthesized in various tissues, especially in liver. Free cholesterols synthesized in and secreted from the liver is incorporated into very low density lipoproteins (VLDL), and metabolized via intermediary density lipoproteins (IDL) to low density lipoproteins by the action of lipoprotein lipase in the blood and hepatic triglyceride lipase (HTGL). LDL thus formed is delivered to peripheral tissues by the blood stream, taken up into peripheral cells such as vascular endothelial cells mediated by peripheral LDL receptors to supply necessary amount of cholesterols for constructing peripheral cells.

The LDL receptor is down-regulated with the increase of intracellular cholesterols due to the LDL uptake so as to prevent its excessive accumulation.

Besides such an uptake of normal LDL into the cells mediated by the LDL receptors, there exist pathways for taking up into the cell modified LDLs (oxidized LDL, acetylated LDL, succinylated LDL, malondialdehyde (MDA) LDL, etc.) produced by various physicochemical alterations due to $Fe^{3+}$, heat, or the like. One is the uptake mediated by macrophage scavenger receptors, and another is that mediated by the oxidized LDL receptors of vascular endothelial cells (Nature, 386, 73-77, 1997).

It has been elucidated that peripheral macrophages usually express almost no normal LDL receptors but the scavenger receptors to recognize modified LDLs such as oxidized LDL and take up modified LDLs via this receptors (Nature, **343**, 531-535, 1990; ibid., **343**, 570-572, 1990; Proc. Natl. Acad. Sci. USA, **87**, 9133-9137, 1990; ibid., **87**, 8810-8814, 1990: Curr. Opin. Lipodol., **2**, 295-300, 1991, and J. Clin. Invest., **90**, 1450-1457, 1991). Since this scavenger receptors, unlike the above LDL receptors, do not undergo downregulation due to an increase of intracellular cholesterols, they take up modified LDLs (cholesterols) in excess to metamorphose macrophages into foam cells. Accumulation of these foam macrophages in the subendothelium and inner wall of blood vessels is considered as one of the principal causes of atherosclerosis. In fact, a recent report confirmed that atherosclerotic lesion was reduced in size in knockout mice in which the macrophage scavenger receptors were inactivated by genetic engineering, compared with control mice, proving profound involvement of the scavenger receptors in the development of atherogenesis. Furthermore, another report revealed that the scavenger receptors are involved in the uptake of the advanced glycation end product (AGE) causative of the protection impairment against bacterial infection and diabetic angiopathy (Nature, **386**, 292-296, 1997).

There are two types of the macrophage scavenger receptors, type I and type II. Both types consist of six domains, differing only in the C-terminal structure. The receptors are unique membrane proteins with the structure of inside-out type trimeric membrane glycoprotein. Amino acid sequences of both type I and type II are highly homologous among animal species including humans, mice, cattle, and rabbits (type I: 64-81%, type II: 60-81%). The domains, from the C-terminus, comprise 1) C-terminal specific domain, 2) collagen-like domain, 3) $\alpha$-helical coiled-coil domain, 4) spacer domain, 5) transmembrane domain, and 6) cytoplasmic domain.

The "C-terminal specific domain" of type I is cysteine-rich, and highly homologous to complement factor 1, CD5, CD6, etc.

The "collagen-like domain" has the glycine (Gly)-X-Y repeat structure peculiar to collagen. Its C-terminal side is abundant in positive-charged amino acid residues that suitably bind to negative-charged ligands. It has been confirmed that a mutant in which C-terminal 22 amino acid residues of the collagen-like domain are deleted no longer binds to the ligand. Furthermore, the experiments using various deletion mutants clarified that the lysine residue (K 337) is critical for the ligand binding.

The "$\alpha$ helical coiled-coil domain" serves to form an active receptor in terms of the protein primary structure by associating three scavenger receptor polypeptides. This domain also plays a role in binding to a ligand such as modified LDLs to enter a cell and dissociating the ligand by altering the higher order structure of the receptor depending on the pH decrease inside endosomes. This domain has an $\alpha$-helical coiled-coil structure in which right-handed heptad repeats turn twice per seven amino acid residues, and said three polypeptides form a trimer inwardly directing hydrophobic amino acids such as leucine and isoleucine, which are present at every seven amino acid residues, and outwardly directing hydrophilic amino acids and glycosylation sites.

It has been experimentally demonstrated that a histidine residue of the heptad repeats close to the collagen-like domain functions in the intracellular dissociation of ligands. Furthermore, an antibody recognizing the region containing the histidine residue in the center and consisting of 15 amino acid residues in this coiled-coil domain inhibits the calcium-independent cell adhesion of macrophages, indicating that the macrophage scavenger receptors can also function as cell adhesion molecules.

The "cytoplasmic domain" has a tight-turn structure characteristic of endocytosis signals similar to NPXY sequence present in the LDL receptors and insulin receptors and YXRF sequence in the transferin receptors, and it has been demonstrated that endocytosis is suppressed by deleting these sequences.

In particular, the sturcture of the collagen-like domain, the ligand-binding site of the scavenger receptors is known to be suitable for removing xenobiotics and wastes such as modified LDLs that are bound and precipitate on the extracellular matrix including collagen.

Previous studies on the macrophage scavenger receptors suggest that the receptors are considered as biological "cleaners" with the principal role to take in xenobiotics and denatured proteins in the body (for example, modified LDLs, glycation end products, xenobiotics derived from infecting bacteria, etc.), and another role to protect the cells and body by removing such foreign substances and denatured proteins from the body as one of biological protection mechanisms.

Disclosure of the Invention

An objective of this invention is to identify a novel scavenger receptor having a structure analogous to that characteristic to the known macrophage scavenger receptors and protectively acting against various intracellularly generated stresses, and provide pharmaceuticals and methods for preventing and treating morbidities and disorders caused by intracellular stresses, xenobiotics or denatured proteins generated in the body.

The present inventors analyzed various human genes transcriptionally controlled by the tumor suppressing protein p53, which belongs to a different technical field from the known LDL receptors, oxidized LDL receptors and macrophage scavenger receptors. As a result, we discovered three novel human cDNA clones (collectively termed "cellular sensor-related or cellular stress response genes (CSR genes )"; cDNAs and proteins encoded by said cDNAs are abbreviated as "CSR1," "CSR2," and "CSR3."), each encoding alternative splicing variant having the extremely similar protein secondary structure to that characteristic of the human macrophage scavenger receptors but no amino acid sequence homology to said receptors, thereby completing this invention.

The novel genes and proteins of this invention are considered to be of new scavenger receptors having the following characteristics to protectively act against various stresses generated in the cell.

(1) The proteins have structures highly homologous to the type I and type II macrophage scavenger receptors (GenBank Accession No.: S08278; Bohrer, L. et al., Nature, **343**, 570-572, 1990) and/or sensor protein (GenBank Accession No.: P30844; Nagasawa, S., J. Biochem., **114**, 350-357, 1993).

(2) The proteins have secondary structures as schematically represented in Fig. 2, consisting of (a) the transmembrane domain comprising a leucine zipper motif with four leucine unit repeats, (b) $\alpha$-helical coiled-coil domain, and (c) collagen-like domain with forty-nine Gly-X-Y sequence unit repeats.

(3) The three molecules having the above unique structures form a trimer by forming $\alpha$-helix in the coiled-coil domain and triple helix in the collagen-like domain.

(4) The collagen-like domain carries a positive net charge at physiological pHs.

(5) The proteins have many phosphorylation sites and glycosylation sites. Specifically, the phosphorylation sites include those for casein kinase 2, protein kinase C, and tyrosine kinase 2.

(6) The untranslated region at the 3'-end of CSR gene comprises six nucleotide sequences, AGTAAA and AATAAA near the 3'-end poly $(A)^+$ region, which are supposed to be profoundly involved in providing the polyadenylation signal.

(7) Expression of CSR is induced by intracellular stresses such as ultraviolet irradiation, hydrogen peroxide, heat shock, etc. as well as many reagents causing oxidative stresses to the cell including DEM, PMA, sodium azide, sodium arsenite, GSNO, SNP, hemin, etc.

(8) The expression of CSR induced by intracellular stresses such as ultraviolet irradiation, hydrogen peroxide, heat shock, etc. is suppressed in the presence of antioxidants.

(9) CSR suppresses cell death caused by intracellular stresses such as ultraviolet irradiation, hydrogen peroxide, heat shock, etc.

(10) The proteins have the heme-binding site, and microbody (peroxisome) C-terminal targeting signal region.

Therefore, genes encoding CSRs, CSR proteins, or fragments thereof are extremely useful for developing pharmaceuticals targeting the genes or proteins that prevent and treat morbidities and disorders (for example, atherosclerosis, diabetic angiopathy, bacterial infection, etc.) caused by intracellular stresses, xenobiotics or denatured proteins produced in the body.

The DNAs of the present invention themselves are useful as antisense pharmaceuticals that regulate the functions of CSR at the gene level, and in gene therapy. The fragments (e.g. extracellular regions, various domains) of the proteins are useful, for example, as soluble protein pharmaceuticals.

The antibodies or a portion of them reactive with the CSR proteins of the present invention are extremely useful as antibody pharmaceuticals that regulate the functions of CSR.

Genes (DNAs), proteins, and antibodies of the present invention are useful as reagents for searching proteins (ligands) interacting with the proteins of the present invention, thereby elucidating the function of said ligands, and developing therapeutic drugs targeting said ligands.

Based on the genetic information of CSRs derived from mammals sucu as mice, one embodiment of DNAs of the present invention, model animals can be produced by disrupting (inactivating) said genes. Function of genes and proteins of the present invention can be elucidated by analyzing the physical, biological, pathologic, and genetic characteristics of these model animals.

Moreover, by introducing the human-derived CSR gene into the model animals whose endogenous gene is thus disrupted, model animals that have only the human-derived gene of the present invention can be produced. By administering drugs (compounds, antibodies, and so on) targeting the introduced human gene to these model animals, the therapeutic effect of the drug can be estimated.

Namely, the present invention provides the DNAs, proteins, expression vectors, transformants, antibodies, pharmaceutical compositions, and cells, mentioned below.

(1) A DNA encoding a protein having the amino acid sequence of SEQ ID NO: 10 or a fragment thereof.
(2) A DNA encoding a protein having the amino acid sequence of SEQ ID NO: 12 or a fragment thereof.
(3) A DNA encoding a protein having the amino acid sequence of SEQ ID NO: 14 or a fragment thereof.
(4) A DNA comprising a nucleotide sequence corresponding to nucleotide residues 276 to 2096 of the nucleotide sequence of SEQ ID NO: 9, or a fragment thereof.
(5) A DNA comprising a nucleotide sequence corresponding to nucleotide residues 276 to 1676 of the nucleotide sequence of SEQ ID NO: 11, or a fragment thereof.
(6) A DNA comprising a nucleotide sequence corresponding to nucleotide residues 364 to 1971 of the nucleotide sequence of SEQ ID NO: 13, or a fragment thereof.
(7) A DNA hybridizing with a DNA having the nucleotide sequence of SEQ ID NO: 9, 11, or 13 under stringent conditions.
(8) A protein having the amino acid sequence of SEQ ID NO: 10 or an amino acid sequence substantially the same as said amino acid sequence, or a fragment thereof.
(9) A protein having the amino acid sequence of SEQ ID NO: 12 or an amino acid sequence substantially the same as said amino acid sequence, or a fragment thereof.
(10) A protein having the amino acid sequence of SEQ ID NO: 14 or an amino acid sequence substantially the same as said amino acid sequence, or a fragment thereof.
(11) An expression vector comprising the DNA of any one of (1) to (7).
(12) A transformant carrying the expression vector of (11).
(13) An antibody or its portion reactive with the proteins of (8) to (10) or their fragments.
(14) The antibody or its portion of (13), wherein the antibody is a monoclonal antibody.
(15) A pharmaceutical composition comprising the antibody or its portion of (13) or (14) and a pharmaceutically acceptable carrier.
(16) A cell producing a monoclonal antibody reactive with the proteins of (8) to (10) or their fragments.
(17) The cell of (16), wherein said cell is hybridoma obtainable by fusing B cells derived from a non-human mammal capable of producing a monoclonal antibody with myeloma derived from a mammal.
(18) The cell of (16), wherein said cell is a recombinant cell transformed with either a DNA encoding a heavy chain of said monoclonal antibody or a DNA encoding a light chain of said monoclonal antibody, or both DNAs.

In the following, the present invention is explained in detail by clarifying the meanings of terms used in the present application and the general production methods of proteins, DNAs, antibodies, and cells of the present invention.

The "proteins or fragments thereof" of this invention meant proteins and fragments thereof derived from mammals such as humans, cattle, sheep, pigs, goats, rabbits, rats, hamsters, guinea pigs and mice, preferably from humans, rabbits, rats or mice, most preferably from humans.

Particularly preferable examples are (1) a protein having the amino acid sequence of SEQ ID NO: 10 or an amino acid sequence substantially the same as said amino acid sequence, or its fragment, (2) a protein having the amino acid sequence of SEQ ID NO: 12 or an amino acid sequence substantially the same as said amino acid sequence, or its fragment, and (3) a protein having the amino acid sequence of SEQ ID NO: 14 or an amino acid sequence substantially the same as said amino acid sequence, or its fragment.

Here, "having substantially the same amino acid sequence" means to include a protein having an amino acid sequence where multiple amino acids, preferably 1 to 10 amino acids, particularly preferably 1 to 5 amino acids, in the amino acid sequence shown in SEQ ID NO: 10, 12, or 14, are substituted, deleted, and/or modified, and a protein having an amino acid sequence where multiple amino acids, preferably 1 to 10 amino acids, particularly preferably 1 to 5 amino acids, are added to said amino acid sequence, as far as the protein has substantially the same biological properties as the protein having said amino acid sequence.

The "fragments of proteins" mean any fragments of amino acid sequences in CSR proteins of this invention, including, for example, each domain such as extracelluar region, transmembrane region or cytoplasmic region, or sites of each region for binding to (interacting with) other proteins and low molecular weight ligands. In addition, fusion proteins comprising the extracellular region and other molecules (for example, the constant region (Fc) of human immunoglobulin heavy chain) or portion thereof are also in the scope of protein fragments of this invention.

The term "extracellular region" used herein is explained below. A transmembrane protein such as CSR proteins of the present invention, various G protein-coupled receptors, or cell surface molecules connects with the membrane through the hydrophobic peptide region penetrating the lipid bilayer of the membrane once or several times and has structure composed of three main regions, that is, extracellular region, transmembrane region, and cytoplasmic region. Such a transmembrane protein exists as a monomer, homodimer, heterodimer, or oligomer with another chain(s) having the same or different amino acid sequence.

The term "extracellular region" used herein means the partial structure (partial sequence) existing outside of the membrane that holds the transmembrane protein as mentioned above among the whole structure of said membrane protein. In other words, it corresponds to the region excluding the region incorporated into the membrane (transmembrane region) and the region existing in the cytoplasm following the transmembrane region (cytoplasmic region). If desired, one to five amino acids derived from the amino acids constituting the transmembrane and/or cytoplasmic region can be added to the N- terminus and/or C- terminus of the extracellular region in the present invention.

"The constant region or its portion of human immunoglobulin heavy chain" used herein means the constant region or the Fc region of human-derived immunoglobulin heavy chain (H chain) as described, or a portion of them. The immunoglobulin can be any immunoglobulin belonging to any class and any subclass. Specifically, examples of the immunoglobulin are IgG (IgG1, IgG2, IgG3, and IgG4), IgM, IgA (IgA1 and IgA2), IgD, and IgE. Preferably, the immunoglobulin is IgG (IgG1, IgG2, IgG3, or IgG4), or IgM. Examples of particularly preferable immunoglobulin of the present invention are those belonging to human-derived IgG (IgG1, IgG2, IgG3, or IgG4).

Immunoglobulin has a Y-shaped structural unit in which four chains composed of two homologous light chains (L chains) and two homologous heavy chains (H chains) are connected through disulfide bonds (S-S bonds). The light chain is composed of the light chain variable region (VL) and the light chain constant region (CL). The heavy chain is composed of the heavy chain variable region (VH) and the heavy chain constant region (CH).

The heavy chain constant region is composed of some domains having the amino acid sequences inherent in each class (IgG, IgM, IgA, IgD, and IgE) and each subclass (IgG1, IgG2, IgG3, and IgG4, IgA1, and IgA2).

The heavy chain of IgG (IgG1, IgG2, IgG3, and IgG4) is composed of VH, CH1 domain, hinge region, CH2 domain, and CH3 domain in this order from N terminus.

Similarly, the heavy chain of IgG1 is composed of VH, $C\gamma_1 1$ domain, hinge region, $C\gamma_1 2$ domain, and $C\gamma_1 3$ domain in this order from N terminus. The heavy chain of IgG2 is composed of VH, $C\gamma_2 1$ domain, hinge region, $C\gamma_2 2$ domain, and $C\gamma_2 3$ domain in this order from N terminus. The heavy chain of IgG3 is composed of VH, $C\gamma_3 1$ domain, hinge region, $C\gamma_3 2$ domain, and $C\gamma_3 3$ domain in this order from N terminus. The heavy chain of IgG4 is composed of VH, $C\gamma_4 1$ domain, hinge region, $C\gamma_4 2$ domain, and $C\gamma_4 3$ domain in this order from N terminus.

The heavy chain of IgA is composed of VH, $C\alpha 1$ domain, hinge region, $C\alpha 2$ domain, and $C\alpha 3$ domain in this order from N terminus.

Similarly, the heavy chain of IgA1 is composed of VH, $C\alpha_1 1$ domain, hinge region, $C\alpha_1 2$ domain, and $C\alpha_1 3$ domain in this order from N terminus. The heavy chain of IgA2 is composed of VH, $C\alpha_2 1$ domain, hinge region, $C\alpha_2 2$ domain, and $C\alpha_2 3$ domain in this order from N terminus.

The heavy chain of IgD is composed of VH, $C\delta 1$ domain, hinge region, $C\delta 2$ domain, and $C\delta 3$ domain in this order from N terminus.

The heavy chain of IgM is composed of VH, $C\mu 1$ domain, $C\mu 2$ domain, $C\mu 3$ domain, and $C\mu 4$ domain in this order from N terminus and have no hinge region as seen in IgG, IgA, and IgD.

The heavy chain of IgE is composed of VH, $C\varepsilon 1$ domain, $C\varepsilon 2$ domain, $C\varepsilon 3$ domain, and $C\varepsilon 4$ domain in this order from N terminus and have no hinge region as seen in IgG, IgA, and IgD.

If, for example, IgG is treated with papain, it is cleaved at the slightly N terminal side beyond the disulfide bonds existing in the hinge region where the disulfide bonds connect the two heavy chains to generate two homologous Fab, in which a heavy chain fragment composed of VH and CH1 is connected with one light chain through a disulfide bond, and one Fc, in which two homologous heavy chain fragments composed of the hinge region, CH2 domain, and CH3 domain are connected through disulfide bonds (See "Immunology Illustrated", original 2nd ed., Nankodo, pp.65-75 (1992); and "Focus of Newest Medical Science 'Recognition Mechanism of Immune System'", Nankodo, pp.4-7 (1991); and so on).

Namely, "a portion of a constant region of immunoglobulin heavy chain" of the present invention means a portion of a constant region of an immunoglobulin heavy chain having the structural characteristics as mentioned above, and preferably, is the constant region without C1 domain, or the Fc region. Specifically, examples thereof are the region composed of hinge region, C2 domain, and C3 domain from each of IgG, IgA, and IgD, and are the region composed of C2 domain, C3 domain, and C4 domain from each of IgM and IgE. A particularly preferable example thereof is the Fc region of human-derived IgG1.

A preferable example of the fusion protein included in the protein fragments of the present invention is that composed of the above-described extracellular region of the protein of the present invention and a constant region or a portion of a constant region of human immunoglobulin heavy chain. Preferably, it is a fusion polypeptide composed of an extracellular region of a protein of the present invention and a portion of a constant region of human IgG heavy chain, and particularly preferably, it is a fusion polypeptide composed of an extracellular region of a protein of the present invention and the region (Fc) composed of a hinge region, CH2 domain, and CH3 domain of human IgG heavy chain. Moreover, IgG1 is preferable among IgG. In addition, a protein derived from human, mouse, or rat (preferably, human) is preferable as the protein of the present invention.

Alphabetical triplet or single letter codes used to represent amino acids in the present specification or figures mean amino acids as follows. (Gly/G) glycine, (Ala/A) alanine, (Val/V) valine, (Leu/L) leucine, (Ile/I) isoleucine, (Ser/S) serine, (Thr/T) threonine, (Asp/D) aspartic acid, (Glu/E) glutamic acid, (Asn/N) asparagine, (Gln/Q) glutamine, (Lys/K) lysine, (Arg/R) arginine, (Cys/C) cysteine, (Met/M)methionine, (Phe/F) phenylalanine, (Tyr/Y) tyrosine, (Trp/W) tryptophane, (His/H) histidine, (Pro/P) proline.

The protein, protein fragment, and fusion protein of the present invention can be produced not only by recombinant DNA technology as mentioned below but also by a method well known in the art such as a chemical synthetic method and a cell culture method, or a modified method thereof.

The above fusion protein of the present invention has the advantage that the fusion polypeptide can be purified extremely easily by using affinity column chromatography using the property of protein A, which binds specifically to the immunoglobulin fragment because the fusion polypeptide of the present invention has a portion of a constant region (for example Fc) of an immunoglobulin such as IgG as mentioned above as a fusion partner. Moreover, since various antibodies against the Fc of various immunoglobulin are available, an immunoassay for the fusion polypeptides can be easily performed with antibodies against the Fc.

The DNA of the present invention encodes the above-mentioned protein of the present invention or its fragment, and includes any nucleotide sequence that can encode the protein of the present invention. The DNA of the present invention includes either a genomic DNA or cDNA. In addition, the DNA includes any DNA composed of any codons encoding the same amino acids.

A preferable examle of the DNA of the present invention is a DNA encoding a human-derived protein.

Specific examples of the DNA are described below.

(1) A DNA encoding a protein having the amino acid sequence of SEQ ID NO: 10, 12, or 14, its fragment, or a biological analog obtained by substituting, deleting, and/or modifying multiple amino acids, preferably 1 to 10 amino acids, particularly preferably 1 to 5 amino acids in the amino acid sequence of said protein or fragment, or by inserting multiple amino acids, preferably 1 to 10 amino acids, particularly preferably 1 to 5 amino acids, in said amino acid sequence.
(2) A DNA hybridizing with a DNA having the nucleotide sequence of SEQ ID NO: 9, 11, or 13 or its fragment.

Specific examples thereof are as follows.

(1) A DNA comprising a nucleotide sequence corresponding to nucleotide residues 276 to 2096 of the nucleotide sequence of SEQ ID NO: 9, or its fragment.
(2) A DNA comprising a nucleotide sequence corresponding to nucleotide residues 276 to 1676 of the nucleotide sequence of SEQ ID NO: 11, or its fragment.
(3) A DNA comprising a nucleotide sequence corresponding to nucleotide residues 364 to 1971 of the nucleotide sequence of SEQ ID NO: 13, or its fragment.

Examples of "stringent conditions" are as follows. When a probe with 50 or more nucleotides is used and hybridization is performed in 0.9% NaCl, the standard of temperature where 50% dissociation occurs (Tm) is calculated using the following formula and the temperature for hybridization can be determined according to the following formula.

Tm = 82.3°C + 0.41 x (G+C) % - 500/n - 0.61 x (formamide) % (n means the number of the nucleotide of probe).

Temperature = Tm -25°C.

In addition, when a probe with 100 or more nucleotides (G+C = 40 to 50% ) is used, it should be considered that Tm varies as (1) and (2) mentioned below.

(1) Tm descends by about 1°C per 1% mismatch.
(2) Tm descends by 0.6 to 0.7°C per 1% formamide.

Accordingly, the temperature conditions for the combination of completely complementary strands can be set as follows.

(A) 65 to 75°C (formamide not added)
(B) 35 to 45°C (in the presence of 50% formamide)

The temperature conditions for the combination of incompletely complementary strands can be set as follows.

(A) 45 to 55°C (formamide not added)
(B) 35 to 42°C (in the presence of 30% formamide)

The temperature conditions when a probe with 23 or less nucleotides is used can be 37°C or can be calculated using the following formula.

Temperature = 2°C x (the number of A+T) + 4°C x (the number of C+G) -5°C.

The DNA of the present invention can be a DNA obtained by any method. For example, the DNA includes complementary DNA (cDNA) prepared from mRNA, DNA prepared from genomic DNA, DNA prepared by chemical synthesis, DNA obtained by PCR amplification with RNA or DNA as a template, and DNA constructed by appropriately combining these methods.
The DNA encoding the protein of the present invention can be obtained by the usual method such as a method to clone cDNA from mRNA encoding the protein of the present invention, a method to isolate genomic DNA and then splice them, chemical synthesis and so on.

(1) cDNA can be cloned from the mRNA encoding the protein of the present invention by, for example, the method described below.

First, the mRNA encoding the protein of the present invention is prepared from the above-described tissues or cells expressing and producing a cell surface molecule (polypeptide) of the present invention. mRNA can be prepared isolating total RNA by a known method such as quanidine-thiocyanate method (Chirgwin et al., Biochemistry, Vol.18, p5294, 1979), hot phenol method, or AGPC method, and subjecting it to affinity chromatography using oligo-dT cellulose or poly-U Sepharose.

Then, with the mRNA obtained as a template, cDNA is synthesized, for example, by a well-known method using reverse transcriptase such as the method of Okayama et al. (Mol. Cell. Biol. Vol.2, p.161 (1982); ibid. Vol.3, p.280 (1983)) or the method of Hoffman et al. (Gene Vol.25, p.263 (1983)), and converted into double-stranded cDNA. A cDNA library is prepared by transforming *E. coli* with plasmid vectors, phage vectors, or cosmid vectors having this cDNA or by transfecting *E. coli* after *in vitro* packaging.

The plasmid vectors used in this invention are not limited as long as they are replicated and maintained in hosts. Any phage vectors that can be replicated in hosts can also be used. Examples of usually used cloning vectors are pUC19, λgt10, λgt11, and so on. When the vector is applied to immunological screening as mentioned below, the vector having a promoter that can express a gene encoding the polypeptide of the present invention in a host is preferably used.

cDNA can be inserted into a plasmid by, for example, the method of Maniatis et al. (Molecular Cloning, A Laboratory Manual, second edition, Cold Spring Harbor Laboratory, p.1.53, 1989). cDNA can be inserted into a phage vector by, for example, the method of Hyunh et al. (DNA cloning, a practical approach, Vol.1, p.49 (1985)). These methods can be simply performed by using a commercially available cloning kit (for example, a product from Takara Shuzo). The recombinant plasmid or phage vector thus obtained is introduced into appropriate host cells such as a prokaryote (for example, *E. coli*: HB101, DH5 α, MC1061/P3, etc.).

Examples of a method for introducing a plasmid into a host are calcium chloride method, calcium chloride/rubidium chloride method described in Molecular Cloning, A Laboratory Manual (second edition, Cold Spring Harbor Laboratory, p.1.74 (1989)), and electroporation method. Phage vectors can be introduced into host cells by, for example, a method in which the phage DNAs are introduced into grown hosts after *in vitro* packaging. *In vitro* packaging can be easily performed with a commercially available *in vitro* packaging kit (for example, a product from Stratagene or Amersham).

The cDNA encoding the protein of the present invention can be isolated from the cDNA library so prepared according to the method mentioned above by combining general cDNA screening methods.

For example, a clone comprising the desired cDNA can be screened by a known colony hybridization method (Crunstein et al. Proc. Natl. Acad. Sci. USA, Vol.72, p.3961 (1975)) or plaque hybridization method (Molecular Cloning, A Laboratory Manual, second edition, Cold Spring Harbor Laboratory, p.2.108 (1989)) using $^{32}P$-labeled chemically synthesized oligonucleotides as probes, which are corresponding to the amino acid sequence of the polypeptide of the present invention. Alternatively, a clone having a DNA fragment encoding a specific region within the polypeptide of the present invention can be screened by amplifying the region by PCR with synthetic PCR primers.

When a cDNA library prepared using a cDNA expression vector (for example, λZAPII phage vector) is used, the desired clone can be screened by the antigen-antibody reaction using an antibody against the polypeptide of the present invention. A screening method using PCR method is preferably used when many clones are subjected to screening.

The nucleotide sequence of the DNA thus obtained can be determined by Maxam-Gilbert method (Maxam et al. Proc. Natl. Acad. Sci. USA, Vol.74, p.560 (1977)) or the dideoxynucleotide synthetic chain termination method using phage M13 (Sanger et al. Proc. Natl. Acad. Sci. USA, Vol.74, pp.5463-5467 (1977)). The whole or a portion of the gene encoding the polypeptide of the present invention can be obtained by excising the clone obtained as mentioned above with restriction enzymes and so on.

(2) The DNA encoding the polypeptide of the present invention can be isolated from the genomic DNA derived from the cells expressing the polypeptide of the present invention as mentioned above by the following methods. Such cells are solubilized preferably by SDS or proteinase K, and the DNAs are deproteinized by repeating phenol extraction. RNAs are digested preferably with ribonuclease. The DNAs obtained are partially digested with appropriate restriction enzymes, and the DNA fragments obtained are amplified with appropriate phage or cosmid to generate a library. Then, clones having the desired sequence are detected, for example, by using radioactively labeled DNA probes, and the whole or a portion of the gene encoding the protein of the present invention is obtained from the clones by excision with restriction enzyme and so on.

cDNA encoding a human-derived protein can be obtained by preparing a cosmid library into which human genomic DNAs are introduced ("Laboratory Manual Human Genome Mapping," M. Hori and Y. Nakamura, eds., Maruzen), screening the cosmid library to obtain positive clones containing DNA corresponding to the coding region of a desired protein, and screening the above cDNA library using the coding region DNA excised from the positive clones as a probe.

(3) The DNA of the present invention can also be chemically synthesized by the usual method, based on the nucleotide sequence of SEQ ID NO: 9, 11, or 27.

The present invention also relates to a recombinant vector comprising the DNA encoding the protein of the present invention. The recombinant vector of the present invention is not limited as long as it can be replicated and maintained or can autonomously replicate in various prokaryotic and/or eukaryotic hosts. The vector of the present invention includes plasmid vectors and phage vectors.

The recombinant vector can easily be prepared by ligating the DNA encoding the protein of the present invention with a vector for recombination available in the art (plasmid DNA and bacteriophage DNA) by the usual method. Specific examples of the vectors for recombination used are *E.coli*-derived plasmids such as pBR322, pBR325, pUC12, pUC13, and pUC19, yeast-derived plasmids such as pSH19 and pSH15, and *Bacillus subtilis*-derived plasmids such as pUB110, pTP5, and pC194. Examples of phages are a bacteriophage such as λ phage, and an animal or insect virus (pVL1393, Invitrogen) such as a retrovirus, vaccinia virus, and nuclear polyhedrosis virus.

An expression vector is useful for expressing the DNA encoding the protein of the present invention and for producing the polypeptide of the present invention. The expression vector is not limited as long as it expresses the gene encoding the polypeptide of the present invention in various prokaryotic and/or eukaryotic host cells and produces this protein. Examples thereof are pMAL C2, pEF-BOS (Nucleic Acids Res. Vol.18, p.5322 (1990)), pME18S (Experimental Medicine: SUPPLEMENT, "Handbook of Genetic Engineering" (1992)), and so on.

When bacteria, particularly *E. coli* are used as host cells, an expression vector is generally comprised of, at least, a promoter/operator region, an initiation codon, the DNA encoding the protein of the present invention, termination codon, terminator region, and replicon.

When yeast, animal cells, or insect cells are used as hosts, an expression vector is preferably comprised of, at least, a promoter, an initiation codon, the DNA encoding the protein of the present invention, and a termination codon. It may also comprise the DNA encoding a signal peptide, enhancer sequence, 5'- and 3'-untranslated region of the gene encoding the protein of the present invention, splicing junctions, polyadenylation site, selectable marker region, and replicon. The expression vector may also contain, if required, a gene for gene amplification (marker) that is usually used.

A promoter/operator region to express the polypeptide of the present invention in bacteria comprises a promoter, an operator, and a Shine-Dalgarno (SD) sequence (for example, AAGG). For example, when the host is *Escherichia*, it preferably comprises Trp promoter, lac promoter, recA promoter, $\lambda$ PL promoter, lpp promoter, tac promoter, or the like. Examples of a promoter to express the polypeptide of the present invention in yeast are PH05 promoter, PGK promoter, GAP promoter, ADH promoter, and so on. When the host is *Bacillus*, examples thereof are SL01 promoter, SP02 promoter, penP promoter and so on. When the host is a eukaryotic cell such as a mammalian cell, examples thereof are SV40-derived promoter, retrovirus promoter, heat shock promoter, and so on, and preferably SV-40 and retrovirus-derived one. As a matter of course, the promoter is not limited to the above examples. In addition, to use an enhancer is effective for expression.

A preferable initiation codon is, for example, a methionine codon (ATG).

The commonly used termination codon (for example, TAG, TGA, TAA, and so on) is illustrated as a termination codon.

Usually used natural or synthetic terminators are used as a terminator region.

A replicon means a DNA capable of replicating the whole DNA sequence in host cells, and includes a wild-type plasmid, an artificially modified plasmid (DNA fragment prepared from a wild-type plasmid), a synthetic plasmid, and so on. Examples of a preferable plasmids are pBR322 or its artificial derivatives (DNA fragment obtained by treating pBR322 with appropriate restriction enzymes) for *E. coli*, yeast 2 $\mu$ plasmid or yeast chromosomal DNA for yeast, and pRSVneo ATCC 37198, pSV2dhfr ATCC 37145, pdBPV-MMTneo ATCC 37224, pSV2neo ATCC 37149, etc. for mammalian cells.

An enhancer sequence, polyadenylation site, and splicing junction that are usually used in the art, such as those derived from SV40 can be also used.

A selectable marker usually used can be used according to the usual method. Examples thereof are resistance genes for antibiotics, such as tetracycline, neomycin, ampicillin, or kanamycin, and thymidine kinase gene.

Examples of a gene for gene amplification are dihydrofolate reductase (DHFR) gene, thymidine kinase gene, neomycin resistance gene, glutamate synthase gene, adenosine deaminase gene, ornithine decarboxylase gene, hygromycin-B-phophotransferase gene, aspartate transcarbamylase gene, etc.

The expression vector of the present invention can be prepared by continuously and circularly linking at least the above-mentioned promoter, initiation codon, DNA (gene) encoding the polypeptide of the present invention, termination codon, and terminator region, to an appropriate replicon. If desired, appropriate DNA fragments (for example, linkers, restriction sites generated with other restriction enzyme), can be used by the usual method such as digestion with a restriction enzyme or ligation using T4 DNA ligase.

Transformants of the present invention can be prepared by introducing the expression vector mentioned above into host cells.

Host cells used in the present invention are not limited as long as they are compatible with an expression vector mentioned above and can be transformed. Examples thereof are various cells such as wild-type cells or artificially established recombinant cells usually used in technical field of the present invention (for example, bacteria (*Escherichia* and *Bacillus*), yeast (*Saccharomyces*, *Pichia*, etc.), animal cells, or insect cells.

*E. coli* or animal cells are preferably used. Specific examples are *E. coli* (DH5$\alpha$, TB1, HB101, etc.), mouse-derived cells (COP, L, C127, Sp2/0, NS-1, NIH 3T3, etc.), rat-derived cells, hamster-derived cells (BHK, CHO, etc.), monkey-derived cells (COS1, COS3, COS7, CV1, Velo, etc.), and human-derived cells (Hela, diploid fibroblast-derived cells, myeloma, Namalwa, etc.).

An expression vector can be introduced (transformed (transduced)) into host cells by known method.

Transformation can be performed, for example, according to the method of Cohen et al. (Proc. Natl. Acad. Sci. USA, Vol.69, p.2110 (1972)), protoplast method (Mol. Gen. Genet., Vol.168, p.111 (1979)), or competent method (J. Mol. Biol., Vol.56, p.209 (1971)) when the hosts are bacteria (*E. coli*, *Bacillus subtilis*, etc.), the method of Hinnen et al. (Proc. Natl. Acad. Sci. USA, Vol.75, p.1927 (1978)), or lithium method (J. Bacteriol., Vol.153, p.163 (1983)) when the host is *Saccharomyces cerevisiae*, the method of Graham (Virology, Vol.52, p.456 (1973)) when the hosts are animal cells, and the method of Summers et al. (Mol. Cell. Biol., Vol.3, pp.2156-2165 (1983)) when the hosts are insect cells.

The protein of the present invention can be produced by cultivating transformants (in the following this term includes transductants) comprising an expression vector prepared as mentioned above in nutrient media.

The nutrient media preferably comprise carbon source, inorganic nitrogen source, or organic nitrogen source necessary for the growth of host cells (transformants). Examples of the carbon source are glucose, dextran, soluble starch, and sucrose, and examples of the inorganic or organic nitrogen source are ammonium salts, nitrates, amino acids, corn steep liquor, peptone, casein, meet extract, soy bean cake, and potato extract. If desired, they may comprise other nutrients (for example, an inorganic salt (for example, calcium chloride, sodium dihydrogenphosphate, and magnesium chloride), vitamins, antibiotics (for example, tetracycline, neomycin, ampicillin, kanamycin, etc.).

Cultivation is performed by a method known in the art. Cultivation conditions such as temperature, pH of the media, and cultivation time are selected appropriately so that the protein of the present invention is overproduced.

Specific media and cultivation conditions used depending on host cells are illustrated below, but are not limited thereto.

When the hosts are bacteria, actinomycetes, yeasts, filamentous fungi, liquid media comprising the nutrient source mentioned above are appropriate. The media with pH 5 to 8 are preferably used.

When the host is *E. coli*, examples of preferable media are LB media, and M9 media (Miller et al. Exp. Mol. Genet., Cold Spring Harbor Laboratory, p.431 (1972)). Using these media, cultivation can be performed usually at 14 to 43 °C for about 3 to 24 hours with aeration and stirring, if necessary.

When the host is *Bacillus*, cultivation can be performed usually at 30 to 40 °C for about 16 to 96 hours with aeration and stirring, if necessary.

When the host is yeast, examples of media are Burkholder minimal media (Bostian, Proc. Natl. Acad. Sci. USA, Vol.77, p.4505 (1980)). The pH of the media is preferably 5 to 8. Cultivation can be performed usually at 20 to 35°C for about 14to 144 hours with aeration and stirring, if necessary.

When the host is an animal cell, examples of media are MEM media containing about 5 to 20% fetal bovine serum (Science, Vol.122, p.501 (1952)), DMEM media (Virology, Vol.8, p.396 (1959)), RPMI1640 media (J. Am. Med. Assoc., Vol.199, p.519 (1967)), and 199 media (Proc. Soc. Exp. Biol. Med., Vol.73, p.1 (1950)). The pH of the media is preferably about 6 to 8. Cultivation can be performed usually at about 30 to 40°C for about 15 to 72 hours with aeration and stirring, if necessary.

When the host is an insect cell, an example of media is Grace's media containing fetal bovine serum (Proc. Natl. Acad. Sci. USA, Vol.82, p.8404 (1985)). The pH thereof is preferably about 5to 8. Cultivation can be performed usually at about 20 to 40°C for 15 to 100 hours with aeration and stirring, if necessary.

The protein of the present invention can be produced by cultivating transformants as mentioned above, in particular animal cells to overexpress the protein of the present invention on the surface of the cells. The protein of the present invention can be produced as a soluble protein fragment such as an extracellular region protein fragment by preparing the transformants as mentioned above using the DNA encoding the extracellular region and by cultivating the transformants to allow them to secrete the soluble polypeptide into the culture supernatant.

Namely, a culture filtrate (supernatant) is obtained by the method such as filtration or centrifugation of the obtained culture, and the protein of the present invention is purified and isolated from the culture filtrate by the usual method commonly used in order to purify and isolate a natural or synthetic protein.

Examples of the isolation and purification method are a method utilizing solubility, such as salting out and solvent precipitation method, a method utilizing the difference in molecular weight, such as dialysis, ultrafiltration, gel filtration, and sodium dodecyl sulfate-polyacrylamide gel electrophoresis, a method utilizing charges, such as ion exchange chromatography and hydroxylapatite chromatography, a method utilizing specific affinity, such as affinity chromatography, a method utilizing the difference in hydrophobicity, such as reverse phase high performance liquid chromatography, and a method utilizing the difference in isoelectric point, such as isoelectric focusing.

When the protein of the present invention exists in the periplasm or cytoplasm of cultured transformants, first, the fungus bodies or cells are harvested by the usual method such as filtration or centrifugation and suspended in appropriate buffer. After the cell wall and/or cell membrane of the cells and so on are disrupted by the method such as lysis with sonication, lysozyme, and freeze-thawing, the membrane fraction comprising the protein of the present invention is obtained by the method such as centrifugation or filtration. The membrane fraction is solubilized with a detergent such as Triton-X100 to obtain the crude extract. Finally, the polypeptide or the polypeptide fragment is isolated and purified from the crude extract by the usual method as illustrated above.

Using the DNA (cDNA or genomic DNA) prepared as mentioned above encoding a human-derived protein included in the protein of the present invention, a non-human transgenic animal in which the above human-derived DNA has been integrated into the endogenous locus of the animal. This non-human transgenic animal express and expresses and secretes the protein of the present invention encoded by the above DNA into its body. The present invention include such non-human transgenic animals.

The non-human tranegenic animal can be prepared according to the method as usually used for producing a transgenic animal (for example, see "Newest Manual of Animal Cell Experiment", LIC press, Chapter 7, pp.361-408, (1990)).

Specifically, for example, a transgenic mouse can be produced as follows. Embryonic stem cells (ES cells) obtained from normal mouse blastocysts are transformed with an expression vector in which the gene encoding human-derived polypeptide of the present invention and a marker gene (e.g., neomycin resistance gene) have been operably inserted. ES cells in which the gene encoding the human-derived polypeptide of the present invention has been integrated into the endogenous gene are screened in terms of the expression of the marker gene by the usual method. Then, the ES cells screened are microinjected into a fertilized egg obtained from another normal mouse (blastocyst) (Proc. Natl. Acad. Sci. USA, Vol.77, No.12, pp.7380-7384 (1980); U.S. Pat. No. 4,873,191).

The blastocyst is transplanted into the uterus of another normal mouse as the foster mother. Then, founder mice (progeny mice) are born from the foster mother mouse. By mating the founder mice with normal mice, heterogeneic transgenic mice are obtained. By mating the heterogeneic transgenic mice with each other, homogeneic transgenic mice are obtained according to Mendel's laws.

A so-called "knockout mouse" can be generated based on the information of the nucleotide sequence of the DNA encoding a mouse-derived protein that is included in the present invention. The "knockout mouse" of the present invention is a mouse wherein the endogenous gene encoding the mouse-derived protein of the present invention has been knocked out (inactivated). It can be prepared, for example, by positive-negative selection method in which homologous recombination is applied (U.S. Pat. No. 5,464,764; No. 5,487,992; No. 5,627,059; Proc. Natl. Acad. Sci. USA, Vol.86, pp.8932-8935 (1989);Nature,Vol.342,pp.435-438 (1989); etc.). This knockout mouse is also one embodiment of the present invention.

The "antibody" of the present invention can be a polyclonal antibody (antiserum) or a monoclonal antibody, and preferably a monoclonal antibody.

Specifically, it is an antibody reactive to (against, which binds to) the above-mentioned protein or its fragment of the present invention.

The antibody of the present invention can be natural antibodies obtained by immunizing mammals such as mice, rats, hamsters, guinea pigs, and rabbits with the protein of the present invention (natural, recombinant, synthetic, or cell-derived ones), its fragment, or transformants overexpressing the designed protein on the surface thereof prepared using recombinant DNA technology as described above on the cell surface. The antibody of the present invention also includes chimeric antibodies and humanized antibodies (CDR-grafted antibodies) that can be produced by recombinant DNA technology, and human antibodies that can be produced using human antibody-producing transgenic animals.

The monoclonal antibody includes those having any one isotype of IgG, IgM, IgA, IgD, or IgE. IgG or IgM is preferable.

The polyclonal antibody (antisera) or monoclonal antibody of the present invention can be produced by the known methods. Namely, a mammal, preferably, a mouse, rat, hamster, guinea pig, rabbit, cat, dog, pig, goat, horse, or cattle, or more preferably, a mouse, rat, hamster, guinea pig, or rabbit is immunized, for example, with an antigen mentioned above with Freund's adjuvant, if necessary. The polyclonal antibody can be obtained from the antiserum obtained from the animal so immunized. In addition, the monoclonal antibodies are produced as follows. Hybridomas are prepared from the antibody-producing cells obtaind from the animal so immunized and myeloma cells that are not capable of producing autoantibodies. The hybridomas are cloned, and clones producing the monoclonal antibodies showing the specific affinity to the antigen used for immunizing the mammal are screened.

Specifically, the monoclonal antibody can be produced as follows. Immunizations are performed by injecting or implanting once or several times the protein of the present invention, its fragment, cells expressing the protein and so on as mentioned above as an immunogen, if necessary, with Freund's adjuvant, subcutaneously, intramuscularly, intravenously, through the footpad, or intraperitoneally into a mouse, rat, hamster, guinea pig, or rabbit, preferably a mouse, rat, or hamster (including a transgenic animal generated so as to produce antibodies derived from another animal such as the transgenic mouse producing human antibody). Usually, immunizations are performed once to four times every one to fourteen days after the first immunization. Antibody-producing cells are obtained from the mammal so immunized in about one to five days after the last immunization.

Hybridomas that secrete a monoclonal antibody can be prepared by the method of Köhler and Milstein (Nature, Vol.256, pp.495-497 (1975)) and by its modified method. Namely, hybridomas are prepared by fusing antibody-producing cells contained in a spleen, lymph node, bone marrow, or tonsil obtained from the mammal immunized as mentioned above, preferably a spleen, with myelomas without autoantibody-producing ability, which are derived from, preferably, a mammal such as a mouse, rat, guinea pig, hamster, rabbit, or human, or more preferably, a mouse, rat, or human.

For example, mouse-derived myeloma P3/X63-AG8.653 (653, ATCC No. CRL1580), P3/NSI/1-Ag4-1 (NS-1), P3/X63-Ag8.U1 (P3U1), SP2/0-Ag14 (Sp2/0, Sp2), PAI, F0, or BW5147, rat-derived myeloma 210RCY3-Ag.2.3., or human-derived myeloma U-266AR1, GM1500-6TG-A1-2, UC729-6, CEM-AGR, D1R11, or CEM-T15 can be used as a myeloma used for the cell fusion.

Hybridoma clones producing monoclonal antibodies can be screened by cultivating hybridomas, for example, in microtiter plates and by measuring the reactivity of the culture supernatant in the well in which hybridoma growth is observed, to the immunogen used for the immunization mentioned above, for example, by enzyme immunoassay such as RIA and ELISA.

The monoclonal antibodies can be produced from hybridomas by cultivating the hybridomas *in vitro* or *in vivo* such as in the ascites fluid of a mouse, rat, guinea pig, hamster, or rabbit, preferably a mouse or rat, more preferably mouse and isolating the antibodies from the resulting the culture supernatant or ascites fluid of a mammal.

Cultivating hybridomas *in vitro* can be performed depending on the property of cells to be cultured, on the object of a test study, and on the various conditions of a cultivating method, by using known nutrient media or any nutrient media derived from known basal media for growing, maintaining, and storing the hybridomas to produce monoclonal antibodies in culture supernatant.

Examples of basal media are low, calcium concentration media such as Ham'F12 medium, MCDB153 medium, or low calcium concentration MEM medium, and high calcium concentration media such as MCDB104 medium, MEM medium, D-MEM medium, RPMI1640 medium, ASF104 medium, or RD medium. The basal media can contain, for example, sera, hormones, cytokines, and/or various inorganic or organic substances depending on the objective.

Monoclonal antibodies can be isolated and purified from the culture supernatant or ascites fluid mentioned above by saturated ammonium sulfate precipitation, euglobulin precipitation method, caproic acid method, caprylic acid method, ion exchange chromatography (DEAE or DE52), affinity chromatography using anti-immunoglobulin column or protein A column.

Furthermore, monoclonal antibodies can be obtaied in a large quantity by cloning a gene encoding a monoclonal antibody from a hybridoma, generating transgenec animals such as a rabbit, goat, sheep, or pig in which the gene encoding the monoclonal antibody is integrated in its endogenous gene using transgenic animal generating technique, and recovering the monoclonal antibody derived from the antibody gene from milk of the transgenic animal (Nikkei Science, No.4, pp.78-84 (1997)).

The "chimeric antibody" of the present invention is a monoclonal antibody prepared by genetic engineering, and specifically means a chimeric antibody such as mouse/human chimeric monoclonal antibody whose variable regions or the other regions are derived from mouse immunoglobulin and whose constant regions are derived from human immunoglobulin.

The constant region derived from human immunoglobulin has the amino acid sequence inherent in each isotype such as IgG, IgM, IgA, IgD, and IgE. The constant region of the recombinant chimeric monoclonal antibody of the present invention can be that of human immunoglobulin belonging to any isotype. Preferably, it is the constant region of human IgG.

The chimeric monoclonal antibody of the present invention can be produced, for example, as follows. Needless to say, the production method is not limited thereto.

A mouse/human chimeric monoclonal antibody can be prepared, referring to Experimental Medicine: SUPPLEMENT, Vol.1.6, No.10 (1988); and examined published Japanese patent application (JP-B) No. Hei 3-73280. Namely, it can be prepared by operably inserting CH gene (C gene encoding the constant region of H chain) obtained from the DNA encoding human immunoglobulin downstream of active VH genes (rearranged VDJ gene encoding the variable region of H chain) obtained from the DNA encoding a mouse monoclonal antibody isolated from the hybridoma producing the mouse monoclonal antibody, and CL gene (C gene encoding the constant region of L chain) obtained from the DNA encoding human immunoglobulin downstream of active VL genes (rearranged VJ gene encoding the variable region of L chain) obtained from the DNA encoding the mouse monoclonal antibody isolated from the hybridoma, into the same or different vectors so as for them to be expressed, following by transforming host cells with the expression vector, and then by cultivating the transformants.

Specifically, DNAs are first extracted from mouse monoclonal antibody-producing hybridomas by the usual method, digested with appropriate restriction enzymes (for example, EcoRI and HindIII), electrophoresed (using, for example, 0.7% agarose gel), and analyzed by Southern blotting. After an electrophoresed gel is stained, for example, with ethidium bromide and photographed, the gel is given with marker positions, washed twice with water, and soaked in 0.25 M HCl for 15 minutes. Then, the gel is soaked in 0.4 N NaOH solution for 10 minutes with gently stirring. The DNAs are transferred to a filter for 4 hours by the usual method. The filter is recovered and washed twice with 2xSSC. After the filter is sufficiently dried, it is baked at 75°C for 3 hours. After baking, the filter is treated with 0.1 x SSC/0.1% SDS at 65°C for 30 minutes. Then, it is soaked in 3 x SSC/0.1% SDS. The filter obtained is treated with prehybridization solution in a plastic bag at 65°C for 3 to 4 hours.

Next, $^{32}P$-labeled probe DNA and hybridization solution are added to the bag and reacted at 65°C about 12 hours. After hybridization, the filter is washed under appropriate salt concentration, reaction temperature, and time (for example, 2 x SSC-0.1% SDS, room temperature, 10 minutes). The filter is put into a plastic bag with a little 2 x SSC, and subjected to autoradiography after the bag is sealed.

Rearranged VDJ gene and VJ gene encoding H chain and L chain of a mouse monoclonal antibody are identified by Southern blotting mentioned above. The region comprising the identified DNA fragment is fractioned by sucrose density gradient centrifugation and inserted into a phage vector (for example, Charon 4A, Charon 28, λEMBL3, λEMBL4, etc.). *E. coli* (for example, LE392, NM539, etc.) is transformed with the phage vector to generate a genomic library. The genomic library is screened by plaque hybridization such as Benton-Davis method (Science, Vol.196, pp.180-182 (1977)) using appropriate probes (H chain J gene, L chain (κ) J gene, etc.) to obtain positive clones comprising rearranged VDJ gene or VJ gene. By making the restriction map and determining the nucleotide sequence of the clones obtained, it is confirmed that genes comprising the desired, rearranged VH (VDJ) gene or VL (VJ) gene are obtained.

Separately, human CH gene and human CL gene used for chimerization are isolated. For example, when a chimeric antibody with human IgG1 is produced, Cγ1 gene as a CH gene, and Cκ gene as a CL gene, are isolated. These genes can be isolated from human genomic library with mouse Cγ1 gene and mouse Cκ gene, corresponding to human Cγ1 gene and human Cκ gene, respectively, as probes, taking advantage of high homology between the nucleotide sequences of mouse immunoglobulin gene and that of human immunoglobulin gene.

Specifically, DNA fragments comprising human Cκ gene and an enhancer region are isolated from human λ Charon 4A HaeIII-AluI genomic library (Cell, Vol.15, pp.1157-1174 (1978)), for example, with a 3 kb HindIII-BamHI fragment of clone Ig146 (Proc. Natl. Acad. Sci. USA, Vol.75, pp.4709-4713 (1978)) and a 6.8 kb EcoRI fragment of clone MEP10 (Proc. Natl. Acad. Sci. USA, Vol.78, pp.474-478 (1981)) as probes. In addition, for example, after human fetal hepatocyte DNA is digested with HindIII and fractioned by agarose gel electrophoresis, a 5.9 kb fragment is inserted into λ788 and then human Cγ1 gene is isolated with the probes mentioned above.

Using mouse VH gene, mouse VL gene, human CH gene, and human CL gene so obtained, and taking promoter region and enhancer region into consideration, human CH gene is inserted downstream mouse VH gene and human CL gene is inserted downstream mouse VL gene into an expression vector such as pSV2gpt or pSV2neo with appropriate restriction enzymes and DNA ligase by the usual method. In this case, chimeric genes of mouse VH gene/human CH gene and mouse VL gene/human CL gene can be respectively inserted in the same expression vector or in different expression vectors.

Chimeric gene-inserted expression vector(s) thus prepared are introduced into myelomas that do not produce antibodies, for example, P3X63 • Ag8 • 653 cells or SP210 cells by protoplast fusion method, DEAE-dextran method, calcium phosphate method, or electroporation method. The transformants are screened by cultivating in media containing a drug corresponding to the drug resistance gene inserted into the expression vector and, then, cells producing desired chimeric monoclonal antibodies are obtained.

Desired chimeric monoclonal antibodies are obtained from the culture supernatant of antibody-producing cells thus screened.

The "humanized antibody (CDR-grafted antibody)" of the present invention is a monoclonal antibody prepared by genetic engineering and specifically means a humanized monoclonal antibody wherein a portion or the whole of the complementarity determining regions of the hypervariable region are derived from the complementarity determining regions of the hypervariable region from a mouse monoclonal antibody, the framework regions of the variable region are derived from the framework regions of the variable region from human immunoglobulin, and the constant region is derived from human a constant region from immunoglobulin.

The complementarity determining regions of the hypervariable region exists in the hypervariable region in the variable region of an antibody and means three regions which directly and complementary binds to an antigen (complementarity-determining residues, CDR1, CDR2, and CDR3). The framework regions of the variable region means four comparatively conserved regions lying upstream, downstream or between the three complementarity determining regions (framework region, FR1, FR2, FR3, and FR4).

In other words, a humanized monoclonal antibody means that in which the whole region except a portion or the whole of the complementarity determining regions of the hypervariable region of a nonhuman mammal-derived monoclonal antibody have been replaced with their corresponding regions derived from human immunoglobulin.

The constant region derived from human immunoglobulin has the amino acid sequence inherent in each isotype such as IgG (IgG1, IgG2, IgG3, IgG4), IgM, IgA, IgD, and IgE. The constant region of a humanized monoclonal antibody in the present invention can be that from human immunoglobulin belonging to any isotype. Preferably, it is the constant region of human IgG. The framework regions of the constant region derived from human immunoglobulin are not particularly limited.

The humanized monoclonal antibody of the present invention can be produced, for example, as follows. Needless to say, the production method is not limited thereto.

For example, a recombinant humanized monoclonal antibody derived from mouse monoclonal antibody can be prepared by genetic engineering, referring to unexamined Japanese patent publication (JP-WA) No. Hei 4-506458 and unexamined Japanese patent publication (JP-A) No. Sho 62-296890. Namely, at least one mouse H chain CDR gene and at least one mouse L chain CDR gene corresponding to the mouse H chain CDR gene are isolated from hybridomas producing mouse monoclonal antibody, and human H chain gene encoding the whole regions except human H chain CDR corresponding to mouse H chain CDR mentioned above and human L chain gene encoding the whole region except human L chain CDR correspond to mouse L chain CDR mentioned above are isolated from human immunoglobulin genes.

The mouse H chain CDR gene(s) and the human H chain gene(s) so isolated are operably inserted into an appropriate vector so that they can be expressed. Similarly, the mouse L chain CDR gene(s) and the human L chain gene(s) are operably inserted into another appropriate vector so that they can be expressed. Alternatively, the mouse H chain CDR gene(s)/human H chain gene(s) and mouse L chain CDR gene(s)/human L chain gene(s) can be operably inserted into the same expression vector so that they can be expressed. Host cells are transformed with the expression vector thus prepared to obtain transformants producing humanized monoclonal antibody. By cultivating the transformants, desired humanized monoclonal antibody is obtained from culture supernatant.

The "human monoclonal antibody" of the present invention is immunoglobulin in which the entire regions comprising the variable and constant region of H chain, and the variable and constant region of L chain constituting immunoglobulin are derived from the gene encoding human immunoglobulin.

The human antibody can be produced in the same way as the production method of polyclonal or monoclonal antibodies mentioned above by immunizing, with an antigen, a transgenic animal which for example, at least human immunoglobulin gene(s) have been integrated into the locus of a non-human mammal such as a mouse by the usual method. For example, a transgenic mouse producing human antibodies is prepared by the methods described in Nature Genetics, Vol.15, pp.146-156 (1997); Nature Genetics, Vol.7, pp.13-21 (1994); JP-WA Nos. Hei 4-504365, International patent publication No. WO94/25585; Nikkei Science, No.6, pp.40-50 (1995); Nature, Vol.368, pp.856-859 (1994); and JP-WA No. Hei 6-500233.

The "portion of an antibody" used in the present invention means a partial region of the antibody, preferably monoclonal antibody of the present invention as mentioned above, and specifically, means $F(ab')_2$, Fab', Fab, Fv (variable fragment of antibody), sFv, dsFv (disulfide stabilized Fv), or dAb (single domain antibody) (Exp. Opin. Ther. Patents, Vol.6, No.5, pp.441-456 (1996)).

"$F(ab')_2$" and "Fab'" can be produced by treating immunoglobulin (monoclonal antibody) with a protease such as pepsin and papain, and means an antibody fragment generated by digesting immunoglobulin near the disulfide bonds existing between the hinge regions in each of the two H chains. For example, papain cleaves IgG upstream of the disulfide bonds existing between the hinge regions in each of the two H chains to generate two homologous antibody fragments in which an L chain composed of VL (L chain variable region) and CL (L chain constant region), and an H chain fragment composed of VH (H chain variable region) and CHγ1 (γ1 region in the constant region of H chain) are connected at their C terminal regions through a disulfide bond. Each of such two homologous antibody fragments is called Fab'. Pepsin also cleaves IgG downstream of the disulfide bonds existing between the hinge regions in each of the two H chains to generate an antibody fragment slightly larger than the fragment in which the two above-mentioned Fab' are connected at the hinge region. This antibody fragment is called $F(ab')_2$.

The "cells producing a monoclonal antibody reactive with proteins or fragments thereof" of this invention mean any cells producing the above-described monoclonal antibody of this invention.

Specific examples include (1) non-human mammal-derived B cells producing a monoclonal antibody reactive with proteins of this invention or fragments thereof, which is obtained by immunizing a non-human mammal with proteins or fragments thereof of this invention or cells producing the proteins, etc. as described above, (2) the above-described hybridomas (fused cells) prepared by fusing antibody producing B cells obtained as described above with myelomas derived from mammals, or (3) monoclonal antibody producing transformants obtained by transforming other cells than the B cells and hybridomas with genes (either the heavy chain-encoding gene or the light chain encoding gene, or both) encoding the monoclonal antibody isolated from the monoclonal antibody producing B cells or hybridomas.

The monoclonal antibody producing transformants of (3) mean recombinant cells producing a recombinant product of the monoclonal antibody produced by B cells of (1) or hybridomas of (2). These recombinant monoclonal antibody producing cells can be produced in the same manner as for the above-described chimeric monoclonal antibody and humanized antibody.

The "pharmaceutical composition" of the present invention comprises any one of the protein, its fragment, antibody, or its portion of the present invention as defined above; and a pharmaceutically acceptable carrier.

The "pharmaceutically acceptable carrier" includes a excipieut, a diluent, an expander, a decomposition agent, a stabilizer, a preservative, a buffer, an emulsifier, an aromatic, a colorant, a sweetener, a viscosity increasing agent, a flavor, a solubility increasing agent, or other additives. Using one or more of such carriers, a pharmaceutical composition can be fomulated into tablets, pills, powders, granules, injections, solutions, capsules, troches, elixirs, suspensions, emulsions, or syrups. The pharmaceutical composition can be administered orally or parenterally. Other forms for parenteral administration include a solution for external application, suppository for rectal administration, and pessary, prescribed by the usual method, which comprises one or more active ingredient.

The dosage can vary depending on the age, sex, weight, and symptom of a patient, effect of treatment, administration route, period of treatment, or the kind of active ingredient (protein or antibody mentioned above) contained in the pharmaceutical composition. Usually, the pharmaceutical composition can be administered to an adult in a dose of 10 μg to 1000 mg (or 10 μg to 500 mg) per one administration. Depending on various conditions, the dosage less than that mentioned above may be sufficient in some cases, and the dosage more than that mentioned above may be necessary in other cases.

In particular, the injection can be produced by dissolving or suspending the antibody in a non-toxic, pharmaceutically acceptable carrier such as physiological saline or commercially available distilled water for injection with adjusting a concentration to 0.1 μg antibody/ml carrier to 10 mg antibody/ml carrier. The injection thus produced can be administered to a human patient in need of treatment in a dose of 1 μg to 100 mg/kg body weight, preferably 50 μg to 50 mg/kg body weight once or more times a day. Examples of administration route are medically appropriate administration routes such as intravenous injection, subcutaneous injection, intradermal injection, intramuscular injection, or intraperitoneal injection, preferably intravenous injection.

The injection can also be prepared into a non-aqueous diluent (for example, propylene glycol, polyethylene glycol, vegetable oil such as olive oil, and alcohol such as ethanol), suspension, or emulsion.

The injection can be sterilized by filtration with a bacteria-non-penetrated filter, by mixing bacteriocide, or by irradiation. The injection can be produced in the form that is prepared upon use. Namely, it is freeze-dried to be a sterile solid composition, and can be dissolved in sterile distilled water for injection or another solvent before use.

The pharmaceutical composition of the present invention is extremely useful for preventing or treating morbidities and disorders (for example, atherosclerosis, diabetic angiopathy, bacterial infection, etc.) caused by intracellular stresses, xenobiotics or denatured proteins generated in the body.

Brief Description of the Drawings

Fig. 1 schematically shows the difference in the protein primary structures of human CSR splicing variants, CSR1, CSR2, and CSR3.

A, B, B'', C, D, E, E'' and F represent exon units, and numerals above each exon unit indicate approximate nucleotide numbers of DNAs encoding the corresponding exons.

Fig. 2 schematically represents the protein secondary structures of human CSR1 and CSR2.

Numerals in the figure indicate the amino acid numbers counted from the N-terminus.

Fig. 3 schematically represents the protein secondary structure of macrophage scavenger receptor.

Fig. 4 shows the protein primary structure of human CSR1.

Symbols (●, * and #) in the row below amino acids indicate potential glycosylation sites (●), phosphorylation sites (*) and α-helical coiled-coil domains (#), respectively.

In Fig. 5 panel (a) shows the locus of human CRS gene on the human chromosome analyzed by FISH method. Two white dots (about 0.3 mm) pointed by arrows indicate the 8q21 site where CSR gene is located. Panel (b) shows chromosome mapping of human cells at mitosis metaphase by R-band differential staining.

Fig. 6 represents human CSR mRNAs expression in various human tissues detected by Northern blot analysis.

Fig. 7 represents gel electrophoretogram of RT-PCR-amplified CSR cDNAs in the analysis of expression regulation of CSR gene by p53 protein.

Ws indicate test results using human colon cancer cell line SW480 transformed with the wild-type p53 gene, and Ms those using the SW480 cells transformed with the mutant p53 gene. Numerals in the upper row indicate the time elapsed prior to RNA extraction.

Fig. 8 shows gel electrophoretogram of RT-PCR-amplified CSR cDNAs in the analysis of expression of CSR gene in various cells.

Lane 1 represents the test result using the normal human fibroblast NHDF4042 cell as a host cell, lane 2 the test result using human cervical cancer-derived epithelium-like cell line HeLa as a host cell, lane 3 the test result using the human lung cancer cell line H1299 as a host cell, lane 4 the test result using the human esophageal cancer cell line TE10 as a host cell, lane 5 the test result using the human neuroglioblastoma cell line T98G as a host cell, lane 6 the test result using the human neuroglioblastoma cell line U87MG as a host cell, lane 7 the test result using the human tumor cell line SKBR3 as a host cell, and lane 8 the test result using the human colon cancer cell line SW480 as a host cell.

Ws indicate cells expressing the wild-type p53, and Ms cells expressing the mutant p53 gene.

Fig. 9 represents CSR mRNA expression detected by Northern blotting in the analysis of effects of serum starvation on the transcription regulation of CSR gene.

A set of two lanes represent, from the left, the test results using the human normal fibroblast cell line NHDF4042, human colon cancer cell line SW480, human neuroglioblastoma cell line T98G, and human esophageal cancer cell line TE13. Plus (+) and minus (—) represent the results with and without serum starvation, respectively.

Fig. 10 represnts CSR mRNAs expression detected by Northern blotting in the analysis of transcription regulation of CSR gene by ultraviolet irradiation stress.

Numerals above the photographs indicate the intensity of ultraviolet irradiation.

Fig. 11 represents CSR mRNA expression detected by Northern blotting in the analysis of transcription regulation of CSR gene by hydrogen peroxide stress.

Numerals above the photographs indicate concentrations of hydrogen peroxide.

Fig. 12 represents CSR mRNA expression detected by Northern blotting in the analysis of transcription regulation of CSR gene by heat shock stress.

Numerals above the photographs indicate the time elapsed after applying heat shock.

In Fig. 13 panel (a) shows the cytoplasmic distribution of CSR proteins analyzed by immunocytochemical staining using the anti-HA epitope polyclonal antibody.

Panel (b) shows the nuclei localized by staining with DAPI.

Fig. 14 shows suppressive effects of CSR on cell death caused by ultraviolet irradiation stress.

Fig. 15 shows suppressive effects of CSR on cell death caused by hydrogen peroxide stress.

In Fig. 16 panel (a) represents the primary protein structure of human CSR1. Panel (b) represents the primary protein structure of human CSR2.

The numeral (451) at the left end of amino acid sequence described in panel (b) indicates the amino acid residue (451) in the human CSR2 protein, and that the amino acid sequence at positions 1 - 450 of CSR2 is identical to that of the human CSR1 protein in panel (a).

Symbols (●, △, *, ◯ and #) in the row below amino acids indicate putative phosphorylation sites by casein kinase 2 (●), protein kinase C (△) and tyrosine kinase 2 (*), heme binding sites ( ◯ ), and microbody (peroxisome) C-terminal targeting signal site, respectively.

The putative $\alpha$-coiled coil domain is enclosed by darkish boxes.

The putative N-terminal transmembrane region is enclosed by clear box.

The N-glycosylation sites are enclosed by elliptical-like boxes (boxes with round corners).

The leucine zipper motif site is double-underlined (repeated leucine residues are described in slightly large letters).

The collagen-like domain having structure of G-X-Y repeats is enclosed by bold line box.

Fig. 17 schematically shows the structure of genomic DNA encoding human CSR protein, and difference in primary structures of splicing variants, CSR1 and CSR2 proteins.

Regions indicated by solid vertical bars 1, 2, 3, 4, 5, 6' and 6" represent exon units. The nucleotide sequence shown in this figure indicates the presence of p53-binding region in the second intron.

Fig. 18 represents gel elctrophoretograms of nuclear extracts from transformants under various conditions in EMSA (electro-mobility shift assay) for analyzing effects of the p53 protein on the expression regulation of CSR gene.

Fig. 19 represents gel elctrophoretograms of CSR cDNAs amplified by RT-PCR for analyzing the expression of CSR gene in various cells.

Fig. 20 (a) shows CSR mRNA expression detected by Northern blotting in the analysis of the transcription regulation of CSR gene by the ultraviolet irradiation stress. Numerals in the upper row indicate the intensity of ultraviolet irradiation. Fig. 20 (b) shows CSR mRNA expression detected by Northern blotting in analysis for the transcription regulation of CSR gene by the hydrogen peroxide stress. Numerals in the upper row indicate the concentration of hydrogen peroxide.

Fig. 21 represents CSR mRNA expression detected by Northern blotting in the analysis for the transcription regulation of CSR gene by adriamycin (ADR). Numerals in the upper row indicate the concentration of adriamycin.

Fig. 22 represents CSR mRNA expression detected by Northern blotting in the analysis for effects of antioxidant on the transcription induction of CSR gene by intracellular stresses due to ultraviolet irradiation and hydrogen peroxide.

"—NAC" and "+NAC" indicate the results without and with preincubation in the presence of NAC, respectively. "—" and "UV" indicate the results without and with ultraviolet irradiation, respectively. Similarly, "—" and "$H_20_2$" indicate the results without and with hydrogen peroxide stress, respectively.

Fig. 23 represents gel elctrophoretic patterns of RT-PCR amplified CSR cDNAS in the analysis for regulation of CSR gene expression by various reagents.

Panel (a) shows the results obtained using DEM, (b) PMA (TPA), (c) sodium azide, (d) sodium arsenite, (e) GSNO, and (f) SNP.

Fig. 24 represents gel elctrophoretic patterns of various cDNAs amplified by RT-PCR in the analysis of expression regulation of various genes exerted by hemin and hydrogen peroxide.

Panel (a) shows the time-course induction of gene expression by hemin, and panel (b) by hydrogen peroxide. Numerals in the upper rows indicate the time elapsed prior to obtaining RNAs.

In Fig. 25 (a) shows the cytoplasmic distribution of CSR proteins analyzed by immunocytochemical staining of cells transformed with an expression vector containing no CSR cDNA insert using an anti-HA epitope polychlonal antibody.

(b) shows the nuclear loci of cells transformed with an expression vector containing no CSR cDNA insert detected by DAPI staining method.
(c) shows the cytoplasmic distribution of CSR proteins analyzed by immunocytochemical staining of cells transformed with an expression vector expresssing CSR cDNA using an anti-HA epitope polychlonal antibody.
(d) shows the nuclear loci of cells transformed with an expression vector expressing CSR cDNA detected by DAPI staining method.
(e) shows the cytoplasmic distribution of CSR proteins analyzed by immunocytochemical staining of cells transformed with an expression vector expressing CSR cDNA using an anti-golgi-58K protein antibody.
(f) shows the nuclear loci of cells transformed with an expression vector expressing CSR cDNA detected by DAPI staining method.
(g) shows the cytoplasmic distribution of CSR proteins analyzed by immunocytochemical staining of cells transformed with an expression vector expresssing CSR cDNA under the oxidative stress due to hydrogen peroxide using an anti-golgi-58K protein antibody.
(h) shows the nuclear loci of cells transformed with an expression vector expressing CSR cDNA under the oxidative stress due to hydrogen peroxide detected by DAPI staining method.

Fig. 26 shows suppressive effects of CSR proteins on cell death caused by ultraviolet irradiation stress.
Fig. 27 shows changes in the amount of intracellular reactive oxygen species measured with a flow cytometer.
(a), (b), (c), and (d) show changes in the amount of intracellular reactive oxygen species in the control H1299-vector group, the H1299-CSR1 group, the H1299-CSR2 group, and the CSR1/CSR2 group, respectively.
Fig. 28 shows time-course morphological changes of cells induced by hydrogen peroxide.

(a) shows the morphology of cells in the H1299-vector group with no addition of hydrogen peroxide,
(b) shows the morphology of cells in the H1299-vector group immediately after the addition of hydrogen peroxide,
(c) shows the morphology of cells in the H1299-vector group cultured for 1 h after the addition of hydrogen peroxide,
(d) shows the morphology of cells in the H1299-vector group cultured for 3 h after the addition of hydrogen peroxide,
(e) shows the morphology of cells in the CSR1/CSR2 group with no addition of hydrogen peroxide,
(f) shows the morphology of cells in the CSR1/CSR2 group immediately after the addition of hydrogen peroxide,
(g) shows the morphology of cells in CSR1/CSR2 group cultured for 1 h after the addition of hydrogen peroxide, and
(h) shows the morphology of cells in CSR1/CSR2 group cultured for 3 h after the addition of hydrogen peroxide.

Fig. 29 schematically represents the structure of targeting vector for producing mouse CSR gene knockout mice.

## Best Mode for Carrying out the Invention

The present invention is illustrated in detail below with reference to examples, but is not to be construed as being limited thereto.

## Example 1 Preparation of human genomic DNA fragment interacting with (binding to) tumor inhibitory protein p53

Human genomic DNA fragment binding to p53 was obtained by using a yeast expression system in which human genomic DNA cloned upstream of a selective reporter gene is expressed concurrently with tumor inhibitory protein p53 (Tokino, et al., Human Molecular Genetics, Vol. 3, No. 9, 1537-1542, 1994).

1-1 Construction of HIS3 reporter vector

GAL1 promoter in wild-type tumor inhibitory gene p53 expression vector pRS314-SN (Nigro, J. M., et al., Mol. Cell. Biol., Vol. 12, 1357-1365, 1992) was replaced with PGK promoter to construct plasmid pRS314-PGK-p53. The *Xho*I-*Bam*HI fragment containing p53 cDNA cleaved from pRS314-SN was subcloned at the *Sal*I and *Bam*HI sites of plasmid pPGK (Poon, D., et al., Mol. Cell Biol., Vol. 11, 4809-4821, 1991) to construct plasmid pPGK-SN. The *Kpn*I-*Sac*I fragment of pPGK-SN containing PGK promoter, p53 cDNA, and PGK terminator was inserted into plasmid pRS314 (Sikorski, R. S., et al., Genetics, Vol. 122, 19-27, 1989) to construct plasmid pRS314-PGK-p53.

1-2 Construction of human genomic library

Human DNA was completely digested with a restriction enzyme *Mbo*I, and cloned at the *Bam*HI site of HIS3 promoter plasmid pBM947 (Wilson, T. E., et al., Science, Vol.252, 1296-1300, 1991). The ligated plasmid was introduced into *Escherichia coli* DH 10B (BRL) by electroporation and the strain was spread (1 x $10^5$ colonies/plate) on agar medium plates with ampicillin (180 plates). The strain was collected from the plates and DNA was purified from the plasmid library by the caesium chloride ultracentrifugation. The obtained DNA was used for the yeast transformation by PEG/lithium acetate method (Gietz, D., et al., Nucleic Acids Res., Vol. 20, 1425, 1992).

Yeast YPH681 containing the above-mentioned p53 expression vector pRS314-PGK-p53 was spread on 100 ml of a SD-Trp medium and incubated overnight (1 x $10^7$ cells/ml). The medium was washed by sterilized water and resuspended with 10 ml of sterilized water. The cells were washed with 10 ml of LiAc/TE (0.1 M LiAc at pH 7.5, 10 mM Tris hydrochloric acid at pH 7.5, and 1 mM EDTA) and resuspended with LiAc/TE (1 x $10^9$ cells/ml). Yeast cell suspension solution (0.2 ml) was mixed with 2 μg of DNA to be used for transformation (1 μg/μl of 1 x TE), 100 μg of single stranded salmon sperm DNA (10 μg/μl), and 0.6 ml of 40% sterilized PEG solution (40% polyethylene glycol 4000/LiAc/TE). The obtained cell mixture was incubated at 30°C for 30 min while vigorously being shaken, heated at 42°C for 15 min and centrifuged to obtain the pellet. The cellular pellet was resuspended with 1 X TE (0.2 ml), spread on a SD-trp-ura plate, and incubated at 30°C for three days. The reporter plasmid library (1 mg) was transformed and spread onto 500 plates. Each plate contains about 2 x $10^4$ clones. The yeast transformants were collected from the plates and pooled for each 10 plates apiece, resuspended in a mixture of 65% glycerol (v/v), 0.1M magnesium sulfate and 25 mM Tris hydrochloric acid (pH 8), and stored at -80°C.

1-3 Screening of library

Yeast cell diluted (0.1 ml) to reach 10 at the absorbance of 600 nm was spread onto the selective SD-trp-ura-his plate and cultured at 30°C for 5 days. A small amount of the transformants was spread on a non-selective SD-trp-ura plate to determine the number of living yeast cells. Each HIS+ positive cell was cultured in the SD-ura medium and the extracted DNA was introduced into Ura-deficient *E. coli* strain KC8. The HIS3 activity in the yeast of the collected reporter plasmids was examined in the presence or absence of the p53 expression vector. The sequences of the p53 dependent clones were analyzed using two primers adjacent to the cloning sites; (1) a primer derived from GAL promoter (SEQ ID NO. 1) (Johnston, M., Mol. Cell. Biol., Vol. 4, 1440-1448, 1984) and (2) that derived from the plasmid pBR322 (SEQ ID NO. 2).

1-4 β-Galactosidase assay

In this experiment β-Galactosidase assay was conducted as follows. Each of the reporter plasmid and expression vector was introduced into yeast EGY048 strain (Gyuris, J., et al., Cell, Vol. 75, 791-803, 1993) to obtain the yeast transformants and cultured in the synthetic medium containing raffinose as a carbon source at 30°C overnight. The culture medium was diluted with the medium containing 1% raffinose and 1% galactose to reach 0.1 at the absorbance of 600 nm, and cultured for 19 hours to carry out β-galactosidase assay (Kern, S. E., et al., Science, Vol. 256, 827-830, 1992).

1-5 Screening of human library

Human genomic DNA clones (1.8 x $10^7$ cells) were inserted into the HIS3 reporter vector to prepare a plasmid library. Yeast was transformed with this first generation clones to obtain 1 x $10^7$ transformants, and separated into 50 pools each containing about 2 x $10^5$ individual cells. About 5 x $10^5$ cells from each pool were spread onto the medium lacking histidine to select clones containing DNA reacting to tumor inhibitory protein p53. In each pool from 20 to 93 (about 50 clones on an average) histidine auxotrophic clones were detected. About 20 clones were randomly selected from each pool to be used for preparing the above-mentioned reporter DNA (refer to 1-1 and 1-2 for details).

Each plasmid thus obtained was used to transform yeast strains containing a p53 expression vector or a control vacant vector without p53 gene. Among examined 942 clones, 273 clones (29%) were p53 dependent. The 273 p53 reactive clones were analyzed by the digestion with restriction enzymes and the nucleotide sequences. The 273 clones contained 57 different types of *Mbo*I digested fragments. Nucleotide sequences of inserts in each clone were determined using the sequences near (just before and just after) the cloning site as primers (refer to 1-3 for details). Among analyzed 57 clones, 46 clones contained 2 copies of 10bp fragment similar to the reported consensus p53 binding sequence (El-Deiry, W.S., et al., Nature Genetics, Vol. 1, 45-49, 1992).

Example 2 Preparation of cosmid library with human genomic DNA

A cosmid library used in this invention was prepared following "Laboratory Manual Human Genome Mapping" (M. Hori and Y. Nakamura, eds., Maruzen, pp. 41-48). The method is briefly described below.

The *Bam*HI site of cosmid vector pWE15 (Wahl, GM., Proc. Natl. Acad. Sci. USA., Vol. 84, 2160-2164, 1987) was filled in using the Klenow enzyme and converted to the *Xho*I site using an oligonucleotide linker (5' CCTCGAGG 3') to construct the modified cosmid vector pWEX15. The vector was digested with *Xho*I and partially filled in with the Klenow enzyme, dCTP, and dTTP.

Human chromosome genomic DNA was digested with *Sau*3AI and fractionated by sucrose concentration gradient centrifugation to obtain genomic DNA fragments. The DNA fragments were filled in with the Klenow enzyme, dATP and dGTP.

The vector DNA (1 μg) and the genomic DNA (1 μg) were ligated in a mixture of 10 x ligation buffer (0.5 M Tris hydrochloric acid buffer at pH 7.5), 100 mM magnesium chloride, 100 mM dithiothreitol, 500 μg/ml bovine serum albumin), 20 mM ATP, 50% polyethylene glycol 8000, 1M sodium chloride, T4 DNA ligase (Boehringer), and distilled water, and *in vitro*-packaged using GIGAPACK II GOLD (Stratagene).

Example 3 Preparation of p53-reactive human genomic DNA fragment-containing fragment with exon sequence by screening human genomic DNA cosmid library

The human genomic DNA insert (SEQ ID NO: 3) in a clone of p53-reactive human genomic DNA fragment obtained and sequenced in Example 1 (clone No. 24, p53-binding consensus-like sequence: CAACTTGTCT, refer to Human Molecular Genetics, Vol. 3, No. 9, 1537-1542, 1994) was radio-labeled to serve as a probe for hybridization. The above-mentioned human DNA cosmid library was screened using the probe by the standard method to obtain 25 positive clones.

To amplify exon sequences in the cosmid clones, each clone was digested with *Bam*HI and *Bgl*II and ligated with exon trapping vector pSPL3 (GIBCO-BRL). Using marathon cDNA amplification kit (Clontech), 5' RACE-PCR (Rapid Amplification Ends-PCR) and 3' RACE-PCR (Proc. Natl. Aca. Sci. USA, Vol. 85, 8998-9002, 1998 and "Gene Amplification PCR Method, Basis and New Approaches" Kyoritsu, 1992) were conducted to confirm the insertion of 29 types of exon-like sequences in 18 cosmid clones.

Example 4 Preparation of cDNA fragment encoding human cellular sensor-related protein (CSR protein, scavenger receptor) by screening human cDNA library

Whole genomic DNA sequences in cosmid 2 (clone No. 2), one of the cosmid clones in which the exon-like fragment was inserted, was analyzed using 377 ABI Automatic Sequencer (Perkin Elmer). One of the analyzed exon sequences was named 2E1 (SEQ ID NO: 4). Exon sequence sites in the genomic DNA sequences were estimated using computer software Grail2 and Hexon.

Reverse transcription PCR (RT-PCR) method ("PCR and its Application" in "Experimental Medicine, Supplement", Vol. 8, No. 9, 1990, Gene Amplification PCR Method, Basis and New Approaches, Kyoritsu, 1992) revealed that all of the estimated cDNA sequences were transcribed to mRNA and linked to each other. The exon cDNA fragment was radio-labeled to serve as a probe for hybridization. 5 x $10^5$ Plaques in a human fetal brain derived random primed cDNA library (Stratagene) were screened using the probe containing the p53 binding site to obtain positive clones. From the positive clones, human cDNA fragments were cleaved to analyze the sequences. The clone contains cDNA fragment encoding cellular sensor-related (CSR) protein (also called as a scavenger receptor) of the invention.

Example 5 Preparation of full-length (or long chain) cDNA encoding human CSR protein

Whole mRNA derived from human fetal brain was converted into cDNA by random priming with d (N) 10 (Boehringer Mannheim) in the presence of M-MuLV reverse transcriptase (200 units/μl, GIBCO-BRL) to prepare a human long chain cDNA library. The long chain cDNA library was screened by the standard method using a human cDNA fragment cleaved from the above-mentioned positive clones as a probe to obtain four positive clones. Multiple long chain cDNAs encoding human CSR protein were cleaved from the positive clones and sequenced. The cDNA encoding CSR protein was then amplified by RT-PCR method using EC1 (SEQ ID NO: 5) and EC2a (SEQ ID NO: 6) and EC2b (SEQ ID NO: 7) as primers.

RT-PCR was conducted 30 to 40 cycles in cDNA mixture solution (25 μl) using Thermocycler (Perkin Elmer Cetus). The cDNA mixture contains 40 ng of cDNA, 1 x PCR buffer (6.7 mM Tris, pH 8.8, 16.6 mM ammonium sulfate, 6.7 μM EDTA, and 10 mM β-mercaptoethanol), 25 pmol sense primer, 25 pmol antisense primer, 0.5 U of Taq DNA polymerase (TAKARA), 250 μM deoxynucleotides, 10% DMSO, and 7.6 mM magnesium chloride. Each PCR cycle was comprised of the reactions at 94°C for 30 sec, at 55°C for 30 sec, and at 72°C for 1 min. The final PCR step was conducted at 72°C for 5 min. The resulting PCR product was applied to the 3% agarose gel electrophoresis, transferred to N+ nylon membrane (PALL), and hybridized with the internal oligonucleotide probe F12 (SEQ ID NO: 8).

cDNA was amplified by 5' RACE-PCR and 3' RACE-PCR with cDNA Amplification Kit (Clonetech) using the cDNA obtained above as a template to identify 5' and 3' ends in each long chain cDNA sequence. Isolation of two full-length cDNA encoding human CSR (proteins encoded by each cDNA were named CRS 1 protein and CSR 2 protein, hereafter abbreviated as CSR1 and CSR2) and one long chain cDNA assumed as the full-length cDNA (the coding protein was named CSR3 protein, hereafter simply referred to as CSR3) was confirmed.

SEQ ID NO: 9 and NO: 10 show cDNA sequence of CRS1 and its deduced amino acid sequence, respectively, SEQ ID NO: 11 and NO: 12 those of CRS2, and SEQ ID NO. 13 and NO. 14 those of CSR3.

The sizes of the gene comprising CSR1 cDNA and its open reading frame (ORF) were about 4.0 kb and 1.8 kb, respectably, to encode 606 amino acids. The sizes of the gene comprising CSR2 cDNA and its ORF were about 1.9 kb and 1.4 kb, respectably, to encode 466 amino acids.

Comparison of nucleotide sequences of these three CSRs confirmed that these are transcripts obtained by alternative splicing. For example, the first to 457th codons of CSR1 and CSR2 were identical. Fig. 1 schematically shows structural differences in these three splicing mutants.

On the other hand, human CSR genomic DNA is about 90 kb, composed of 6 or more coding exons, and the partial sequence (SEQ ID NO: 20) existing in the second intron sequence (between exon A and exon B in Fig. 1) was considered to fit with the reported p53 binding sequence (nucleotide sequence composed of 2 copies of symmetry 10 mers [5' RRRC (A/T) (T/A) GYYY 3'' (R is purine, and Y is prymidine); El-Deiry, W.S., et al., Nature Genetics, Vol. 1, 45-49, 1992).

As shown in Fig. 1, it was assumed that CSR1 was composed of 6 exons, A, B, C, D, E, and E'', CSR 2 of 6 exons, A, B, C, D, E, and F, and CSR 3 of at least 4 exons, C, D, E, and E'' (CSR3 may comprise B and B'' as exons because coding sequences corresponding to B and B'' at 5' end of CSR3 cDNA were found).

Fig. 17 shows that CSR1 genomic DNA is composed of 6 exons, exons 1, 2, 3, 4, 5, and 6'. Genomic DNA of CSR2 was composed of 6 exons, exons 1, 2, 3, 4, 5, and 6'', in which exon 6' was replaced by exon 6'' by splicing.

Amino acid homology search using various computer softwares did not detect any known proteins homologous to the CSR proteins of the present invention. On the other hand, exon E in Fig. 1 shows 61% amino acid homology to collagen. Also, exon D shows homology in amino acids to yeast chromosome segregation protein-smc2P, and human myosin heavy chain, and exon C to hibernation related protein-25 (HP25).

Structural similarities between the CSR proteins of this invention and known proteins were investigated based on amino acid composition, electrical charge, (CSR1: PI = 6.4, CSR 2: PI = 5.5) and molecular weight (CSR1: 65 Kd, CSR2: 52 Kd) by computer software PROP SEARCH. Such reliable and high homology as 80% or more was found between CSR1 protein and macrophage scavenger receptors I and II (GenBank Accession NO. S08278; Rohrer, L., et al., Nature, Vol. 343, 575-572, 1990) and between CSR2 protein and sensor protein (GenBank Accession NO. P30844; Nagasawa, S., et al., J. Biochem., Vol. 114, 350-357, 1993).

Secondary structures of the proteins were analyzed using computer software PROSITE, COIL, Tmpred, and PSORT. CSR1 and CSR2 presumably have the structure as schematically shown in Fig. 2. CSR1 is composed of three major different domains, (1) transmembrane domain with leucine zipper motif with four repeating leucine units, (2) α-helical coiled-coil domain, and (3) collagen-like domain with 49 repeating Gly-X-Y sequence units. Three molecules comprising these characteristic structures form a trimer, forming α helix at coiled-coil domain, and triple helix at collagen-like domain.

This structure is highly analogous to that in the above-mentioned macrophage scavenger receptor II (Fig. 3) (refer to A. Matsumoto, Chapter IV "Phlogocytes, 1. Scavenger Receptor" in "Molecular Arteriosclerotic Study", S. Morisaki, eds, Medical Review, p. 251, 1995). CSR2 protein lacks collagen-like domain of CSR1 protein and comprises therefor C terminus composed of 10 amino acids.

The collagen-like domain is known to bind to a wide range of molecules, such as lipids, plasma components (e.g. fibronectin, laminin, collagen, fibrinogen, etc.), and many negatively charged molecules. Analysis using computer software DNASIS revealed that the collagen-like domain of CSR protein has positive net charge at physiological pH.

It was also confirmed that CSR protein comprised many phosphorylation sites and glycosylation sites, heme binding sites and microbody (peroxisome) C-terminal targeting signal (Figs. 4 and 16). These results imply that CSR proteins undergo post-translational modification.

Non-translational region at 3' end of CSR1 and CSR2 genes comprised sequences composed of 6 base-sequences, AGTAAA and AATAAA, respectively, near the 3' end poly (A) site. Previous studies suggest that these sequences relates to addition of polyadenylation signals.

Example 6 Localization of CSR genes on chromosome

Localization of human CSR genes on human chromosomes was analyzed by fluorescence in situ hybridization (FISH) method ("Experimental Medicine, Supplement, Genetic Engineering Hand Book", Yodo, pp. 271-277, 1992) following the standard method.

The above-mentioned cosmid clone 2 containing exon sequence of CSR gene was used as a probe. To stain chromosomes by G band method, human metaphase chromosomes were prepared using thymidine synchronization method and bromodeoxyuridine release method.

Prior to hybridization, human cells at metaphase were stained with Hoechst-33258 (1 μg/ml) and exposed to ultraviolet light. The probe was labeled with biotin-16-UTP (Boehringer) by the nick translation method to hybridize with the denatured metaphase cells. Hybridization signals were detected with fluorescein isothiocyanate-avidin (FITC-avidin; Boehringer). The signals were precisely detected by visually confirming the duplicated G band. As a result, CSR gene was mapped at 8p21 on the duplicated G band chromosome sample (Fig. 5 (a)). The chromosome sample of metaphase human cells was mapped on R-banded karyotype [Fig. 5 (b)].

Example 7 Expression of CSR gene in various tissues

Using each CSR1 cDNA and CSR2 cDNA obtained above as a probe, expression of mRNA of CSR1 and CSR2 in various human tissues was examined by Human Multiple Tissue Northern Blot (Clonetech) by the standard method. The results are shown in Fig. 6.

The expression of CSR gene was detected in all tissue except for peripheral leukocytes, and at a relatively low level in spleen and liver.

The experiment of this example was performed as follows.

Poly $(A)^+$ RNA blotting membrane derived from human heart, brain, placenta, lung, liver, skeletal muscle, kidney, pancreas, spleen, thymus, prostate, testis, ovary, small intestine, colon, and peripheral leukocyte (each 2 μg of poly $(A)^+$ RNA was blotted; Clonetech) was hybridized with the above-mentioned CSR1 and CSR2 labeled with [$\alpha$-$^{32}$p]dCTP. The hybridized membrane was washed with 2 x SSC/0.05% SDS for 15 min at room temperature twice, and with 0.1 x SSC/0.1% SDS for 10 min at 50°C, and subjected to autoradiography using BAS1000 Imaging Plate (Fuji) for 12 to 24 hours. The expression level of human β actin RNA was used as a control.

Example 8 Regulation of CSR gene expression by p53 protein

The following analysis was conducted to examine whether the expression of CSR gene is regulated by interaction with tumor inhibitory protein p53 or not.

Wild-type p53 gene or mutant p53-273 gene expression vector was transiently transfected into human colorectal cancer line SW480 expressing mutant p53 (with mutation at 273rd and 309th codons) (Rodrigues, N.R. et al., Proc. Natl. Acad. Sci. USA, Vol. 87, 7555-7559, 1990) (transformants with wild-type gene and those with mutant gene are referred as W and M, respectively). After the transfection, RNA was continuously collected from transformants M and N to examine the expression parttern of CSR gene by RT-PCR. The results are shown in Fig. 7.

CSR gene expression pattern in (1) normal human fibroblast cell line NHDF4042 (Clonetics), (2) human cervical carcinoma derived epithelium-like cell line Hela, (3) human lung cancer cell line H1299, (4) human esophagus cancer cell line TE10, (5), human neuroglioblastoma cell line T98G, (6) human neuroglioblastoma cell line U87MG, (7) human tumor cell line SKBR3, and (8) the above-mentioned colorectal cancer cell line SW480 was analyzed by RT-PCR in the same manner as above.

The results are shown in Fig. 8. The RT-PCR was conducted as follows.

Extracted whole mRNA was converted into cDNA by random priming using $d(N)_{10}$ (Boehringer Mannheim)in the presence of M-MuLV reverse transcriptase (200 units/μl, GIBCO-BRL) to prepare a cDNA library. EC12 (SEQ ID NO: 15), and EC2 (SEQ ID NO: 16) were used as primers to amplify CSR cDNA by RT-PCR.

RT-PCR was conducted 30 to 40 cycles in a cDNA mixture (25 μl) using Thermocycler (Perkin Elmer Cetus). The cDNA mixture contains 40 ng of cDNA, 1 x PCR buffer (6.7 mM Tris, pH 8.8, 16.6 mM ammonium sulfate, 6.7 μM EDTA, and 10 mM β -mercaptoethanol), 25 pmol of sense primer, 25 pmol of antisense primer, 0.5 U of Tag DNA polymerase (TAKARA), 250 μM deoxynucleotides, 10% DMSO, and 7.6 mM magnesium chloride. Each PCR cycle was comprised of the reactions at 94°C for 30 sec, at 55°C for 30 sec, and at 72°C for 1 min. The final step was performed at 72°C for 1 min. The PCR product was subjected to 3% agarose gel electrophoresis, transferred to $N^+$ nylon membrane (PALL), and Southern-hybridized using the internal oligonucleotide probe F12 (SEQ ID NO: 8). In the RT-PCR, the amplification of GAPDH transcript using HGS (SEQ ID NO: 17) and HGA (SEQ ID NO: 18) as primers was used as a control. The internal probe (SEQ ID NO: 19) was used in Southern hybridization.

The expression of wild-type and mutant types CSR gene was not upregulated depending on the presence or absence of p53 protein, indicating the possibility that CSR gene expression be upregulated by other unknown factors.

<u>Example 8' Regulation of CSR gene expression by p53 protein</u>

In order to confirm the result of Example 8, whether p53 protein binds to CSR gene or not was examined by electorophoretic mobility shift assay (EMSA).

Human lung cancer cell line H1299, which inherenly does not express p53 protein and weakly expresses CSR mRNA, was transformed with wild-type p53 gene or mutant p53-273 gene expression plasmids (each 15 µg) (wild-type gene transformant and mutant gene transformant are referred to as W and M, respectively).

Nuclear extract was prepared from each transformant W and M 24 hours after the transformation following the known method (Mol. Cell. Biol., Vol. 12, p2866-2871, 1992).

Specifically, the transformants (1 X $10^6$ or more cells each) were suspended in 5 volumes of buffer A (20 mM of HEPES, pH 7.5, 20% glycerol, 10 mM NaCl, 10 mM $MgCl_2$, 0.2 mM EDTA, 1 mM DTT, 0.1% NP40, and protease inhibitor), incubated for 10 min on ice, and centrifuged for 10 min at 4°C. The resulting nuclear pellet was suspended in 2 volumes of buffer B (same as buffer A except for containing 500 mM NaCl), incubated for 30 min on ice, and centrifuged for 15 min at 14,000 rpm to collect supernatant. This supernatant was adjusted to 1 to 5 µl/ml for the EMSA test.

Each EMSA test run was conducted using nuclear extract (8 µl), buffer A (9 µl), poly (dI-dC) (1 µg, Boehringer Mannheim), monoclonal antibody PAb421 (1 µl, Oncogene Science), and/or competitor DNA (100 times or more of the amount of a non-labeled probe) on the double-stranded oligonucleotide (about 5 ng) with the labeled ends. After incubation for 30 min at room temperature, the product was electrophoresed in the gel (4% polyacrylamide/0.5 x Tris-borate-EDTA) at 200 V for 3 hours and transferred to a film at -80°C.

As a positive control probe, p53 DNA binding consensus sequence (p53-CON, SEQ ID NO: 21) was used. The probe against putative p53 binding sequence existing in CSR gene (p53-CSR, SEQ ID NO: 22) was constructed with annealing oligonucleotide (Fig. 18).

Both p53 proteins derived from tranformants W and M bound to p53 DNA binding consensus sequence (p53-CON, SEQ ID NO: 21) at the same extent. In contrast, only p53 derived from transformant W was bound to the probe against putative p53 binding sequence, even though the binding was weaker that that to p53 DNA binding consensus sequence. This result suggests that CSR gene transcription can partially be induced by the binding of p53 protein to CSR gene.

<u>Example 8'' Correlation between CSR gene expression in various tumor cells and the presence or absence of p53 protein</u>

In order to further analyze effect of presence or absence of p53 protein on the expression CSR gene, CSR expression in many tumor cells other than those used in Example 8 was analyzed by Southern hybridization using RT-PCR. The amplification of human β actin transcript was used as a control. In RT-PCR against human β actin, oligonucleotides of SEQ ID NO: 23 and SEQ ID NO: 24 were used as forward and reverse primers, respectively. The internal oligonucleotide of SEQ ID NO: 25 was used as a probe for Southern hybridization.

In this experiment, the following normal cells and tumor cells were used.

(1) Cells expressing wild-type p53 protein

Normal human fibroblast cell line NHDF4042 (Clonetics), human neuroglioblastoma cell line U87MG, human breast adenocarcinoma cell line MCF7, ceacum adenocarcinoma SNU-C4, and human colorectal carcinoma line HCT116.

(2) Cells expressing mutant p53 protein

Human cervical cancer-derived epithelium-like cell line Hela, human lung cancel cell line H1299, human esophagus cell lines (TE-3, TE10), human neuroglioblastoma cell line T98G, human carcinoma cell line SK-BR-3, and human colorectal carcinoma cell lines (SW480, HCT-15, and DLD-1), human metastatic prostate cancer cell line LNCap.FGC, human ceacum adenocarcinoma cell lines (H498, SNU-C2A), human stomach cancer cell lines (SNU-1, SNU-5, and SNU-16), and human lung cancer cell lines (ACC-LC174, ACC-LC176).

The result shown in Fig. 19 indicates that status of p53 gene (expression of p53 protein) does not necessarily correlate with CSR gene expression.

In other words, the presence or absence of p53 protein may partially be involve in induction of CSR gene expression and CSR gene expression may be regulated by other unknown factors.

<u>Example 9 Transcriptional induction of CSR gene by intracellular stresses</u>

In order to investigate the functions of CSR, whether CSR expression depends on intracellular stresses and damages ((1) serum starvation, (2) ultraviolet irradiation, (3) hydrogen peroxide, and (4) heat shock) was examined as follows.

9-1 Stress by serum starvation

Each of the above-mentioned human normal fibroblast cell line NHDF4042 (3 x $10^4$ cells), human colorectal carcinoma cell line SW480 (3 x $10^5$ cells), human neuroglioblastoma cell line T98G (3 x $10^5$ cells), human esophagus cancer cell line TE13 (3 x $10^5$ cells), and human cervical cancer derived epithelium-like cell line Hela (3 x $10^5$ cells) were spread onto the complete medium containing 10% fetal bovine serum (FBS, GIBCO-BRL) in a culture plate with a diameter of 100 mm and cultured under 5% $CO_2$ at 37°C. After one to two days, the culture medium was replaced by the complete medium containing 0.1% FBS and cultured for 4 days. RNA was extracted from each plate to examine the expression pattern of CSR mRNA by Northern blotting assay following the standard method. Northern blotting assay was conducted as follows.

RNA was extracted from each cell line using TRIZOL (GIBCO-BRL), and mRNA was isolated using Oligotex-dT30〈Super〉(JSR, Japan). The mRNA (0.51 μg) was added onto the 1.5% agarose gel with 20% formaldehyde and transferred onto Biodyne A Transfer Membrane (PALL). Using human CSR cDNA isolated from the cDNA library derived from human fetal brain prepared previously and labeled with [$\alpha$-$^{32}$P] dCTP (1 x $10^6$ cpm/ml) as a probe, hybridization was performed in a solution containing 50% (v/v) formamide, 5 x SSPE, 2 x Denharts soluion, and 1.25% (w/v) SDS. The expression level of RNA was analyzed using the expression level of actin RNA as a control.

The hybridized membrane was washed with 2 x SSC/0.05% SDS twice (at room temperature for 15 min for each wash), and with 0.1 x SSC/0.1 % SDS once (at 65°C for 10 min). The membrane was exposed to Imaging Plate BAS1000 (Fuji, Japan) for 24 to 48 hours. As a molecular size marker for electrophoresis, 0.24 to 9.5 kb of RNAs (GIBCO-BRL) were used (Fig. 9).

Removal of serum from the medium (serum starvation condition) induced expression of CSR mRNA in all cell lines.

9-2 Stress by ultraviolet irradiation

The above-mentioned human normal fibroblast cell line NHDF4042 (50% to 70% confluent by culturing in a culture medium for 24 to 48 hours) was irradiated with 5 to 120 J/$m^2$ ultraviolet light. RNA was extracted from the cells to examine the expression pattern of CSR mRNA by Northern blotting assay in the same manner as above. The expression level of mRNA was analyzed using the expression level of actin RNA as a control.

Ultraviolet irradiation was performed as follows. The cells were recovered from the culture medium and washed with phosphate butter twice prior to ultraviolet irradiation. The cells were irradiated with ultraviolet light 50 cm apart from a 40W-ramp for 5 to 120 seconds. The amount of ultraviolet irradiation was measured by Black Ray UV meter.

The results are shown in Fig. 10.

The expression of CSR mRNA was induced by ultraviolet irradiation. In this experiment, the highest expression was observed at the irradiation of 15 J/$m^2$.

Time-course of CSR mRNA expression patterns after the ultraviolet irradiation was monitored. RNA was extracted with the passage of time immediately after the addition of the medium irradiated with 15 J/$m^2$ ultraviolet and analyzed by Northern blotting in the same manner as above. As a result, the highest expression induction was observed from 20 to 24 hours after the ultraviolet irradiation.

9-3 Stress by hydrogen peroxide

The above-mentioned human normal fibroblast cell line NHDF4042 (50% to 70% sub-confluent by culturing in a culture medium for 24 to 48 hours) was washed with phosphate buffer twice, spread onto the complete medium supplemented with 1% fetal bovine serum, and cultured in the presence of 5 to 800 μM 30% hydrogen peroxide solution (Wako Pure Chemicals Industries) for 1 hour. The medium was then replaced with the fresh one to further continue culturing for 24 hours. RNA was extracted from the cells, and expression patterns of CSR mRNA were assayed by Northern blotting in the same manner as above. The expression levels of mRNA were analyzed using the expression level of actin RNA as a control.

The results are shown in Fig. 11.

This experiment confirmed that the expression of CSR mRNA was induced by intracellular stresses caused by hydrogen peroxide. The highest expression induction was observed when the concentration of hydrogen peroxide was 10 μM in this experiment. Hydrogen peroxide is known to produce reactive oxygen and free radical *in vivo*. Therefore, the induction of CSR mRNA expression confirmed in this experiment depends on oxidative stresses imparted to the cells by hydrogen peroxide.

9-4 Stress by heat shock

The above-mentioned human normal fibroblast cell line NHDF4042 (80 % confluent, 5 ml of complete medium) was cultured at 43°C for 40 min, and further cultured at 37°C. RNA was extracted from the cells with the passage of time, and expression patterns of CSR mRNA were assayed by Northern blotting in the same manner as above. The expression levels of mRNA were analyzed using the expression level of actin RNA as a control.

The results are shown in Fig. 12.

The expression of CSR mRNA was detected from 24 to 48 hours after the heat treatment. Expression induction by heat shock, observed in this experiment occurred later than the expression induction by the stresses caused by ultraviolet irradiation and hydrogen peroxide. This implies that induction by ultraviolet irradiation and hydrogen peroxide was more directly acted via a shorter pathway compared with the induction by heat shock.

<u>Example 9' Transcriptional induction of CSR gene by intracellular stresses</u>

The results obtained in Example 9 regarding stress caused by ultraviolet irradiation and hydrogen peroxide was reexamined as follows.

9'-1 Stress by irradiation of ultraviolet

The above-mentioned human normal fibroblast cell line NHDF4042 (3 x $10^4$ cells) were spread onto the complete medium (a 10-cm culture plate) and cultured for 24 to 48 hours. The medium was removed from the sub-confluent cultured system (50 to 70%), and the cells were washed with phosphate buffer twice. These cells were exposed to 5 to 120 J/$m^2$ ultraviolet radiation, and the fresh culture medium was added thereto. The ultraviolet light was radiated 50 cm apart from a 40W-ramp for 5 to 120 seconds. The amount of ultraviolet radiation was measured with a radiometer (Model 254, ATTO).

After culturing for 24 hours, RNA was extracted from the cells, and the expression levels of CSR mRNA were assayed by Northern blotting in the same manner as above. The expression levels of mRNA were analyzed using the expression level of actin RNA as a control.

The results are shown in Fig. 20(a).

Similar to the result of Example 9 (9-2), the expression of CSR mRNA was confirmed to be induced by ultraviolet irradiation. The highest expression was observed also in this experiment at 15 J/$m^2$ ultraviolet radiation.

9'-2 Stress by hydrogen peroxide

The above-mentioned human normal fibroblast cell line NHDF4042 (3 x $10^4$ cells) were spread onto the complete medium (a 10-cm culture plate) and cultured for 24 to 48 hours. The medium was removed from the sub-confluent cultured system (50 to 70%), and the cells were washed with phosphate buffer twice. A 1% fetal bovine serum-containing complete medium supplemented with 5 to 400 μM of 30% hydrogen peroxide (Wako Pure Chemical Industries), was added to these cells, and the cells were cultured for 1 hour. The medium was replaced with the fresh medium and the cells were further cultured for 24 hours.

RNA was extracted from the cells to assay the expression patterns of CSR mRNA by Northern blotting in the same manner as above. The expression levels of mRNA were analyzed using the expression level of actin RNA as a control. The results are shown in Fig. 20(b). Similar to the result of Example 9 (9-3), the expression of CSR mRNA was confirmed to be induced by intracellular stresses caused by hydrogen peroxide. The highest expression was also observed in this experiment when the cells were exposed to 10μM of hydrogen peroxide.

Example 9" Transcriptional induction of CSR gene by DNA damage

The effect of poisonous reagents against the gene on the expression of CSR mRNA and p53 mRNA was examined using Adriamycin, which shows an antitumor effect by intercchalating double stranded DNA to inhibit the DNA and RNA syntheses.

The above-mentioned human normal fibroblast cell line NHDF4042 (3 x $10^4$ cells) were spread onto the complete medium (a 10-cm culture plate) and cultured for 24 to 48 hours. Adriamycin (ADR) (0.02 to 1 μg/ml) was added to the sub-confluent cell culture system (50% to 70%), and cultured for 24 hours. The cells were then washed with phosphate buffer twice and the fresh complete medium was added for further culturing.

RNA was extracted from the cells to assay expression patterns of CSR mRNA by Northern blotting in the same manner as above. The expression levels of mRNA were analyzed using the expression level of actin RNA as a control. The results are shown in Fig. 21.

Adriamycin induced the expression of p53 mRNA, but did not influence the expression of CSR mRNA at all. This result indicates that the expression of CSR gene was induced not by the poisonous reagent against the gene such as Adriamycin, but mainly by intracellular stresses such as serum starvation, ultraviolet irradiation, hydrogen peroxide, or heat shock.

Example 10 Inhibition of CSR gene expression by antioxidant

The following experiment is conducted to confirm whether oxidative stresses are involved in expression induction of CSR gene or not.

The above-mentioned human normal fibroblast cell line NHDF4042 is cultured in the 10% FBS-containing complete medium supplemented with 40 mM acethylcysteine (NAC), which is thio compound capable of eliminating oxidative stresses. Trypan blue staining by the standard method can be used to confirm that acetylcysteine is not toxic to the cells. After stresses by ultraviolet radiation or hydrogen peroxide are imparted to the cells, RNA is extracted to assay by Northern blotting in the same manner as described above. The expression levels of mRNA are analyzed using the expression level of actin RNA as a control.

The results showing that the pre-treatment with antioxidant can inhibit expression induction of CSR mRNA by intracellular stresses caused by ultraviolet radiation and hydrogen peroxide indicates that oxidative stresses generated in cells are profoundly involved in the expression of CSR gene and that p53 protein functions as a sensor against intracellular oxidative stresses.

Example 10': Inhibition of CSR gene expression by antioxidant

The following experiment was conducted to confirm whether oxidative stresses are involved in expression induction of CSR gene or not.

The above-mentioned human normal fibroblast cell line NHDF4042 was spread onto the complete medium and cultured for 24 to 48 hours. The sub-confluent cells (50% to 70%) were cultured for 1 hour in the presence of 3 mM acethylcysteine (NAC), which is a thio compound capable of eliminating oxidative stresses. Trypan blue staining by the standard method can be used to confirm that acetylcysteine is not toxic to the cells.

After stresses by ultraviolet radiation or hydrogen peroxide were given to the cells, RNA was extracted to be subjected to Northern blotting in the same manner as in Examples 9 and 9'. The mRNA expression level was analyzed using the expression level of actin RNA as a control. The results are shown in Fig. 22.

The pre-treatment with the antioxidant inhibited expression induction of CSR mRNA by intracellular stresses due to ultraviolet irradiation and hydrogen peroxide.

Example 10" Analysis of expression induction of CSR gene by various reagents

Whether or not various reagents shown below, which produce intracellular stresses, induce CSR gene expression was examined. The expression patterns of c-fos gene, a component of AP-1 complex formed by oxidative stress, was also examined.

The expression of CSR gene was assayed by Southern hybridization by RT-PCR in the same manner as in Example 8. Amplification of human β actin transcript was used as a control.

In RT-PCR of human β actin, the oligonucleotides of SEQ ID NO: 23 and SEQ ID NO: 24 were used as forward and reverse primers, respectively. The internal oligonucleotide of SEQ ID NO: 25 was used as a probe for Southern hybridization.

In RT-PCR of c-fos, the oligonucleotides of SEQ ID NO: 26 and SEQ ID NO: 27 were used as forward and reverse primers, respectively. The internal oligonucleotide of SEQ ID NO: 28 was used as a probe for Southern hybridization.

The above-mentioned human normal fibroblast cell line NHDF4042 were treated as follows to give stresses before mRNA was isolated therefrom. mRNA was isolated within 2 hours after the the treatment and subjected to RT-PCR.

(1) culturing with 0.001 to 1% diethylmaleate (DEM, Sigma) for 1 hour. DEM produces oxidative stress by decreasing intracellular reduced glutathione.
(2) culturing with 300 to 2400 μg/ml 4β-phorbol 12-myristate 13-acetate (PMA) or tertadecanoyl phorbol acatetae (TPA) (SIGMA) for 1 hour. PMA activates membrane-bound NADPH oxidase and protein kinase C.
(3) culturing with 0.005 to 0.2% sodium azide ($NaN_3$, Sigma) for 6 hours. Sodium azide inhibits intracellular respiration by interfering with cytochrome C oxidase in mitochondria.
(4) culturing with 50 μM of sodium arsenite ($NaAsO_3$, Sigma) for 0 to 24 hours. Sodium arsenite strongly induces intracellular heat shock-like reaction and following oxidative stress.
(5) culturing with 0.5 mM of S-nitrosoglutathione (GSNO, Sigma) for 0 to 24 hours. GSNO stimulates the production of nitrogen monoxide (NO).
(6) culturing with 50 to 1200 μM of sodium nitroprusside (SNP, Sigma) for 2.5 hours. SNP stimulates the production of nitrogen monoxide (NO).

The results are shown in Fig. 23 (a) to (f).

The expression of CSR gene was significantly induced by 0.1% DEM, 600 μg/ml PAM, and 0.01% sodium azide. In addition, significant induction of CSR gene expression was observed for 50 μM sodium arsenite, for 0.5 mM GSNO (only four-hour culturing), and 50 to 100 μM SNP.

Nitrogen monoxide is known to function as an important regulation factor in center, immune and circulation systems, while it acts as a cause of such as septic shock, hypertension, infarct, neurodegenerative disorder, etc. (Annu. Rev. Biochem. Vol. 63, p. 175-195, 1994). Therefore, the fact that CSR gene expression was induced by SNP and GSNO suggests that CSR gene is an oxidative stress-reactive gene and is involved in development of the diseases described above.

<u>Example 10''-1 Analysis of expression induction of various genes by oxidative stresses</u>

In order to examine whether other genes except for CSR gene are induced by oxidative stresses or not, gene expression of CSR, c-fos, p53, waf-1, c-src, HO-1, catalase, SOD, and beta-actin, following oxidative stress due to hydrogen peroxide and hemin was monitored with the passage of time by RT-PCR.

Prior to isolation of mRNA, the above-mentioned human normal fibroblast cell line NHDF4042 was cultured with 10 μM hemin or 20 μMhydrogen peroxide for 1 to 50 hours. RNA was sampled with the passage of time to conduct RT-PCR.

The expression of each gene was analyzed by Southern hybridization with RT-PCR in the same manner as in Example 8. Forward and reverse primers used for RT-PCR and internal oligonucleotide probes used for Southern hybridization are as follows.

(1) CRS

Forward primer: SEQ ID NO: 15
Reverse primer: SEQ ID NO: 16
Internal oligonucleotide probe: SEQ ID NO: 8

(2) c-fos

Forward primer: SEQ ID NO: 26
Reverse primer: SEQ ID NO: 27
Internal oligonucleotide probe: SEQ ID NO: 28

(3) p53

Forward primer: SEQ ID NO: 29
Reverse primer: SEQ ID NO: 30
Internal oligonucleotide probe: SEQ ID NO: 31

(4) waf-1

Forward primer: SEQ ID NO: 32
Reverse primer: SEQ ID NO: 33
Internal oligonucleotide probe: SEQ ID NO: 34

(5) c-src

Forward primer: SEQ ID NO: 35
Reverse primer: SEQ ID NO: 36
Internal oligonucleotide probe: SEQ ID NO: 37

(6) HO-1

Forward primer: SEQ ID NO: 38
Reverse primer: SEQ ID NO: 39
Internal oligonucleotide probe: SEQ ID NO: 40

(7) catalase

Forward primer: SEQ ID NO: 41
Reverse primer: SEQ ID NO: 42
Internal oligonucleotide probe: SEQ ID NO: 43

(8) SOD

Forward primer: SEQ ID NO: 44
Reverse primer: SEQ ID NO: 45
Internal oligonucleotide probe: SEQ ID NO: 46

(9) beta-actin

Forward primer: SEQ ID NO: 23
Reverse primer: SEQ ID NO: 24
Internal oligonucleotide probe: SEQ ID NO: 25

The results are shown in Fig. 24(a) and (b).

Example 11 Distribution of CSR proteins in cells

Distribution of CSR proteins in cells was determined by the following immunocytochemical staining.

Full-length CSR cDNA obtained in the same manner as described above was amplified by the standard PCR method using a mixture of 0.25 U of Tag polymerase (TAKARA) and 0.5 U of pfuDNA polymerase (Stratagene), and cloned in a mammalian expression vector pCDNA3.1 (Invitrogen). The insertion of cDNA was confirmed by analyzing nucleotide sequences. The 5' and 3' ends of CSR cDNA were tagged with the nucleotide sequence of hemagglutinin (HA) epitope (YPYDVPDYA; Wilson, I.A., et al., Cell, Vol. 37, 767-778, 1984).

Human lung cancer cell line H1299 and human cervical cancer cell line Hela were transformed with the cDNA. The cells were cultured for 10 days in the presence of Geneticin (G418, GIBCO-BRL). The cells constantly expressing CSR were selected by RT-PCR, Western blotting and SDS polyacrylamide electrophoresis (SDS-PAGE). The selected cells were sowed onto a 2-well slide chamber (Falcon) 24 hours before immunohistochemical staining. After culturing, the cells were gently washed with phosphate buffer and fixed with 4% paraformaldehyde. The cells were washed with phosphate buffer, cultured for 30 min at room temperature in the presence of 2% bovine serum albumin, then cultured with anti-HA epitope polyclonal antibody (MBL) at 37°C for 1 hour. The cells were washed with phosphate buffer three times, cultured with FITC-labeled secondary antibody at 37°C for 40 min, and observed under a fluorescence microscope (Fig. 13 (a)). The nuclei were stained with 4',6'-diamino-2-phenylindole (DAPI, Sigma) and observed under a fluorescence microscope (Figure 13 (b)).

Fig. 13 (a) shows the wider stained region (around the nuclei) than the region of nuclei stained with DAPI at the same position (Fig. 13 (b)), indicating that CSR protein exists in the cytoplasm regardless of HA-tagged sites (C- and N-termini).

The reference staining is conducted using anti golgi-58K protein antibody (Sigma) for further examination in the same manner is described above.

The localization of CSR protein damaged by intracellular stresses (cytotoxicity) can be examined as follows.

The cells constantly expressing CSR on the slide chamber are incubated with 20 μM hydrogen peroxide as a cytotoxic reagent for 1 hour. After the medium is replaced by a fresh one, the cells are further cultured for 24 hour. The cells are then immunohistochemically stained with anti-HA epitope polyclonal antibody in the same manner as described above.

If CRS protein is distributed in the cytoplasm like when intracellular stresses (cytotoxicity) are not given and expressed more strongly than when the stresses are not given, secretion of CSR protein in cytoplasm depends on the stresses.

Example 11' Intracellular distribution of CSR protein

The result of Example 11 was further reexamined in the same manner as in Example 11.

Full-length cDNAs of CSR1 and CSR2 obtained above were amplified by PCR using a mixture of 0.25 U of Taq polymerase (TAKARA) and 0.5 U of pfuDNA polymerase (Stratagene) by the standard method, and cloned in a mammalian expression vector pCDNA3.1 (Invitrogen). The insertion of cDNA was confirmed by analyzing nucleotide sequences. The 5' and 3' ends of CSR cDNA were tagged with the nucleotide sequence of hemagglutinin (HA) epitope (YPYDVPDYA; Wilson, I.A., et al., Cell, Vol. 37, 767-778, 1984).

Human cervical cancer cell line Hela was transformed with the cDNA. The cells were cultured for 10 days in the presence of Geneticin (G418, GIBCO-BRL). The cells constantly expressing CSR were selected by RT-PCR, Western blotting, and SDS polyacrylamide electrophoresis (SDS-PAGE). The selected cells were spread onto a 2-well slide chamber (Falcon) 24 hours before immunohistochemical staining. After culturing, the cells were gently washed with phosphate buffer and fixed with cold methanol. The cells were washed with phosphate buffer, cultured for 30 min at room temperature in the presence of 2% bovine serum albumin, then cultured with anti-HA epitope polyclonal antibody (MBL) at 37°C for 1 hour. The cells were washed with phosphate buffer three times, cultured with FITC-labeled secondary antibody at 37°C for 40 min and observed under a fluorescence microscope. The nuclei were stained with 4',6'-diamino-2-phenylindole (DAPI, Boehringer Mannheim) and observed under a fluorescence microscope. The results are shown in Fig. 25 (a) to (d).

The cells transformed with vacant vectors (a negative control) were not stained at all (Fig. 25 (a)). In the cells transformed with the full-length CSR cDNA expression vector, the wider stained region (around the nuclei) than the region of the nuclei stained with DAPI at the same position (Fig. 25 (d)), indicating that CSR protein exists in the cytoplasm regardless of HA tagged sites (C- and N-termini).

To further analyze the presence of CSR protein in the cytoplasm, the reference staining was conducted using anti golgi-58K protein antibody (Sigma) and the secondary antibody ligated with Rhodamine (Leinco Tech). The results are shown in Fig. 25(e) and (f). Signal was detected at the same position as that of the above-mentioned HA tagged CSR protein (Fig. 25 (e)).

The localization of CSR protein damaged by intracellular stresses (cytotoxicity) was examined as follows.

The cells constantly expressing CSR on the slide chamber were incubated with 20 μM of hydrogen peroxide as a cytotoxic reagent for 1 hour. After the medium was replaced by a fresh one, the cells were further cultured for 24 hours. The cells were then immunohistochemically stained with anti-HA epitope polyclonal antibody in the same manner as described above. The results are shown in Fig. 25 (g) and (h).

CRS protein was distributed in the cytoplasm like when intracellular stress (cytotoxicity) was not given, and expressed more strongly than when the stress was not given. This result suggests that secretion of CSR protein in cytoplasm depends on the stresses.

<u>Example 12 Role of CSR on cell death caused by intracellular stresses</u>

Proteins expressed when cells receive various stresses are known to be profoundly involved in cell death or viability of the cells. In order to investigate the function and role of CSR when cells receive intracellular stresses (cellular damage), the following cell viability test was conducted.

Full-length cDNAs of CSR1 and CSR 2 were each cloned in a mammalian expression vector pCDNA3.1 (Invitrogen), and human lung cancer cell line H1299 was transformed with the vector. The cells were cultured with Geneticin (GIBCO-BRL) for 10 days to select the cells constantly expressing CSR. The overexpression of CSR was detected by RT-PCR. The cells overexpresssing CSR (500 cells) were spread onto a culture plate wit a diameter of 60 mm, cultured for 18 hours, and exposed to 5 to 60 J/$m^2$ ultraviolet radiation or 50 to 200 μM hydrogen peroxide, and cultured for 1 hour. The medium was replaced by a fresh one, and the cells were further cultured for 10 to 20 days. The obtained colonies were fixed with cold methanol and stained with Giemsa's solution (Merck) to count the number of living cells.

As controls, the same experiment was conducted using H1299 cells transformed with pCNDA3.1 containing no CSR gene, and the H1299 parental cells which were not transformed with any vector.

The results were shown in Figs. 14 (ultraviolet irradiation) and 15 (hydrogen peroxide).

The viability of the CSR-expressing cells that received intracellular stresses due to ultraviolet irradiation and hydrogen peroxide was significantly higher than those of the cells in the control groups. These results indicate that CSR has an inhibitory activity against cell death caused by various intracellular stresses (cytotoxicity).

<u>Example 12' Role of CSR on cell death caused by intracellular stresses</u>

The result obtained for the ultraviolet irradiation in Example 12 was further examined in the same manner as in Example 12. In this experiment, the cells co-transfected with the above-mentioned CSR1 gene expression vector and CSR2 gene expression vector (CSR1/CSR2) were also examined (Fig. 26).

Transformants with the CSR1 gene expression vector (H1299-CSR1), those with the CSR2 gene expression vector (H1299-CSR2), and those with both vectors (CSR1/CSR2) were all resistant to the cell death caused by intracellular stresses due to ultraviolet irradiation, compared with the control H1299 parental cells (H1299) and with the cells transfected with the vacant vector without CSR gene (H1299-vector).

CSR/CSR2 showed the highest resistance, followed by H1299-CSR1 and H1299-CSR2 in this order. These results indicate that CSR protein has a inhibiting activity against cell death caused by various intracellular stresses (cytotoxicity).

<u>Example 12'' Role of CSR on cell death caused by intracellular stresses</u>

The mechanism of resistance expression of CSR protein against oxidative stresses, which was confirmed in Examples 12 and 12,' was analyzed as follows.

Four types of cells (H1299-vector, H1299-CSR1, H1299-CSR2, and CSR1/CSR2, each 2 x $10^5$ cells) used in Example 12' were cultured in the presence of hydrogen peroxide (20 μM) for 1 hour. After the medium was replaced with a fresh complete medium, the cells were further cultured for 24 hours. The cells were then cultured in the presence of 5 μM 2',7,-dichlorofluorescein diacetate (DCFH-DA, Molecular Probes) at 37°C for 30 min, and suspended in phosphate buffer. The amount of reactive oxygen species produced by cells in each cell group was measured with FACS Calibur (Becton Dickinson).

In this method, the amount of reactive oxygen species was determined based on the fluorescence intensity that increases with the oxidation level of DCFH-DA in cells. The results are shown in Fig. 27 (a) to (d).

The amount of reactive oxygen species was significantly increased in the control H1299-vector group (Fig. 27 (a)). On the other hand, it was significangly decreased in H1299-CSR1 and H1299-CSR2 compared with that in the control H1299-vector (Fig. 27 (b) and (c)). Suprisingly, almost no reactive oxygen species was produced in the CSR1/CSR2 group (Fig. 27 (d)).

The presence or absence of resistance to the cell death (resulting in cell death through the morphological change as fragmentation) induced by stresses due to hydrogen peroxide was examined by analyzing the cell morphology. Morphology of H1299-vector and CSR1/CSR2 groups was observed under a microscope by the standard method at the the time of adding hydrogen peroxide (0 hour), 1 and 3 hours after culturing cells in the presence of hydrogen peroxide.

The results are shown in Fig. 28 (a) to (h). The morphological change (fragmentation) was observed in the control H1299-vector group from immediately after loading of oxidative stresses due to hydrogen peroxide, indicating that the cells were led to cell death with the passage of time. In contrast, in the CSR1/CSR 2 group, the morphological change was temporarily observed immediately after the loading of oxidative stresses due to hydrogen peroxide, but the cell morphology was gradually recovered.

This result indicates that CSR protein inhibits the cell death caused by the various oxidative stresses.

Example 13 Generation of CSR gene knockout mice

Knockout mice in which endogenous CSR gene corresponding to human CSR gene was inactivated (knocked out) were generated as follows.

(1) Construction of a targeting vector

A targeting vector for generation of a knockout mouse, in which the endogenous gene encoding mouse CSR protein was inactivated (knocked out) by homologous recombination (Nikkei-Science, No.5, pp. 52-62, 1994) was constructed as follows.

The cDNA encoding human CSR protein, which was cloned in the previous Example, was labeled with $^{32}P$ by the standard method to obtain a probe used in hybridization. The probe was used to screen a cosmid library in which mouse genomic DNA was introduced (prepared in accordance with the method described in "Laboratory Manual" (M. Hori and Y. Nakamura, eds., Maruzen), and a mouse CSR genomic DNA clone containing exons (mouse exons corresponding to a part of exons A, B, C, D, and E of human CSR gene shown in Fig. 1) that encode mouse CSR protein was isolated.

The plasmid pBluescript II SK(-) was digested with KpnI and ligated with blunted HindIII-XhoI-digested thymidine kinase gene (TK, as a negative selection marker).

The resulting pBluescript II SK(-) was digested with XbaI and SalI and ligated with a blunted HpaI-Acc65I-digested DNA fragment of the mouse CSR genomic DNA.

Then, the neomycin resistance gene (neo, as a positive selection marker) was digested with XbaI and EcoRV, blunted, and inserted between the NciI site of exon B and the Aor51HI site of the exon D in the mouse CSR genomic DNA. The resulting plasmid was digested with NotI and linealized to use as a targeting vector.

The structure of the thus-prepared targeting vector is schematically shown in Fig. 29.

(2) Transfection of the targeting vector into ES cells

Mouse embryonic stem cells (ES cells, 1 x $10^8$ cells) (Nature (1993) 362, 255-258; Nature (1987) 326, 292-295), which were cultured in DMEM containing 15% fetal bovine serum, were trypsinized to obtain single isolated cells, washed three times in phosphate buffer, and then prepared as a cell suspension of 1 x $10^7$ cells/ml. The targeting vector was added to the (25 μg/1 ml cell suspension), and electroporation was performed with a single pulse of 350 V/cm (25 μF). Then, the ES cells were seeded into 10 cm dishes (1 x $10^7$ cells/dish), cultured 1 day in maintenance medium, and then the medium was replaced with selection medium (containing G418 (250 μg/ml) and 2 μM gancyclovir). The culture was continued with replacing the medium every two days. On the tenth day after transfection, several hundreds of neomycin resistant ES clones were isolated using a micropipet under microscopic observation. The clones were cultured separately in 24 well plates layered with feeder cells, and replica of neomycin resistant ES cells were obtained.

(3) Screening of knockout ES cells

Each neomycin resistant ES clone is examined by genomic Southern blotting whether its endogenous gene encoding mouse CSR protein is inactivated (knocked out) through homologous recombination.

Genomic DNA is extracted from each neomycin resistant ES clone, and genomic Southern blotting is performed on EcoRI digested genomic DNA fragments according to the standard method using the two probes; one comprises 5'-end sequence of mouse CSR genomic DNA and another comprises 3'-end sequence thereof. DNA is purified using an automated DNA purification robot (Kubota).

In this way, whether a desired knockout occurs in ES cell clones can be confirmed. The ES cell clones are used for generation of knockout mice as described below.

(4) Generation of knockout mice

The ES clones obtained above, having inactivation in the endogenous gene encoding mouse CSR protein as a result of homologous recombination, are microinjected into blastocysts obtained by crossing C57BL6 mice (Japan Charles River) (15 cells/embryo). Immediately after microinjection, the blastocysts are transferred to uterines of ICR mice (Clea Japan) (10 blastocysts/one side of the uterine), which have undergone pseudopregnancy treatment two days and half before. As a result, desired knockout chimera mice are obtained from each ES clone.

The chimera are crossed with normal C57BL6 mice to obtain agouti mice whose color is attributed to a gene determining hair color, originating from ES cells.

Industrial Applicability

The novel genes and proteins having characteristics described below are considered as novel scavenger receptors protectively acting against various stresses generated in cells and are thus extremely useful for developing pharmaceuticals targeting the genes or proteins of the scavenger receptors (cellular sensor-related protein, CSR) of the present invention that prevent and treat morbidities and disorders (for example, atherosclerosis, diabetic angiopathy, bacterial infection, etc.) caused by intracellular stresses, xenobiotics or denatured proteins produced in the body.

The genes can be used as antisense pharmaceuticals and used in gene therapy. The proteins or soluble fragments (extracellular regions, various domains) thereof can be produced to serve as soluble protein pharmaceuticals.

The antibodies or a portion thereof reactive with the proteins or fragments thereof are extremely useful as antibody pharmaceuticals that regulate the functions of CSR.

## Claims

1. A DNA encoding a protein having the amino acid sequence of SEQ ID NO: 10 or a fragment thereof.

2. A DNA encoding a protein having the amino acid sequence of SEQ ID NO: 12 or a fragment thereof.

3. A DNA encoding a protein having the amino acid sequence of SEQ ID NO: 14 or a fragment thereof.

4. A DNA comprising a nucleotide sequence corresponding to nucleotide residues 276 to 2096 of the nucleotide sequence of SEQ ID NO: 9, or a fragment thereof.

5. A DNA comprising a nucleotide sequence corresponding to nucleotide residues 276 to 1676 of the nucleotide sequence of SEQ ID NO: 11, or a fragment thereof.

6. A DNA comprising a nucleotide sequence corresponding to nucleotide residues 364 to 1971 of the nucleotide sequence of SEQ ID NO: 13, or a fragment thereof.

7. A DNA hybridizing with a DNA having the nucleotide sequence of SEQ ID NO: 9, 11, or 13 under stringent conditions.

8. A protein having the amino acid sequence of SEQ ID NO: 10 or an amino acid sequence substantially the same as said amino acid sequence, or a fragment thereof.

9. A protein having the amino acid sequence of SEQ ID NO: 12 or an amino acid sequence substantially the same as said amino acid sequence, or a fragment thereof.

10. A protein having the amino acid sequence of SEQ ID NO: 14 or an amino acid sequence substantially the same as said amino acid sequence, or a fragment thereof.

11. An expression vector comprising the DNA of any one of claims 1 to 7.

12. A transformant carrying the expression vector of claim 11.

**13.** An antibody or its portion reactive with the proteins of any one of claims 8 to 10 or their fragments.

**14.** The antibody or its portion of claim 13, wherein the antibody is a monoclonal antibody.

**15.** A pharmaceutical composition comprising the antibody or its portion of claims 13 or 14 and a pharmaceutically acceptable carrier.

**16.** A cell producing a monoclonal antibody reactive with the proteins of any one of claims 8 to 10 or their fragments.

**17.** The cell of claim 16, wherein said cell is hybridoma obtainable by fusing B cells derived from a non-human mammal capable of producing a monoclonal antibody with myeloma derived from a mammal.

**18.** The cell of claim 16, wherein said cell is a recombinant cell transformed with either a DNA encoding a heavy chain of said monoclonal antibody or a DNA encoding a light chain of said monoclonal antibody, or both DNAs.

Figure 1

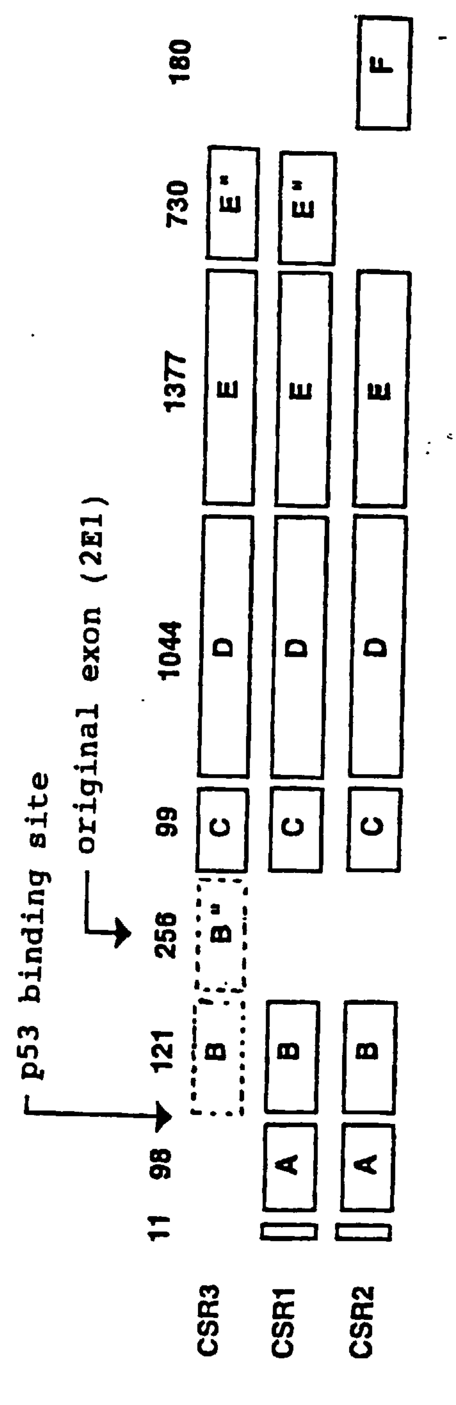
p53 binding site
original exon (2E1)
11
98
121
256
99
1044
1377
730
180
CSR3
CSR1
CSR2
A
B
B"
C
D
E
E"
F

Figure 2

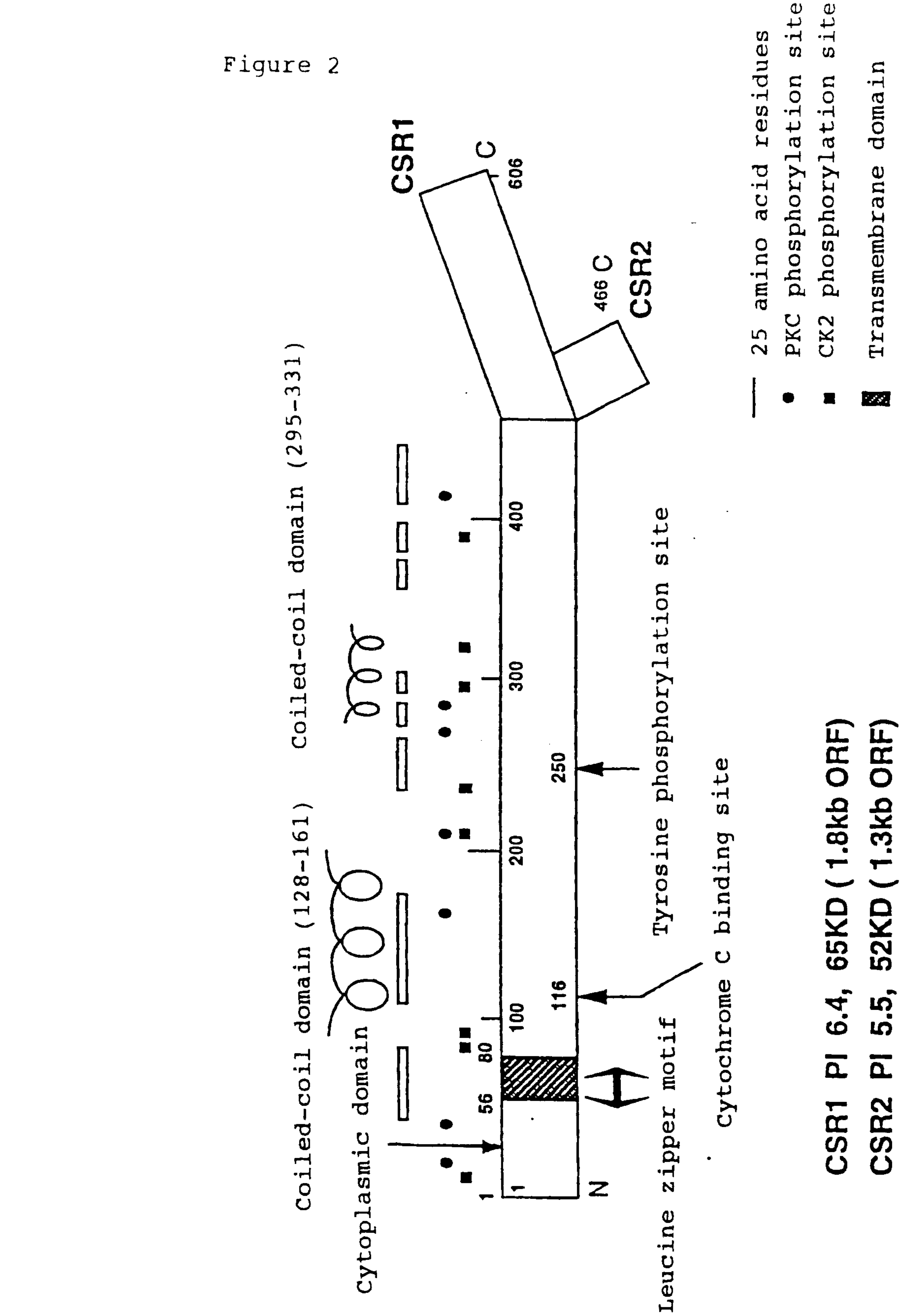
CSR1
C
606
466 C
CSR2
Coiled-coil domain (128-161)
Coiled-coil domain (295-331)
Cytoplasmic domain
1
56
80
100
116
200
250
300
400
N
1
Leucine zipper motif
Cytochrome C binding site
Tyrosine phosphorylation site
25 amino acid residues
PKC phosphorylation site
CK2 phosphorylation site
Transmembrane domain
CSR1 PI 6.4, 65KD ( 1.8kb ORF)
CSR2 PI 5.5, 52KD ( 1.3kb ORF)

Figure 3

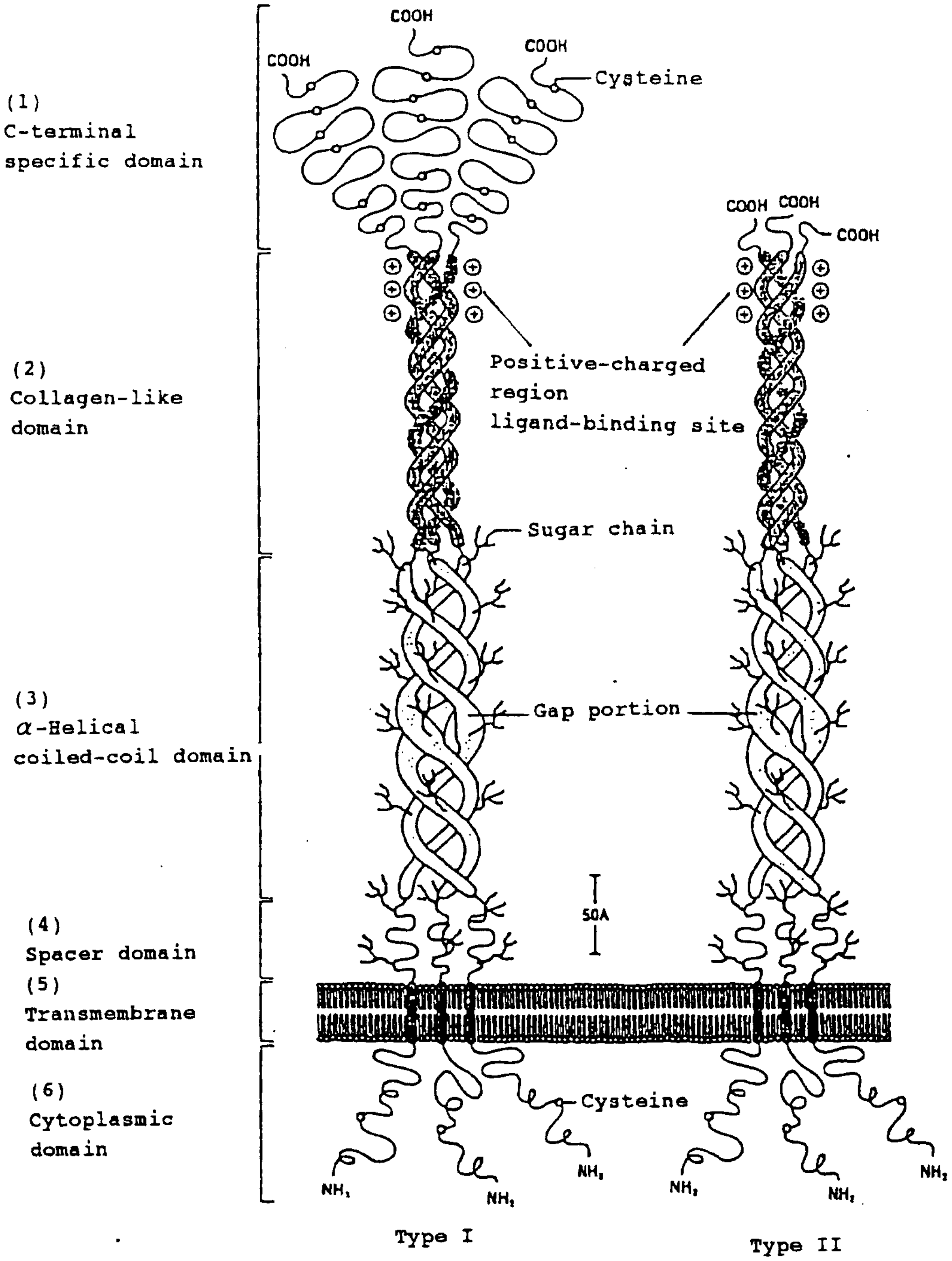
(1)
C-terminal
specific domain
(2)
Collagen-like
domain
(3)
α-Helical
coiled-coil domain
(4)
Spacer domain
(5)
Transmembrane
domain
(6)
Cytoplasmic
domain
COOH
Cysteine
Positive-charged
region
ligand-binding site
Sugar chain
Gap portion
50A
NH₂
Type I
Type II

Figure 4

Met Lys Val Arg Ser Ala Gly Gly Asp Gly Asp Ala Leu Cys Val Thr Glu Glu Asp Leu Ala Gly
Asp Asp Glu Asp Met Pro Thr Phe Pro Cys Thr Gln Lys Gly Arg Pro Gly Pro Arg Cys Ser Arg
Cys Gln Lys Asn Leu Ser Leu His Thr Ser Val Arg Ile Leu Tyr Leu Phe Leu Ala Leu Leu Leu
Val Ala Val Ala Val Leu Ala Ser Leu Val Phe Arg Lys Val Asp Ser Leu Ser Glu Asp Ile Ser
Leu Thr Gln Ser Ile Tyr Asp Lys Lys Leu Val Leu Met Gln Lys Asn Leu Gln Gly Leu Asp Pro
Lys Ala Leu Asn Asn Cys Ser Phe Cys His Glu Ala Gly Gln Leu Gly Pro Glu Ile Arg Lys Leu
Gln Glu Glu Leu Glu Gly Ile Gln Lys Leu Leu Leu Ala Gln Glu Val Gln Leu Asp Gln Thr Leu
Gln Ala Gln Glu Val Leu Ser Thr Thr Ser Arg Gln Ile Ser Gln Glu Met Gly Ser Cys Ser Phe
Ser Ile His Gln Val Asn Gln Ser Leu Gly Leu Phe Leu Ala Gln Val Arg Gly Trp Gln Ala Thr
Thr Ala Gly Leu Asp Leu Ser Leu Lys Asp Leu Thr Gln Glu Cys Tyr Asp Val Lys Ala Ala Val
His Gln Ile Asn Phe Thr Val Gly Gln Thr Ser Glu Trp Ile His Gly Ile Gln Arg Lys Thr Asp
Glu Glu Thr Leu Thr Leu Gln Lys Ile Val Thr Asp Trp Gln Asn Tyr Thr Arg Leu Phe Ser Gly
Leu Arg Thr Thr Ser Thr Lys Thr Gly Glu Ala Val Lys Asn Ile Gln Ala Thr Leu Gly Ala Ser
Ser Gln Arg Ile Ser Gln Asn Ser Glu Ser Met His Asp Leu Val Leu Gln Val Met Gly Leu Gln
Leu Gln Leu Asp Asn Ile Ser Ser Phe Leu Asp Asp His Glu Glu Asn Met His Asp Leu Gln Tyr
His Thr His Tyr Ala Gln Asn Arg Thr Val Glu Arg Phe Glu Ser Leu Glu Gly Arg Met Ala Ser
His Glu Ile Glu Ile Gly Thr Ile Phe Thr Asn Ile Asn Ala Thr Asp Asn His Val His Ser Met
Leu Lys Tyr Leu Asp Asp Val Arg Leu Ser Cys Thr Leu Gly Phe His Thr His Ala Glu Glu Leu
Tyr Tyr Leu Asn Lys Ser Val Ser Ile Met Leu Gly Thr Thr Asp Leu Leu Arg Glu Arg Phe Ser
Leu Leu Ser Ala Arg Leu Asp Leu Asn Val Arg Asn Leu Ser Met Ile Val Glu Glu Met Lys Ala
Val Asp Thr Gln His Gly Glu Ile Leu Arg Asn Val Thr Ile Leu Arg Gly Ala Pro Gly Pro Pro
Gly Pro Arg Gly Phe Lys Gly Asp Met Gly Val Lys Gly Pro Val Gly Gly Arg Gly Pro Lys Gly
Asp Pro Gly Ile Leu Gly Pro Leu Gly Pro Gln Gly Pro Gln Gly Gln Pro Gly Glu Ala Gly Pro
Val Gly Glu Arg Gly Pro Val Gly Pro Arg Gly Phe Pro Gly Leu Lys Gly Ser Lys Gly Ser Phe
Gly Thr Gly Gly Pro Arg Gly Gln Pro Gly Pro Lys Gly Asp Ile Gly Pro Pro Gly Pro Glu Gly
Pro Pro Gly Ser Pro Gly Pro Ser Gly Pro Gln Gly Lys Pro Gly Ile Ala Gly Lys Thr Gly Ser
Pro Gly Gln Arg Gly Ala Met Gly Pro Lys Gly Glu Pro Gly Ile Gln Gly Pro Pro Gly Leu Pro
Gly Pro Pro Gly Pro Pro Gly Ser Gln Ser Phe Tyr

Figure 5

(a)

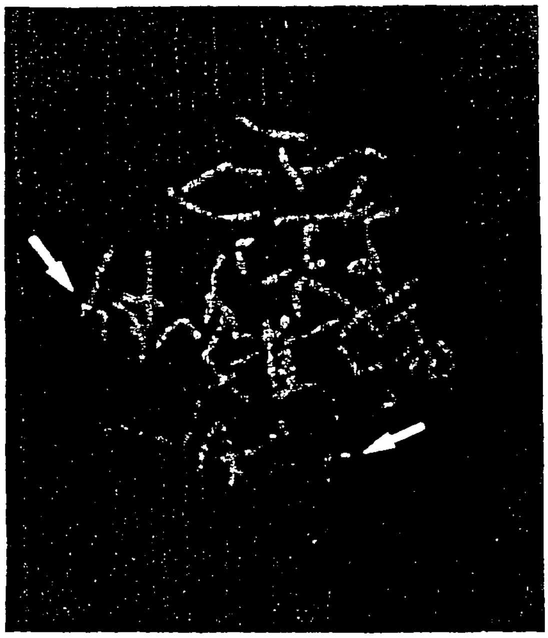

(b)

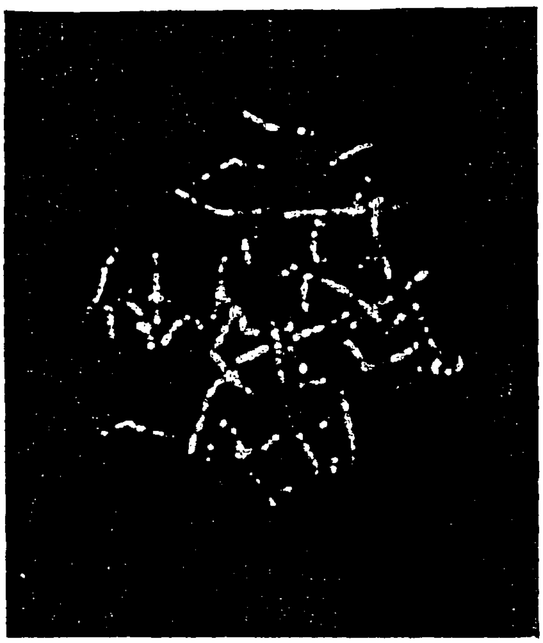

Figure 6

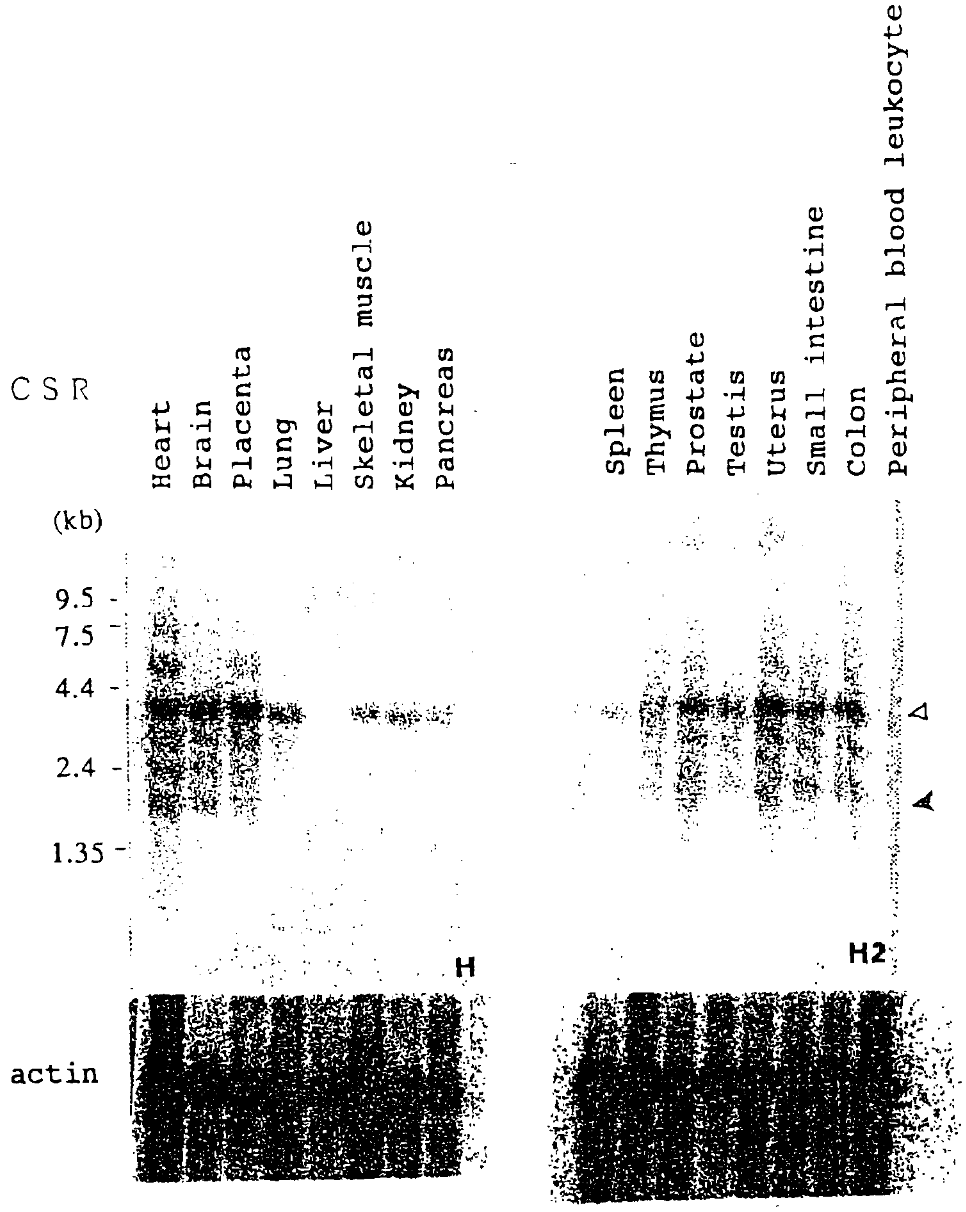
CSR
Heart
Brain
Placenta
Lung
Liver
Skeletal muscle
Kidney
Pancreas
Spleen
Thymus
Prostate
Testis
Uterus
Small intestine
Colon
Peripheral blood leukocyte
(kb)
9.5
7.5
4.4
2.4
1.35
H
H2
actin

Figure 7

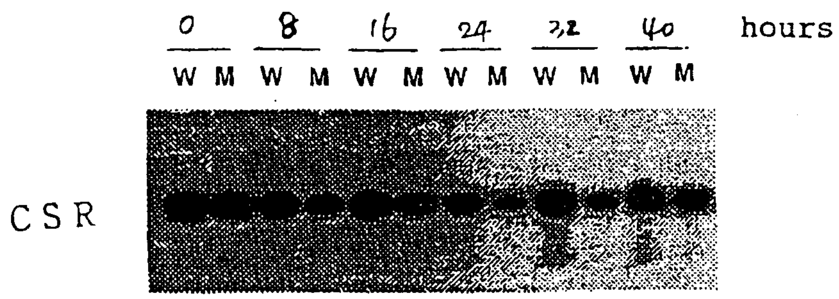

Figure 8

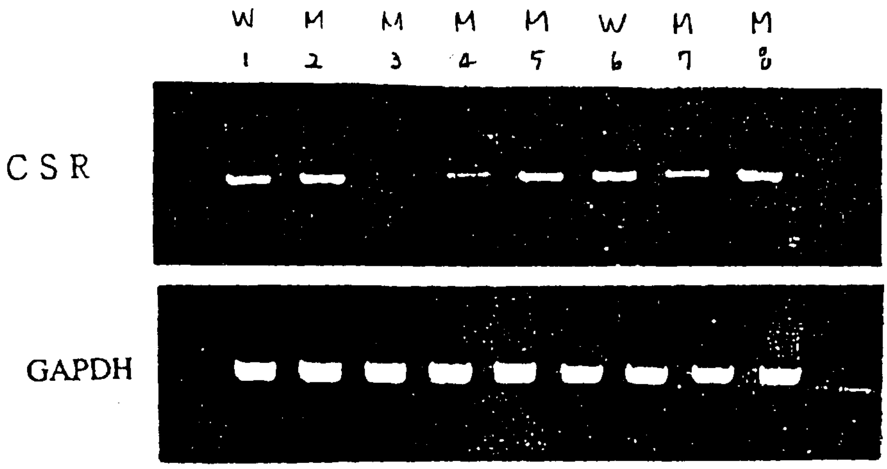

Figure 9

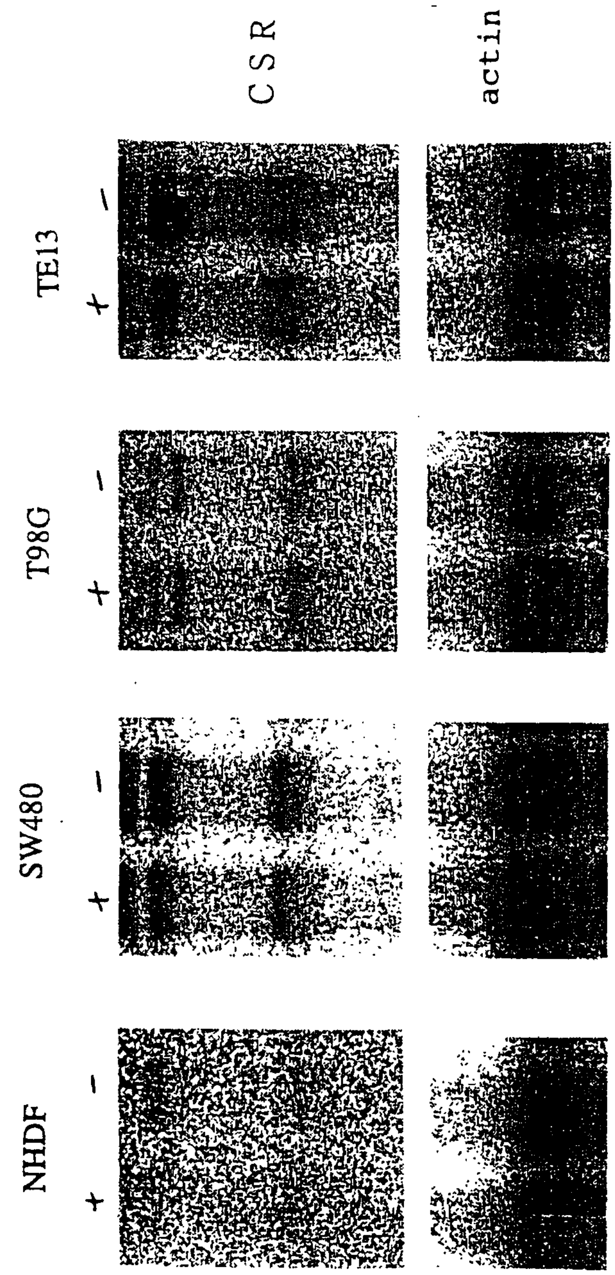

Figure 10

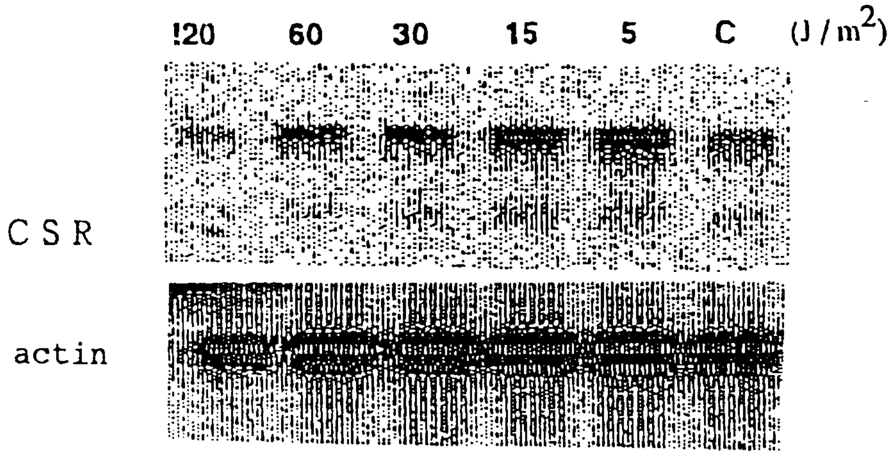

Figure 11

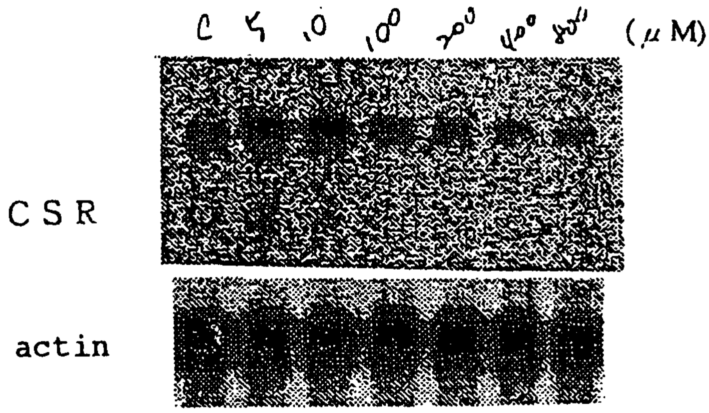

Figure 12

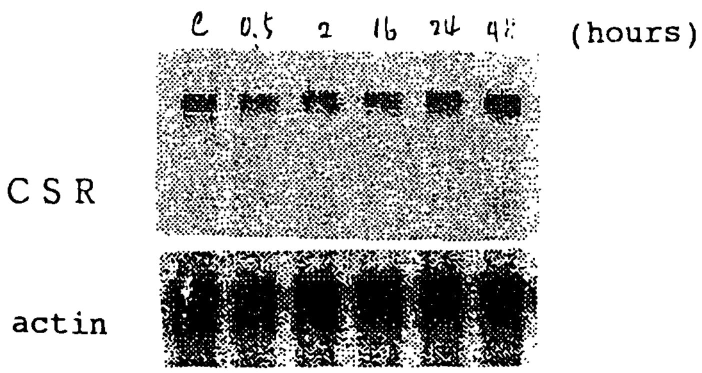

Figure 13

(a)

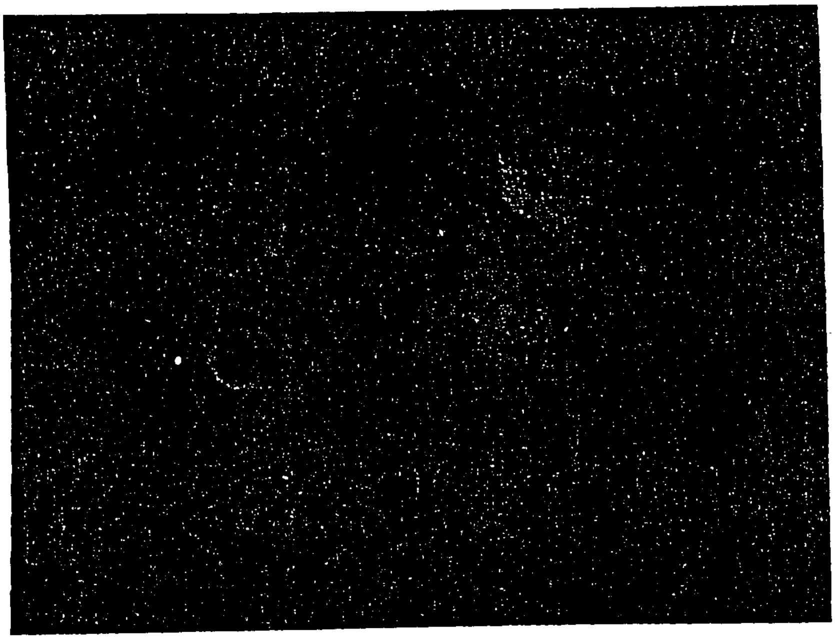

(b)

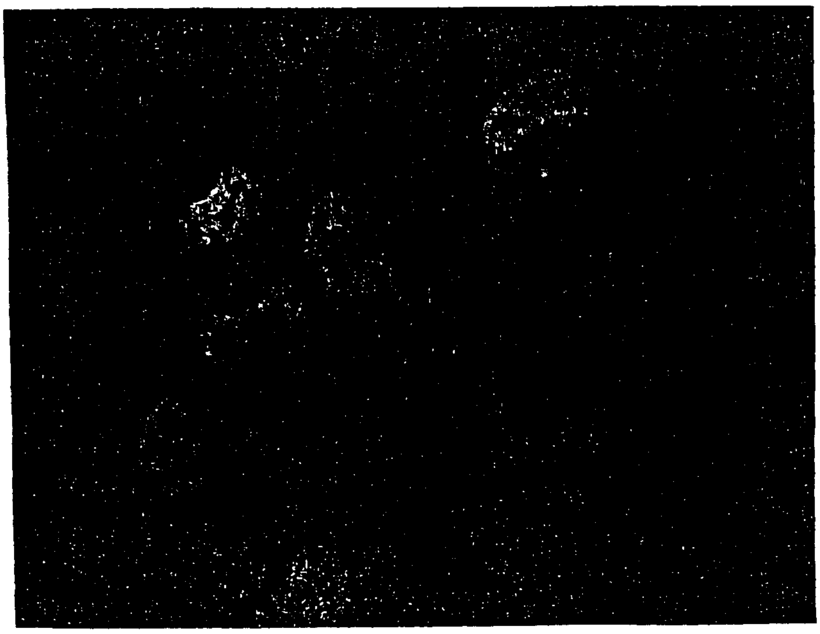

Figure 14

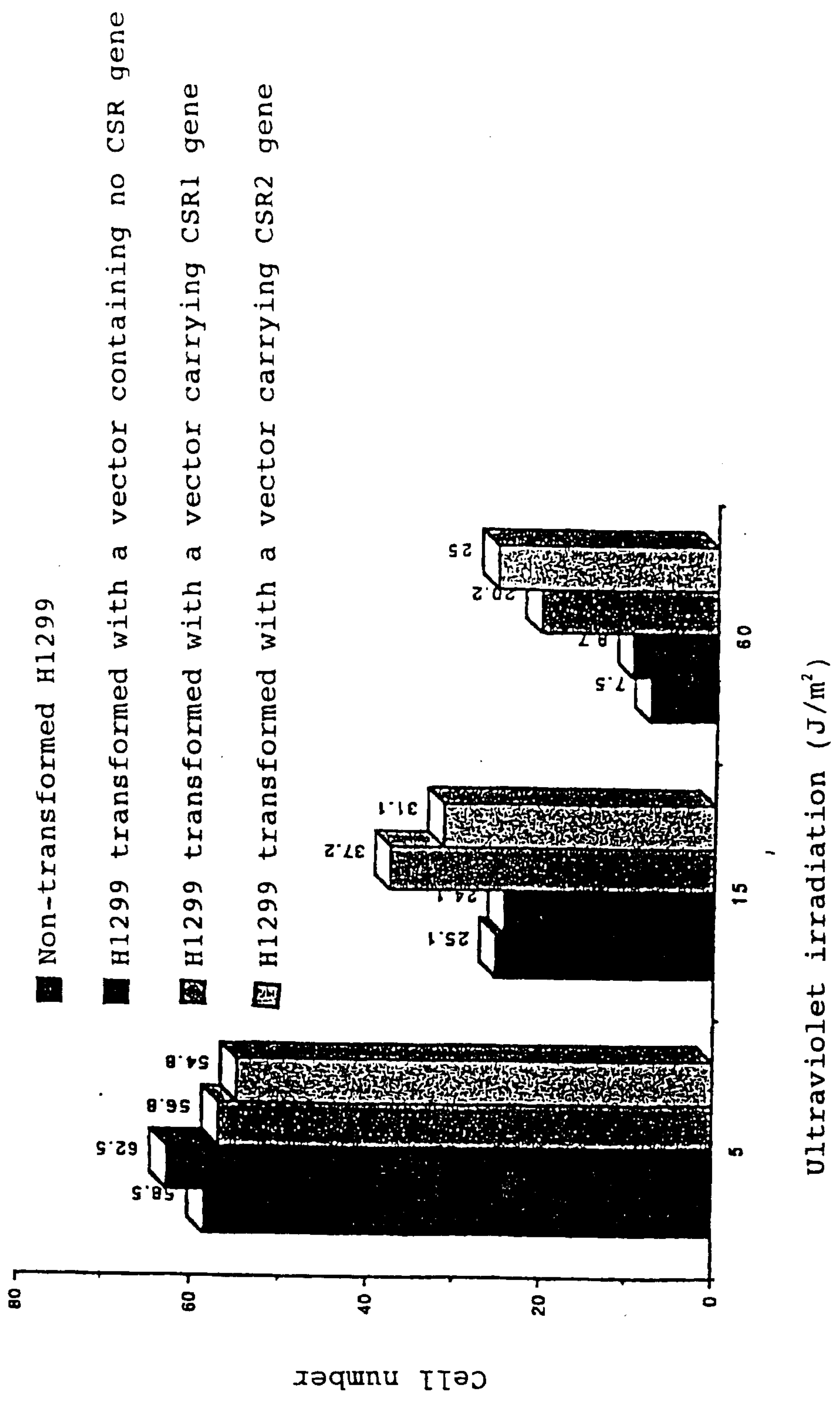
Non-transformed H1299
H1299 transformed with a vector containing no CSR gene
H1299 transformed with a vector carrying CSR1 gene
H1299 transformed with a vector carrying CSR2 gene
Cell number
Ultraviolet irradiation (J/m²)
0
20
40
60
80
5
15
60
58.5
62.5
56.8
54.8
25.1
24.1
37.2
31.1
7.5
8.7
20.2
25

Figure 15

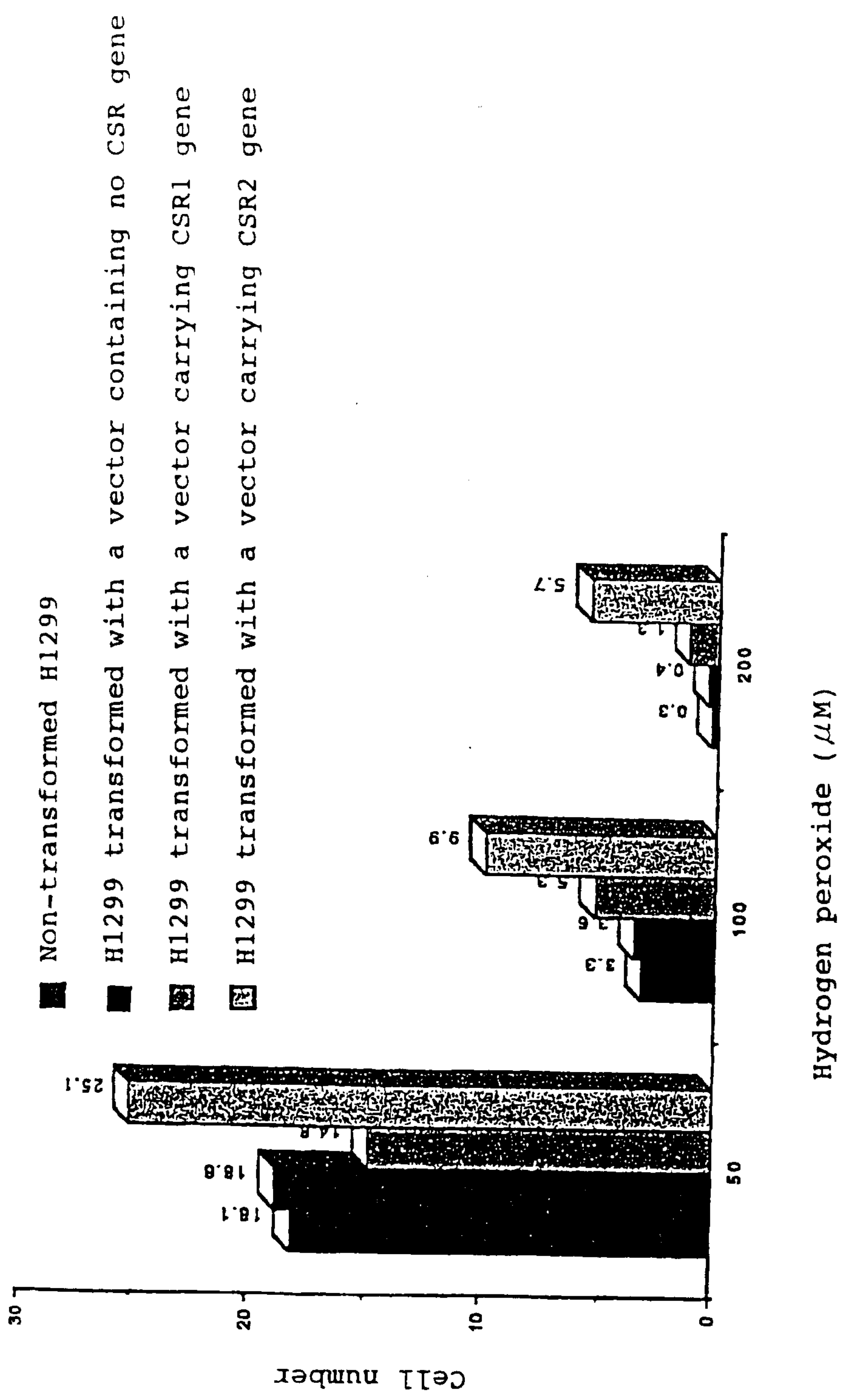
Non-transformed H1299
H1299 transformed with a vector containing no CSR gene
H1299 transformed with a vector carrying CSR1 gene
H1299 transformed with a vector carrying CSR2 gene
Cell number
0
10
20
30
Hydrogen peroxide (μM)
50
100
200
18.1
18.8
14.8
25.1
3.3
9.9
0.3
0.4
5.7

Figure 16

1 M K V R S A G G D G D A L C V T E E D L A G D D E 25
26 D M P T F P C T Q K G R P G P R C S R C Q K N L S 50
51 L H T S V R I L Y L E L A L L L V A V A V L A S L 75
76 V F R K V D S L S E D I S L T Q S I Y D K K L V L 100
101 M Q K N L Q G L D P K A L N N C S F C H E A G Q L 125
126 G P E I R K L Q E E I E G I Q K H L L A Q E V Q L 150
151 D Q T L Q A Q E V L S T T S R Q I S Q E M G S C S 175
176 F S I H Q V N Q S L G L F L A Q V R G W Q A T T A 200
201 G L D L S L K D L T Q E C Y D V K A A V H Q I N F 225
226 T V G Q T S E W I H G I Q R K T D E E T L T L Q K 250
251 I V T D W Q N Y T R L F S G L R T T S T K T G E A 275
276 V K N I Q A T L G A S S Q R I S Q N S E S M H D C 300
301 V L Q V M G L Q L Q L D N T S S F L D D H E E N M 325
326 H D L Q Y H T H Y A Q N R T V E R F E S L E G R M 350
351 A S H E I E I G T I F T N I N A T D N H V H S M L 375
376 K Y L D D V R L S C T L G F H T H A E E L Y Y L N 400
401 K S V S I M L G T T D L L R E R F S L L S A R L D 425
426 L N V R N L S M I V E E M K A V D T Q H G E I L R 450
451 N V T I L R G A P G P P G P R G F K G D M G V K G 475
476 P V G G R G P K G D P G I L G P L G P Q G P Q G Q 500
501 P G E A G P V G E R G P V G P R G F P G L K G S K 525
526 G S F G T G G P R G Q P G P K G D I G P P G P E G 550
551 P P G S P G P S G P Q G K P G I A G K T G S P G Q 575
576 R G A M G P K G E P G I Q G P P G L P G P P G P P 600
601 G S Q S F Y * 606

(a)

451 N V T I L R G H T L F S H N R I * 466

(b)

Figure 17

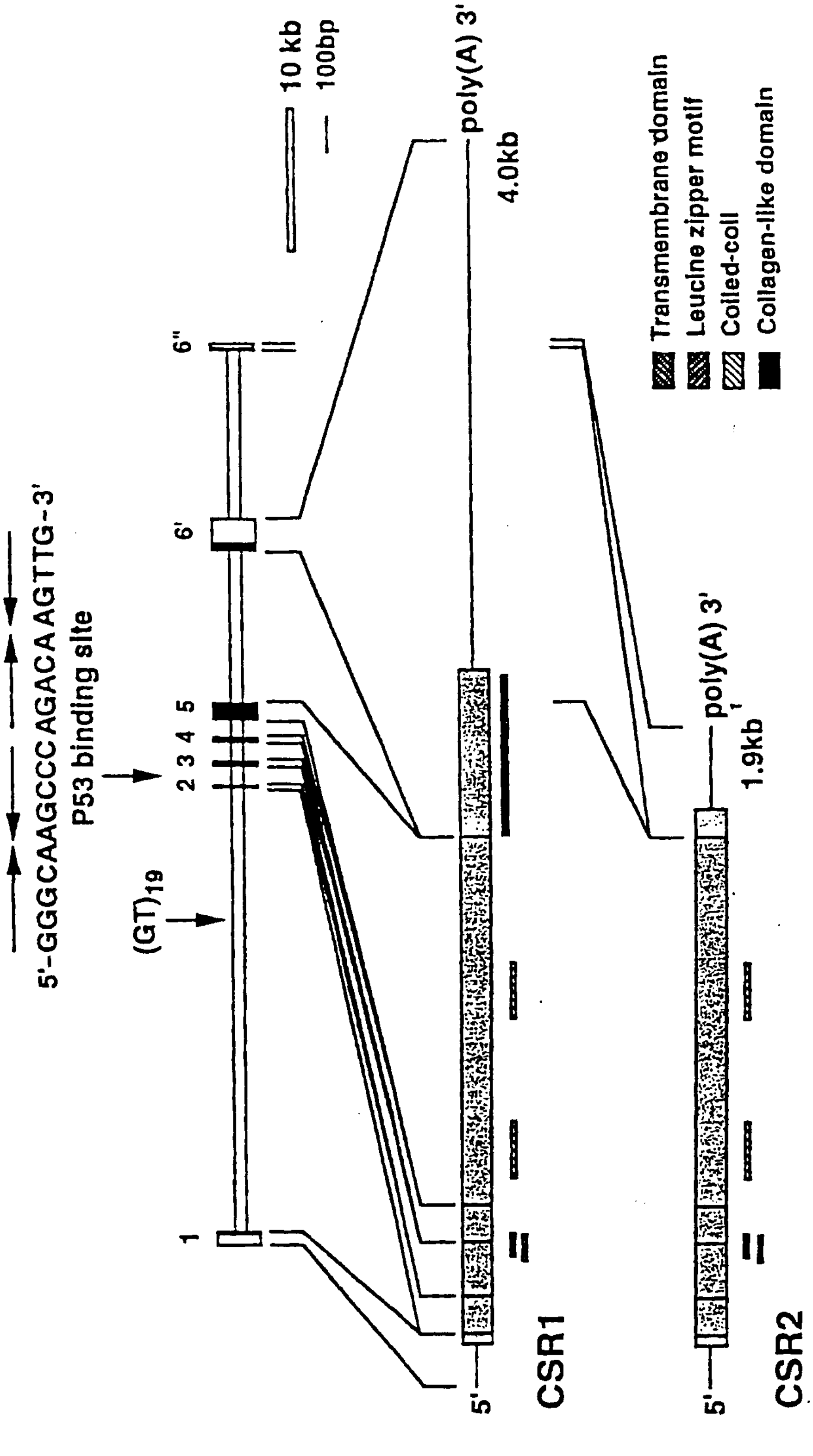
5'-GGGCAAGCCCAGACAAGTTG-3'
P53 binding site
$(GT)_{19}$
1
2 3 4 5
6'
6''
10 kb
100bp
poly(A) 3'
4.0kb
5'
CSR1
poly(A) 3'
1.9kb
5'
CSR2
Transmembrane domain
Leucine zipper motif
Coiled-coil
Collagen-like domain

Figure 18

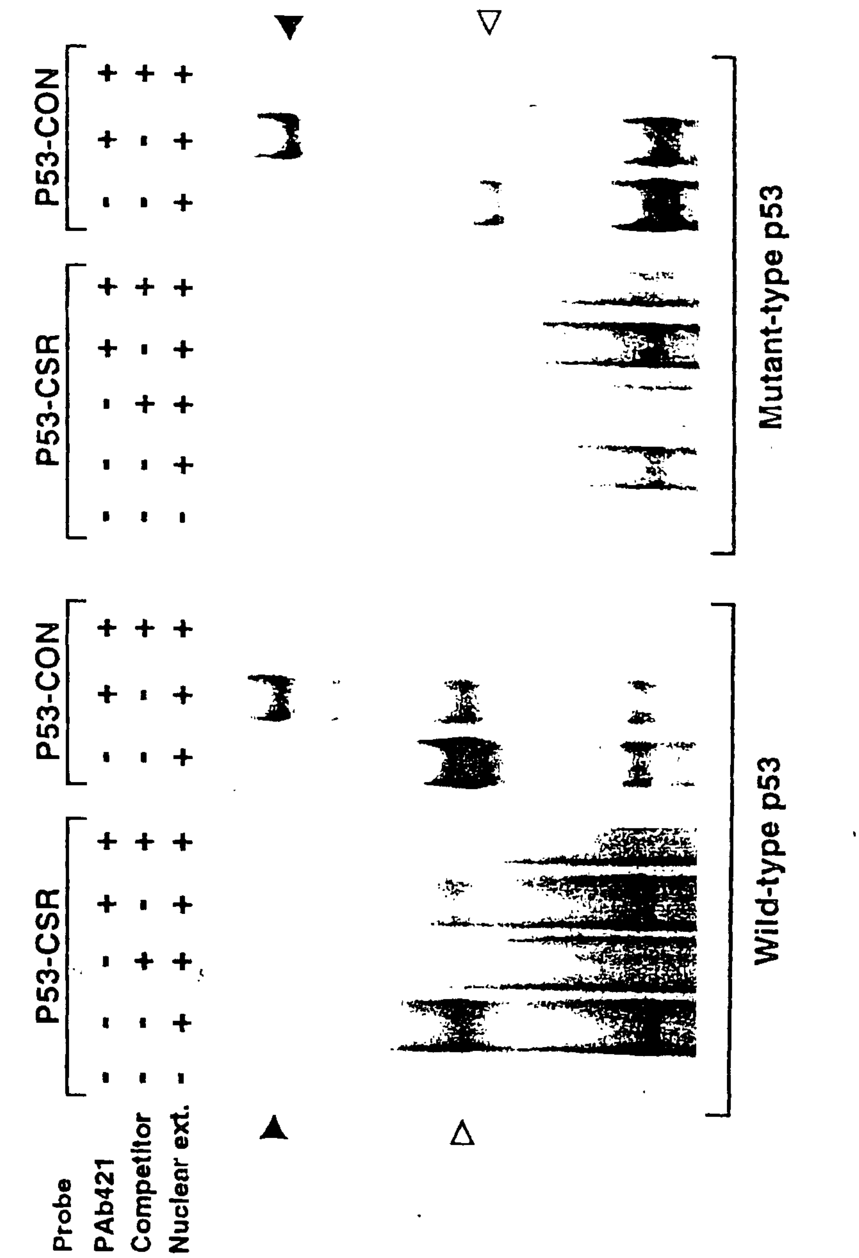
Probe
PAb421
Competitor
Nuclear ext.
P53-CSR
P53-CON
P53-CSR
P53-CON
Wild-type p53
Mutant-type p53

Figure 19

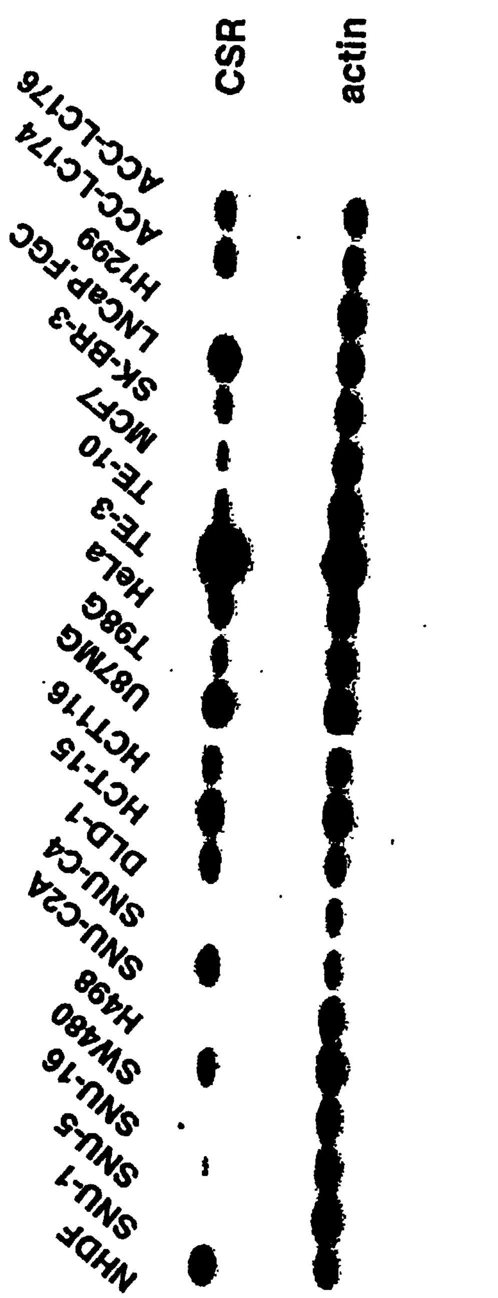
CSR
actin
ACC-LC176
ACC-LC174
H1299
LNCaP.FGC
SK-BR-3
MCF7
TE-10
TE-3
HeLa
T98G
U87MG
HCT116
HCT-15
DLD-1
SNU-C4
SNU-C2A
H498
SW480
SNU-16
SNU-5
SNU-1
NHDF

Figure 20

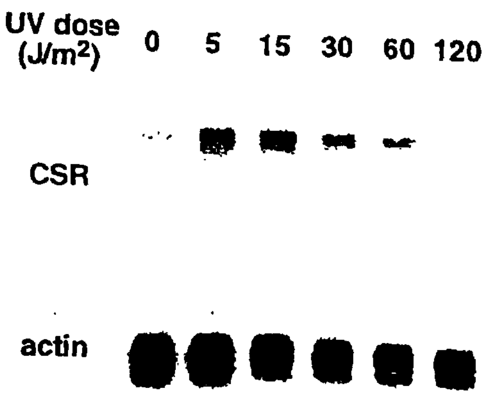
UV dose (J/m2)
0
5
15
30
60
120
CSR
actin

(a)

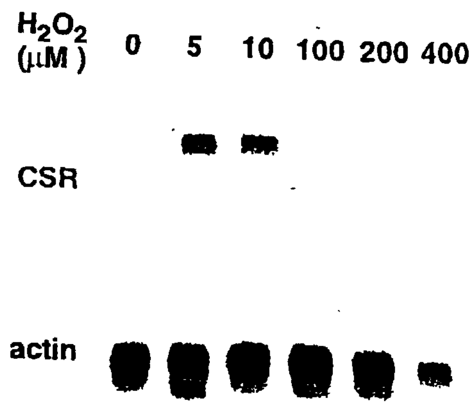
H2O2 (µM)
0
5
10
100
200
400
CSR
actin

(b)

Figure 21

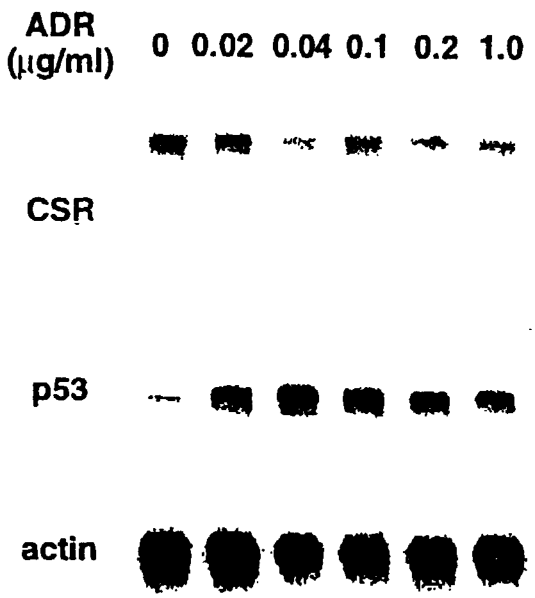

Figure 22

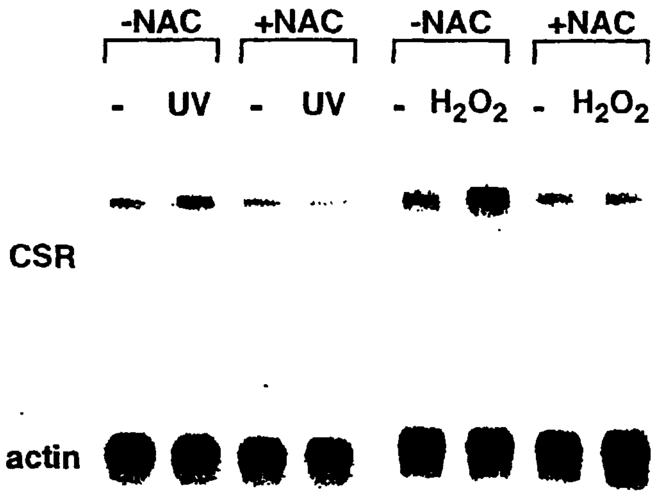

Figure 23

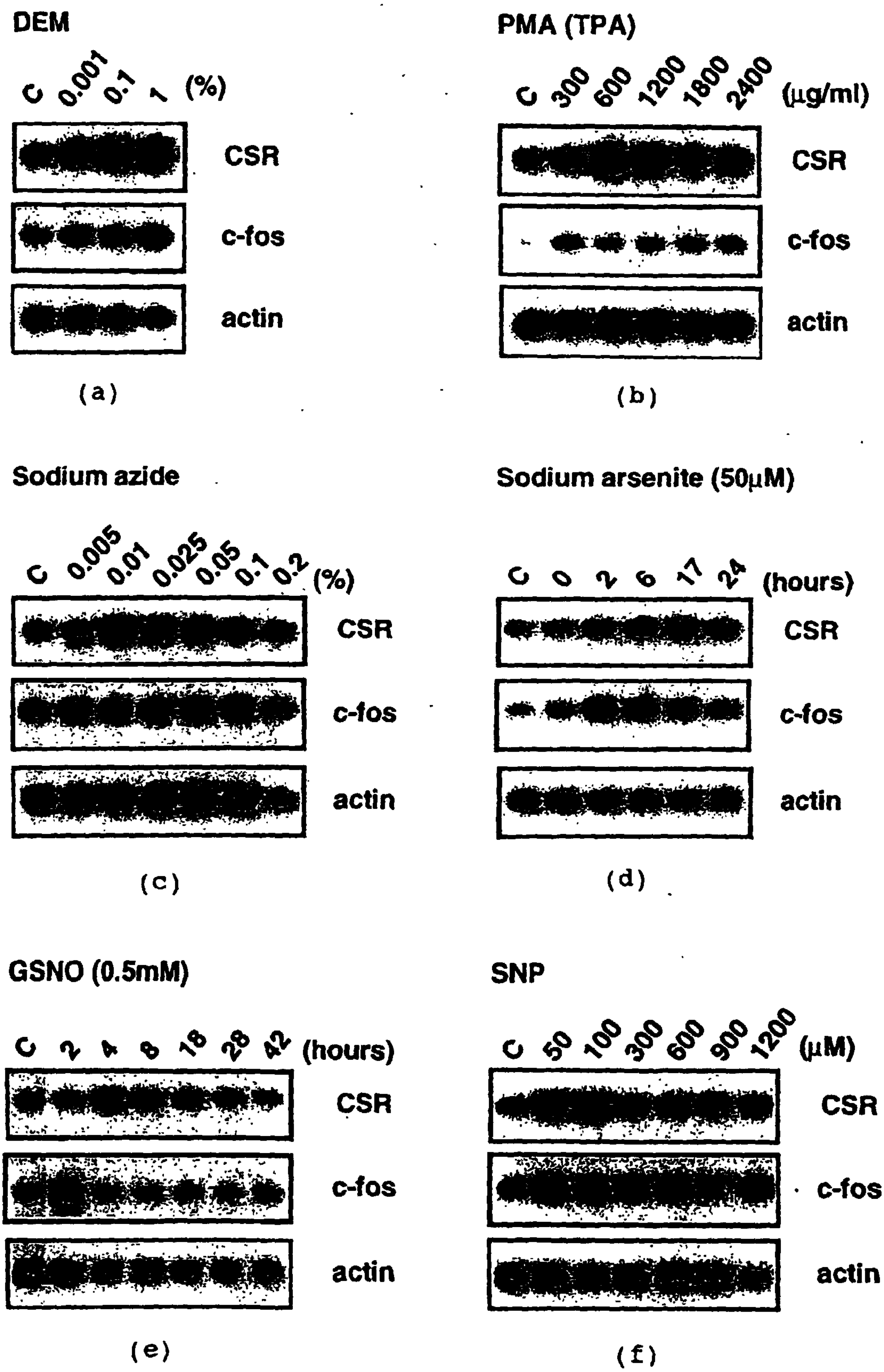
DEM
C 0.001 0.1 1 (%)
CSR
c-fos
actin
(a)
PMA (TPA)
C 300 600 1200 1800 2400 (µg/ml)
CSR
c-fos
actin
(b)
Sodium azide
C 0.005 0.01 0.025 0.05 0.1 0.2 (%)
CSR
c-fos
actin
(c)
Sodium arsenite (50µM)
C 0 2 6 17 24 (hours)
CSR
c-fos
actin
(d)
GSNO (0.5mM)
C 2 4 8 18 28 42 (hours)
CSR
c-fos
actin
(e)
SNP
C 50 100 300 600 900 1200 (µM)
CSR
c-fos
actin
(f)

Figure 24

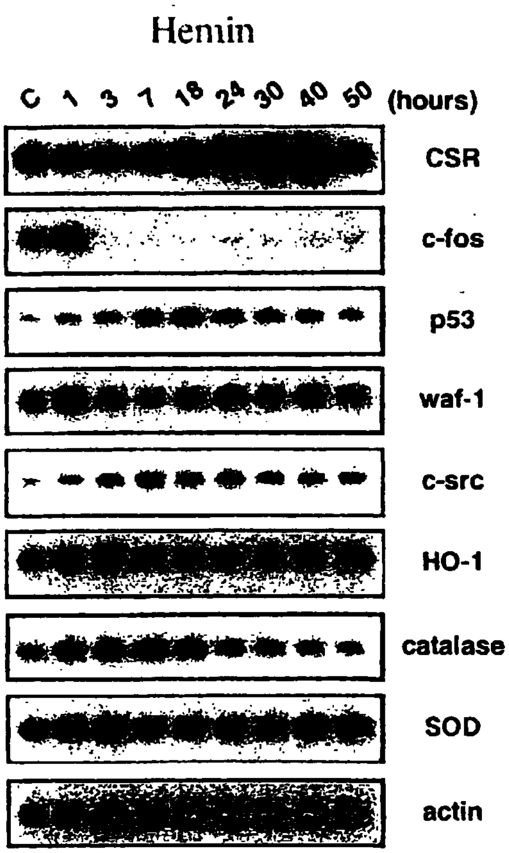
Hemin
C 1 3 7 18 24 30 40 50 (hours)
CSR
c-fos
p53
waf-1
c-src
HO-1
catalase
SOD
actin

(a)

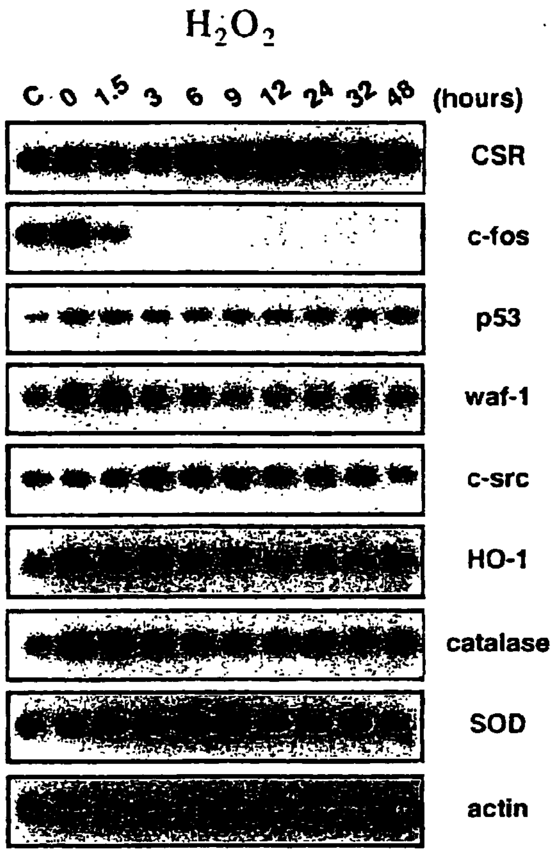
$H_2O_2$
C 0 1.5 3 6 9 12 24 32 48 (hours)
CSR
c-fos
p53
waf-1
c-src
HO-1
catalase
SOD
actin

(b)

Figure 25

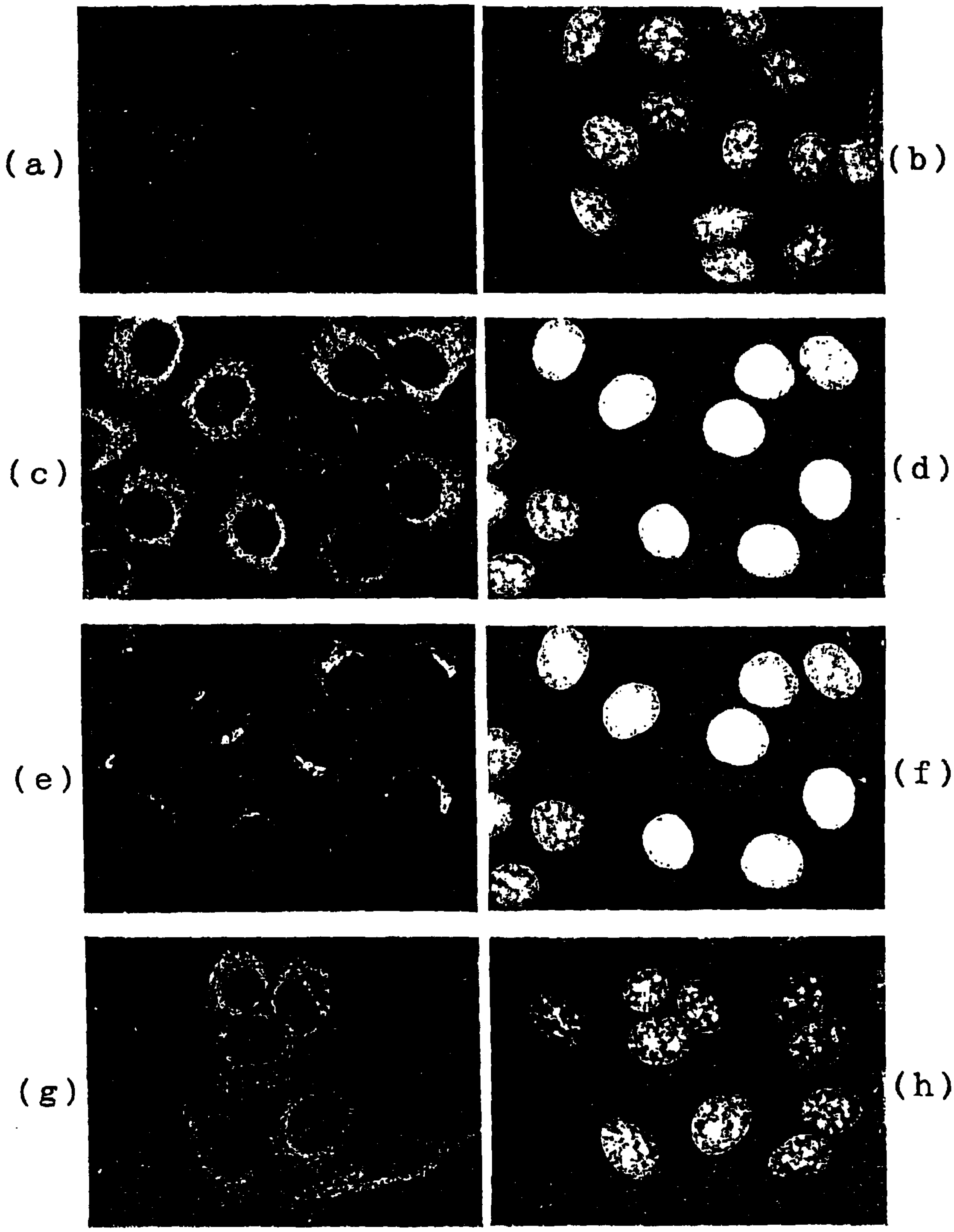

Figure 26

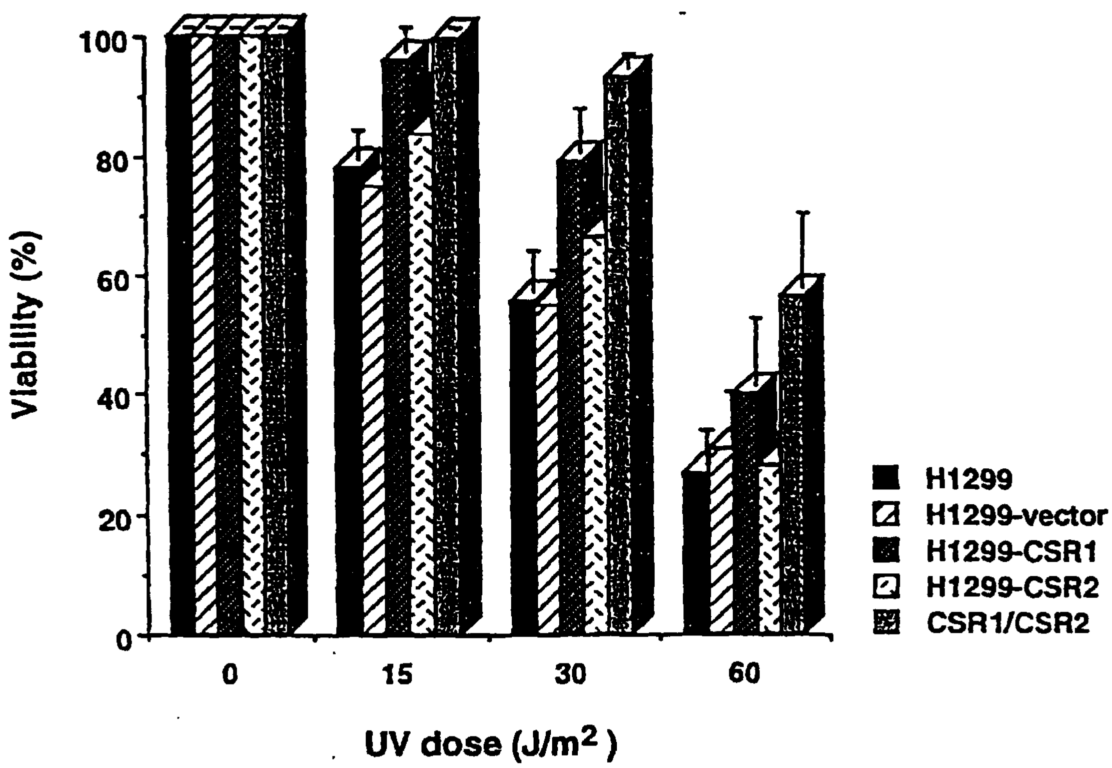
100
80
60
40
20
0
Viability (%)
0
15
30
60
UV dose (J/m2 )
H1299
H1299-vector
H1299-CSR1
H1299-CSR2
CSR1/CSR2

Figure 27

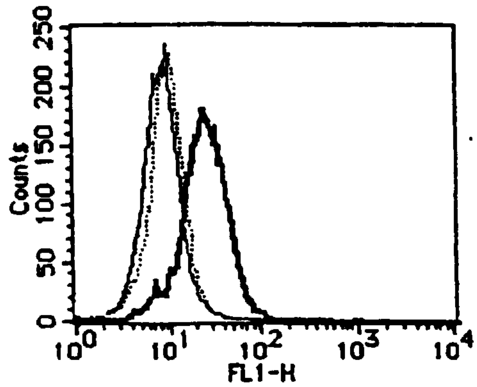


(a)

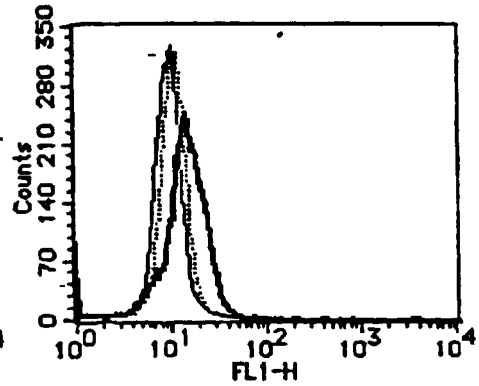


(b)

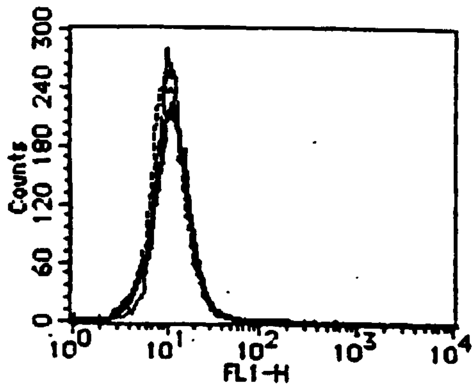


(c)

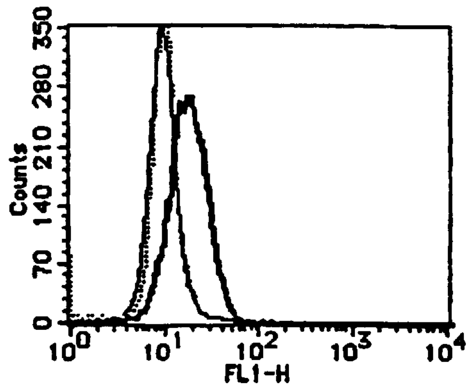


(d)

Figure 28

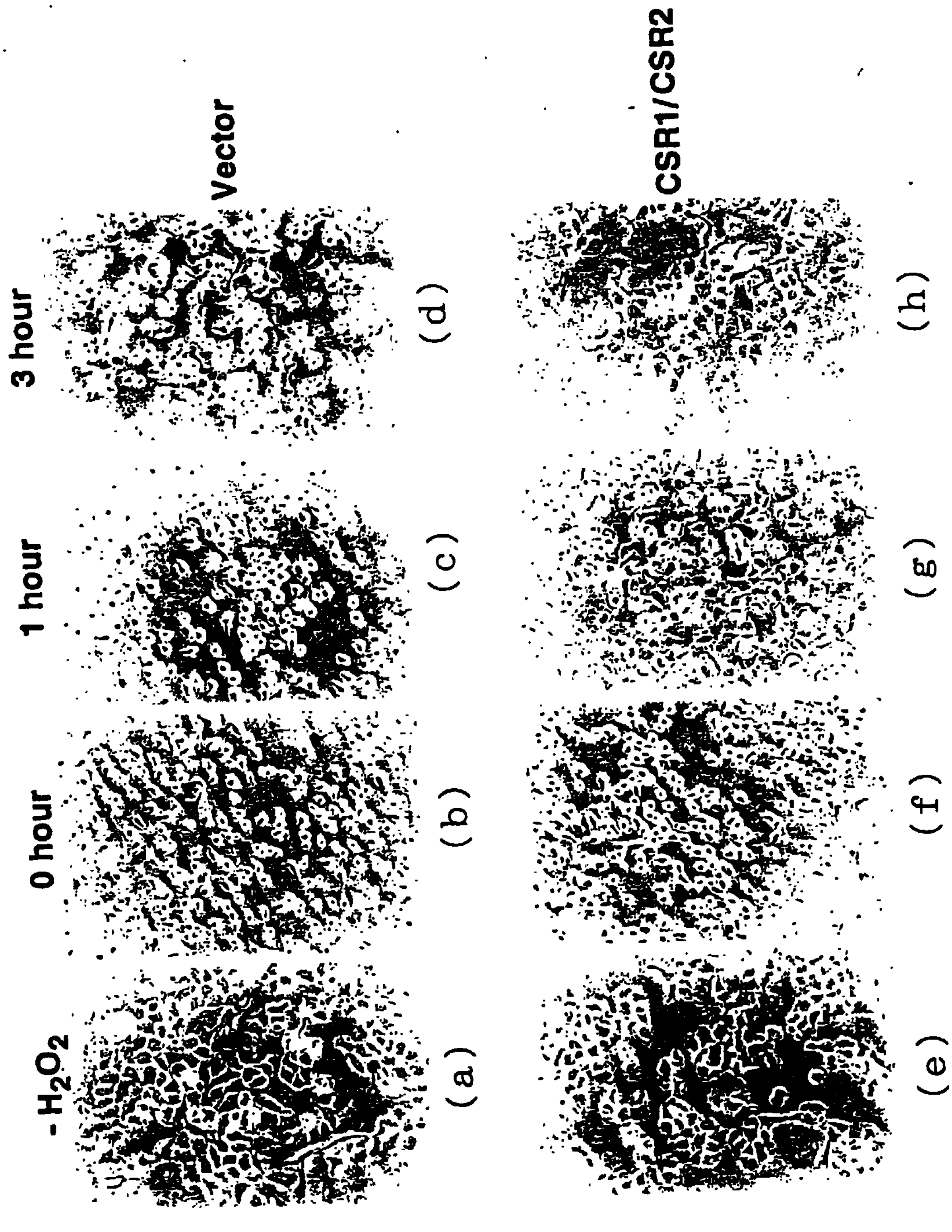

Figure 29

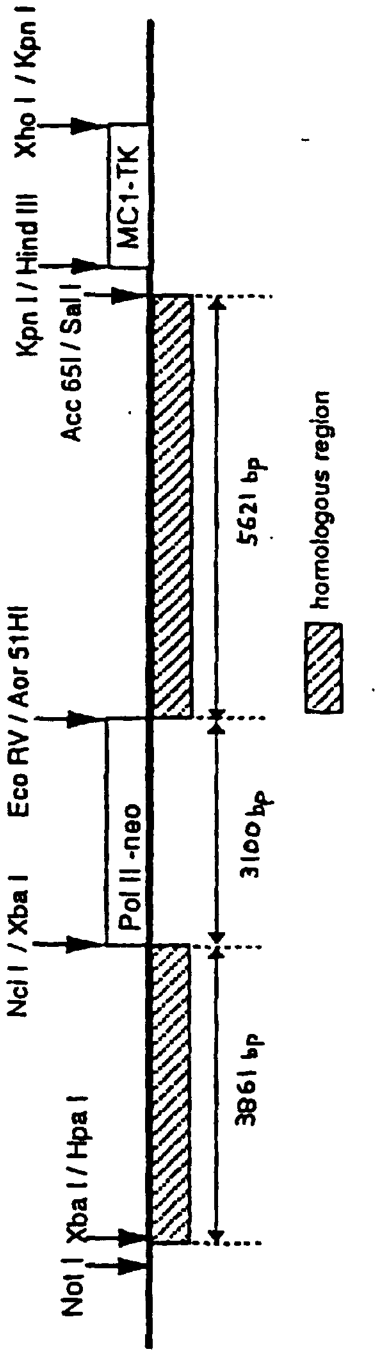
Xho I / Kpn I
Kpn I / Hind III
MC1-TK
Acc 65I / Sal I
Kpn I / Sal I
Eco RV / Aor 51HI
Ncl I / Xba I
Pol II -neo
Xba I / Hpa I
Not I
5621 bp
3100 bp
3861 bp
homologous region

## INTERNATIONAL SEARCH REPORT

International application No.
PCT/JP98/03602

A. CLASSIFICATION OF SUBJECT MATTER
Int.Cl[6] C12N15/12, C12P21/08, C12N5/20, C12N5/10

According to International Patent Classification (IPC) or to both national classification and IPC

B. FIELDS SEARCHED

Minimum documentation searched (classification system followed by classification symbols)
Int.Cl[6] C12N15/12, C12P21/08, C12N5/20, C12N5/10

Documentation searched other than minimum documentation to the extent that such documents are included in the fields searched

Electronic data base consulted during the international search (name of data base and, where practicable, search terms used)
BISIS (DIALOG), WPI (DIALOG), GenBank/EMBL (geneseq)

C. DOCUMENTS CONSIDERED TO BE RELEVANT

| Category* | Citation of document, with indication, where appropriate, of the relevant passages | Relevant to claim No. |
|---|---|---|
| X/A | US, 5691147, A (MITOTIX INC.), 25 November, 1997 (25. 11. 97) & WO, 95/33819, A2 & AU, 9526627, A | 1-14, 16-18/15 |

☐ Further documents are listed in the continuation of Box C. ☐ See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 29 October, 1998 (29. 10. 98) | 10 November, 1998 (10. 11. 98) |
| Name and mailing address of the ISA/ Japanese Patent Office | Authorized officer |
| Facsimile No. | Telephone No. |

Form PCT/ISA/210 (second sheet) (July 1992)